(19) Europäisches Patentamt / European Patent Office / Office européen des brevets

(11) EP 4 653 445 A1

(12) **EUROPEAN PATENT APPLICATION**
published in accordance with Art. 153(4) EPC

(43) Date of publication:
**26.11.2025 Bulletin 2025/48**

(21) Application number: **24744229.6**

(22) Date of filing: **16.01.2024**

(51) International Patent Classification (IPC):
***C07D 498/14** (2006.01)* ***C07D 519/00** (2006.01)*
***A61K 31/519** (2006.01)* ***A61K 31/4985** (2006.01)*
***A61P 35/00** (2006.01)*

(52) Cooperative Patent Classification (CPC):
**A61K 31/4985; A61K 31/519; A61P 35/00; C07D 498/14; C07D 519/00**

(86) International application number:
**PCT/CN2024/072459**

(87) International publication number:
**WO 2024/153053 (25.07.2024 Gazette 2024/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.01.2023 CN 202310199886**

(71) Applicants:
- **CSPC Zhongqi Pharmaceutical Technology (Shijiazhuang) Co., Ltd.**
  **Shijiazhuang, Hebei 050035 (CN)**
- **Hangzhou Innogate Pharma Co., Ltd.**
  **Hangzhou, Zhejiang 311121 (CN)**
- **CSPC OUYI Pharmaceutical Co.Ltd**
  **Shijiazhuang, Hebei 052165 (CN)**

(72) Inventors:
- **ZHAO, Jie**
  **Shijiazhuang, Hebei 050035 (CN)**
- **SUN, Xiaowei**
  **Shijiazhuang, Hebei 050035 (CN)**
- **GUO, Xiaofeng**
  **Shijiazhuang, Hebei 050035 (CN)**
- **GAO, Yuan**
  **Shijiazhuang, Hebei 050035 (CN)**
- **ZHANG, Hancheng**
  **Hangzhou, Zhejiang 311121 (CN)**
- **WANG, Yueqiao**
  **Shijiazhuang, Hebei 050035 (CN)**
- **ZHANG, Xiaolin**
  **Shijiazhuang, Hebei 050035 (CN)**
- **CAI, Congcong**
  **Hangzhou, Zhejiang 311121 (CN)**
- **DU, Xiaoli**
  **Shijiazhuang, Hebei 050035 (CN)**

(74) Representative: **Gill Jennings & Every LLP**
**The Broadgate Tower**
**20 Primrose Street**
**London EC2A 2ES (GB)**

# (54) CDK INHIBITOR AND CRYSTAL FORM OF PHARMACEUTICALLY ACCEPTABLE SALT THEREOF, AND USE THEREOF

(57) Provided is compound A or a pharmaceutically acceptable salt thereof in a crystalline form. The structure of compound A is represented by formula (I) below. Studies have shown that a crystalline form A-1 of the free base of compound A, crystalline form B of a hydrochloride salt of compound A and crystalline form C of a benzenesulfonate salt of compound A have certain physical stability and chemical stability. The compound A or the pharmaceutically acceptable salt thereof in a crystalline form can significantly inhibit the proliferation of various cells of tumors such as breast cancer, colorectal cancer, ovarian cancer, melanoma, liver cancer, lung cancer and acute myeloid leukemia; and also has a good inhibitory effect on palbociclib-resistant (including primary drug resistance and acquired drug resistance) breast cancer and liver cancer. Therefore, the compound A or the pharmaceutically acceptable salt thereof in a crystalline form has good clinical application prospects in the treatment of advanced malignant tumors, and provides a new drug choice for the treatment of CDK4/6 inhibitor-resistant tumors.

EP 4 653 445 A1

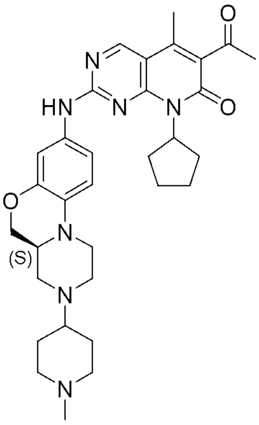

(I)

## Description

### CROSS-REFERENCE TO RELATED APPLICATION

This application claims priority to and the benefits of Chinese Patent Application No. 202310199886.6, filed with the China National Intellectual Property Administration on January 17, 2023, the entire content of which is incorporated herein by reference.

### TECHNICAL FIELD

The present application belongs to the medicine field, and specifically relates to a CDK inhibitor, a crystalline form of a pharmaceutically acceptable salt thereof, and uses thereof.

### BACKGROUND

In recent years, tumors have surpassed cardiovascular diseases to become the leading cause of death globally. Researches on anti-tumor drugs are of significant academic and practical importance. Excessively activated and sustained cell proliferation is a fundamental characteristic of tumors. Therefore, inducing cell cycle arrest can effectively inhibit tumor growth. Cyclin dependent kinases (CDKs), belonging to the serine/threonine protein kinase family, are key kinases involved in cell cycle regulation. To date, 20 distinct CDKs have been reported, which can be broadly categorized into two classes based on their functions. One class of CDKs, mainly including CDK1, CDK2, CDK4, CDK6, etc., participates in cell cycle regulation. The other class of CDKs, mainly including CDK7, CDK8, CDK9, CDK10, CDK11, etc., participates in transcription regulation. In tumor cells, overexpression or over-activation of cyclins, inhibition of CDKi activity, persistent activation of upstream mitogenic signals, etc. can lead to change of CDK activity. Aberrant CDK activity directly or indirectly causes uncontrolled cell proliferation, genomic instability (increased DNA mutations, chromosomal deletions, etc.), and chromosomal instability (changes in chromosome number), etc., contributing to tumorigenesis and tumor progression. As CDK activity is essential for cell division and is frequently enhanced in tumor cells, CDKs have long been regarded as a promising target for developing drugs against tumors and other proliferative diseases.

Currently, the clinical application of several CDK4/6 inhibitors, such as Pfizer's Palbociclib, Eli Lilly's Abemaciclib, Novartis' Ribociclib, and Hengrui's Dalpiciclib, has fully validated the efficacy of this target, for treating hormone receptor-positive (HR+)/human epidermal growth factor receptor 2-negative (HER2-) advanced or metastatic breast cancer.

For HR+/HER2- advanced or metastatic breast cancer patients, early-stage treatment often involves endocrine therapy. For example, commonly used endocrine therapy drugs include Anastrozole, Letrozole, Exemestane, Tamoxifen, Toremifene, Fulvestrant, Megestrol, Fluoxymesterone, Ethinylestradiol, etc. Patients with intermediate-to-high risk in early stages may also receive chemotherapy. For advanced or metastatic patients, the current first-line standard therapy combines CDK4/6 inhibitors with endocrine therapy. Multiple authoritative guidelines, including the Chinese National Health Commission's Breast Cancer Diagnosis and Treatment Guidelines (2022 Edition), the CSCO Breast Cancer Diagnosis and Treatment Guidelines (2022 Edition), and the NCCN Clinical Practice Guidelines in Oncology: Breast Cancer (2022 Edition), have elevated the evidence level for the CDK4/6 inhibitor in combination with endocrine therapy to Grade I, recommending its clinical use in HR+/HER2- advanced breast cancer.

WO2021139817A1 discloses a CDK inhibitor compound A with a structure represented by Formula (I) below:

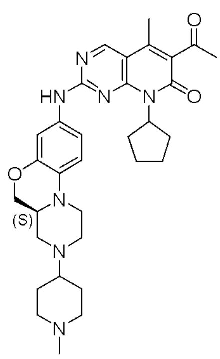

(I).

In vitro studies have shown that the compound exhibits potent inhibitory effects on multiple CDK kinases and TRK kinases, as well as favorable in vivo bioavailability, suggesting its potential for treating tumors and other related diseases. However, systematic studies on the crystalline forms of this compound and the pharmaceutically acceptable salt thereof

have not yet been conducted.

## SUMMARY OF INVENTION

In a first aspect, the present application provides a compound A or a pharmaceutically acceptable salt thereof in a crystalline form. The compound A has a structure represented by Formula (I) below:

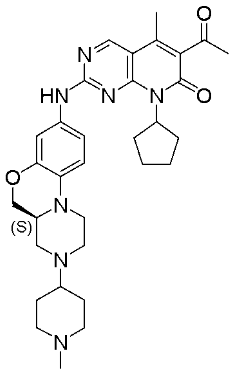

(I).

In some embodiments, a differential scanning calorimetry (DSC) profile of the compound A in the crystalline form has an endothermic peak at 256±3 °C.

In some embodiments, the present application provides the compound A in the crystalline form. In some embodiments, the compound A in the crystalline form is an anhydrate.

In some embodiments, the compound A in the crystalline form is a crystalline form A-I, an X-ray powder diffraction pattern of which, using Cu-Kα radiation and expressed as 2θ angles (°), has characteristic peaks at 5.2 ± 0.2°, 11.4 ± 0.2°, 15.7 ± 0.2°, 20.2 ± 0.2°, and 22.0 ± 0.2°.

In some embodiments, the compound A in the crystalline form is a crystalline form A-I, an X-ray powder diffraction pattern of which, using Cu-Kα radiation and expressed as 2θ angles (°), has characteristic peaks at 5.2 ± 0.2°, 9.5 ± 0.2°, 11.4 ± 0.2°, 15.7 ± 0.2°, 16.3 ± 0.2°, 20.2 ± 0.2°, and 22.0 ± 0.2°.

In some embodiments, the compound A in the crystalline form is a crystalline form A-I, an X-ray powder diffraction pattern of which, using Cu-Kα radiation and expressed as 2θ angles (°), has characteristic peaks at 5.2 ± 0.2°, 9.5 ± 0.2°, 10.4 ± 0.2°, 11.4 ± 0.2°, 12.8 ± 0.2°, 14.1 ± 0.2°, 15.7 ± 0.2°, 16.3 ± 0.2°, 18.6 ± 0.2°, 20.2 ± 0.2°, 22.0 ± 0.2°, and 25.8 ± 0.2°.

In some embodiments, the compound A in the crystalline form is a crystalline form A-I, an X-ray powder diffraction pattern of which, using Cu-Kα radiation and expressed as 2θ angles (°), has diffraction peaks with peak positions and relative peak intensities shown in Table 1 below.

**Table 1 Data of** X-ray **Powder Diffraction Pattern of Crystalline Form A-I**

| No. | 2θ Angle (°) | Relative Peak Intensity (%) |
|---|---|---|
| 1 | 5.24 | 62.6 |
| 2 | 9.62 | 12.5 |
| 3 | 10.44 | 11.7 |
| 4 | 11.46 | 27.1 |
| 5 | 12.89 | 14.1 |
| 6 | 14.19 | 10.0 |
| 7 | 15.77 | 39.1 |
| 8 | 16.36 | 34.1 |
| 9 | 18.69 | 14.6 |
| 10 | 20.28 | 100.0 |
| 11 | 20.63 | 91.3 |
| 12 | 22.07 | 47.2 |

(continued)

| No. | 2θ Angle (°) | Relative Peak Intensity (%) |
|---|---|---|
| 13 | 25.89 | 25.6 |

In some embodiments, the compound A in the crystalline form is a crystalline form A-I, an X-ray powder diffraction pattern of which, using Cu-Kα radiation, is substantially as shown in FIG. 1-1.

In some embodiments, the compound A in the crystalline form is a crystalline form A-I, a DSC profile of which has an endothermic peak at 256±3 °C.

In some embodiments, the compound A in the crystalline form is a crystalline form A-I, a DSC profile of which is substantially as shown in FIG. 1-3.

In some embodiments, the compound A in the crystalline form is a crystalline form A-II, an X-ray powder diffraction pattern of which, using Cu-Kα radiation and expressed as 2θ angles (°), has characteristic peaks at 4.1±0.2°, 16.2±0.2°, 16.8+0.2°, 20.1±0.2°, and 20.7±0.2°.

In some embodiments, the compound A in the crystalline form is a crystalline form A-II, an X-ray powder diffraction pattern of which, using Cu-Kα radiation and expressed as 2θ angles (°), has characteristic peaks at 4.1±0.2°, 16.2±0.2°, 16.8±0.2°, 17.7±0.2°, 20.1±0.2°, and 20.7±0.2°.

In some embodiments, the compound A in the crystalline form is a crystalline form A-II, an X-ray powder diffraction pattern of which, using Cu-Kα radiation and expressed as 2θ angles (°), has diffraction peaks with peak positions and relative peak intensities shown in Table 2 below.

**Table 2 Data of X-ray Powder Diffraction Pattern of Crystalline Form A-II**

| No. | 2θ Angle (°) | Relative Peak Intensity (%) |
|---|---|---|
| 1 | 4.08 | 100.0 |
| 2 | 16.17 | 37.8 |
| 3 | 16.79 | 21.4 |
| 4 | 17.65 | 5.2 |
| 5 | 20.13 | 47.0 |
| 6 | 20.68 | 70.9 |

In some embodiments, the compound A in the crystalline form is a crystalline form A-II, an X-ray powder diffraction pattern of which, using Cu-Kα radiation, is substantially as shown in FIG. 2-1.

In some embodiments, the compound A in the crystalline form is a crystalline form A-II, which is a solvate. In some embodiments, the crystalline form A-II is a trifluoroethanol solvate of the compound A in the crystalline form. In some embodiments, the crystalline form A-II is a trifluoroethanol solvate of the compound A in the crystalline form, wherein a molar ratio of the compound A to trifluoroethanol is 1:1.

In some embodiments, the compound A in the crystalline form is a crystalline form A-II, a DSC profile of which has endothermic peaks at 100 °C to 130 °C and at 256±3 °C.

In some embodiments, the compound A in the crystalline form is a crystalline form A-II, a DSC profile of which is substantially as shown in FIG. 2-3.

In some embodiments, the compound A in the crystalline form is a crystalline form A-III, an X-ray powder diffraction pattern of which, using Cu-Kα radiation and expressed as 2θ angles (°), has characteristic peaks at 6.6±0.2°, 13.2±0.2°, 16.5±0.2°, 20.1±0.2°, and 23.0±0.2°.

In some embodiments, the compound A in the crystalline form is a crystalline form A-III, an X-ray powder diffraction pattern of which, using Cu-Kα radiation and expressed as 2θ angles (°), has characteristic peaks at 6.6±0.2°, 13.2±0.2°, 15.2±0.2°, 16.5±0.2°, 20.1±0.2°, 23.0±0.2°, and 28.2±0.2°.

In some embodiments, the compound A in the crystalline form is a crystalline form A-III, an X-ray powder diffraction pattern of which, using Cu-Kα radiation and expressed as 2θ angles (°), has diffraction peaks with peak positions and relative peak intensities shown in Table 3 below.

**Table 3 Data of X-ray Powder Diffraction Pattern of Crystalline Form A-III**

| No. | 2θ Angle (°) | Relative Peak Intensity (%) |
|---|---|---|
| 1 | 6.58 | 49.9 |

(continued)

| No. | 2θ Angle (°) | Relative Peak Intensity (%) |
|---|---|---|
| 2 | 13.19 | 27.0 |
| 3 | 15.2 | 13.3 |
| 4 | 16.53 | 28.8 |
| 5 | 20.08 | 100.0 |
| 6 | 23.01 | 30.8 |
| 7 | 28.17 | 11.9 |

In some embodiments, the compound A in the crystalline form is a crystalline form A-III, an X-ray powder diffraction pattern of which, using Cu-Kα radiation, is substantially as shown in FIG. 3-1.

In some embodiments, the compound A in the crystalline form is a crystalline form A-III, which is a solvate. In some embodiments, the crystalline form A-III is a dimethyl sulfoxide solvate of the compound A in the crystalline form. In some embodiments, the crystalline form A-III is a dimethyl sulfoxide solvate of the compound A in the crystalline form, wherein a molar ratio of the compound A to dimethyl sulfoxide is 1:1.

In some embodiments, the compound A in the crystalline form is a crystalline form A-III, a DSC profile of which has endothermic peaks at 100 °C to 150 °C and 256±3 °C.

In some embodiments, the compound A in the crystalline form is a crystalline form A-III, a DSC profile of which is substantially as shown in FIG. 3-3.

In some embodiments, the compound A in the crystalline form is a crystalline form A-IV, an X-ray powder diffraction pattern of which, using Cu-Kα radiation and expressed as 2θ angles (°), has characteristic peaks at 4.1±0.2°, 5.4±0.2°, 9.5±0.2°, 12.1±0.2°, 15.9±0.2°, and 20.7±0.2°.

In some embodiments, the compound A in the crystalline form is a crystalline form A-IV, an X-ray powder diffraction pattern of which, using Cu-Kα radiation and expressed as 2θ angles (°), has characteristic peaks at 4.1+0.2°, 5.4 ±0.2°, 9.5±0.2°, 12.1±0.2°, 15.9±0.2°, 17.8±0.2°, 19.3±0.2°, and 20.7±0.2°.

In some embodiments, the compound A in the crystalline form is a crystalline form A-IV, an X-ray powder diffraction pattern of which, using Cu-Kα radiation and expressed as 2θ angles (°), has diffraction peaks with peak positions and relative peak intensities shown in Table 4 below.

**Table 4 Data of X-ray Powder Diffraction Pattern of Crystalline Form A-IV**

| No. | 2θ Angle (°) | Relative Peak Intensity (%) |
|---|---|---|
| 1 | 4.1 | 11.4 |
| 2 | 5.36 | 43.4 |
| 3 | 9.52 | 20.4 |
| 4 | 12.08 | 20.1 |
| 5 | 15.9 | 40.7 |
| 6 | 17.8 | 19.3 |
| 7 | 19.26 | 8.4 |
| 8 | 19.87 | 17.8 |
| 9 | 20.44 | 100.0 |
| 10 | 20.72 | 89.1 |

In some embodiments, the compound A in the crystalline form is a crystalline form A-IV, an X-ray powder diffraction pattern of which, using Cu-Kα radiation, is substantially as shown in FIG. 4-1. In some embodiments, the compound A in the crystalline form is a crystalline form A-IV, a DSC profile of which is substantially as shown in FIG. 4-3.

In some embodiments, the pharmaceutically acceptable salt of the compound A in the crystalline form is selected from the group consisting of sulfate, methanesulfonate, tartrate, benzenesulfonate, hydrochloride, p-toluenesulfonate, fumarate, citrate, and malate; preferably, benzenesulfonate, hydrochloride, p-toluenesulfonate, fumarate, citrate, and malate; more preferably, hydrochloride and benzenesulfonate.

In some embodiments, the pharmaceutically acceptable salt of the compound A in the crystalline form is selected from hydrochloride salts. In some embodiments, the hydrochloride salt of the compound A in the crystalline form is a hydrate. In some embodiments, the hydrochloride salt of the compound A in the crystalline form is a hydrochloride monohydrate of the compound A.

In some embodiments, the hydrochloride salt of the compound A in the crystalline form is a crystalline form B, an X-ray powder diffraction pattern of which, using Cu-K$\alpha$ radiation and expressed as 2$\theta$ angles (°), has characteristic peaks at 4.1±0.2°, 8.9±0.2°, 14.3±0.2°, and 19.9±0.2°.

In some embodiments, the hydrochloride salt of the compound A in the crystalline form is a crystalline form B, an X-ray powder diffraction pattern of which, using Cu-K$\alpha$ radiation and expressed as 2$\theta$ angles (°), has characteristic peaks at 4.1±0.2°, 8.9±0.2°, 12.3±0.2°, 14.3±0.2°, 15.4±0.2°, 16.8±0.2°, and 19.9±0.2°.

In some embodiments, the hydrochloride salt of the compound A in the crystalline form is a crystalline form B, an X-ray powder diffraction pattern of which, using Cu-K$\alpha$ radiation and expressed as 2$\theta$ angles (°), has characteristic peaks at 4.1±0.2°, 8.3±0.2°, 8.9±0.2°, 9.5±0.2°, 12.3±0.2°, 14.3±0.2°, 15.0±0.2°, 15.4±0.2°, 16.8±0.2°, 19.1±0.2°, 19.9 ±0.2°, 20.6+0.2°, and 25.5±0.2°.

In some embodiments, the hydrochloride salt of the compound A in the crystalline form is a crystalline form B, an X-ray powder diffraction pattern of which, using Cu-K$\alpha$ radiation and expressed as 2$\theta$ angles (°), has characteristic peaks at 4.1±0.2°, 8.3±0.2°, 8.9±0.2°, 9.5±0.2°, 11.1±0.2°, 12.3±0.2°, 14.3±0.2°, 15.0±0.2°, 15.4±0.2°, 16.8±0.2°, 17.4 ±0.2°, 19.1±0.2°, 19.9+0.2°, 20.6+0.2°, 21.6±0.2°, 25.2±0.2°, 25.5±0.2°, 26.6±0.2°, and 29.1±0.2°.

In some embodiments, the hydrochloride salt of the compound A in the crystalline form is a crystalline form B, an X-ray powder diffraction pattern of which, using Cu-K$\alpha$ radiation and expressed as 2$\theta$ angles (°), has diffraction peaks with peak positions and relative peak intensities shown in Table 5 below.

**Table 5 Data of X-ray Powder Diffraction Pattern of Crystalline Form B**

| No. | 2θ Angle (°) | Relative Peak Intensity (%) | No. | 2θ Angle (°) | Relative Peak Intensity (%) |
|---|---|---|---|---|---|
| 1 | 4.071 | 27.30% | 11 | 17.441 | 11.90% |
| 2 | 8.246 | 20.60% | 12 | 19.104 | 15.30% |
| 3 | 8.906 | 100.00% | 13 | 19.866 | 39.90% |
| 4 | 9.522 | 15.60% | 14 | 20.641 | 17.30% |
| 5 | 11.142 | 7.00% | 15 | 21.57 | 10.10% |
| 6 | 12.318 | 23.80% | 16 | 25.229 | 29.10% |
| 7 | 14.263 | 58.90% | 17 | 25.531 | 32.50% |
| 8 | 14.982 | 23.20% | 18 | 26.574 | 12.00% |
| 9 | 15.387 | 33.50% | 19 | 29.057 | 16.10% |
| 10 | 16.788 | 31.40% | | | |

In some embodiments, the hydrochloride salt of the compound A in the crystalline form is a crystalline form B, an X-ray powder diffraction pattern of which, using Cu-K$\alpha$ radiation, is substantially as shown in FIG. 5-1.

In some embodiments, the hydrochloride salt of the compound A in the crystalline form is a crystalline form B, a thermogravimetric analysis (TGA) profile of which shows a weight loss of approximately 4.4% at a temperature of from room temperature to 130 °C.

In some embodiments, the hydrochloride salt of the compound A in the crystalline form is a crystalline form B, a DSC profile of which has an endothermic peak at 253±3 °C. In some embodiments, the hydrochloride salt of the compound A in the crystalline form is a crystalline form B, a DSC profile of which has two endothermic peaks at 27±3 °C and 253±3 °C.

In some embodiments, the crystalline form B is a hydrochloride monohydrate of the compound A in the crystalline form, wherein a molar ratio of the compound A, hydrochloric acid, and water is 1:1:1.

In some embodiments, the pharmaceutically acceptable salt of the compound A in the crystalline form is selected from benzenesulfonate. In some embodiments, the benzenesulfonate of the compound A in the crystalline form is an anhydrate. In some embodiments, a molar ratio of a free base of the compound A to benzenesulfonic acid in the benzenesulfonate of the compound A in the crystalline form is 1:1.

In some embodiments, the benzenesulfonate of the compound A in the crystalline form is a crystalline form C, an X-ray powder diffraction pattern of which, using Cu-K$\alpha$ radiation and expressed as 2$\theta$ angles (°), has characteristic peaks at 8.5±0.2°, 15.3±0.2°, 19.2±0.2°, 21.7±0.2°, 22.1±0.2°, and 26.1±0.2°.

In some embodiments, the benzenesulfonate of the compound A in the crystalline form is a crystalline form C, an X-ray powder diffraction pattern of which, using Cu-Kα radiation and expressed as 2θ angles (°), has characteristic peaks at 8.5±0.2°, 15.3±0.2°, 16.4±0.2°, 17.1 ±0.2°, 17.8±0.2°, 19.2±0.2°, 21.7±0.2°, 22.1±0.2°, and 26.1+0.2°.

In some embodiments, the benzenesulfonate of the compound A in the crystalline form is a crystalline form C, an X-ray powder diffraction pattern of which, using Cu-Kα radiation and expressed as 2θ angles (°), has characteristic peaks at 8.5±0.2°, 15.3±0.2°, 16.4±0.2°, 17.1 ±0.2°, 17.8±0.2°, 18.1±0.2°, 19.2±0.2°, 20.2±0.2°, 21.7±0.2°, 22.1±0.2°, and 26.1±0.2°.

In some embodiments, the benzenesulfonate of the compound A in the crystalline form is a crystalline form C, an X-ray powder diffraction pattern of which, using Cu-Kα radiation and expressed as 2θ angles (°), has characteristic peaks at 8.5±0.2°, 11.7±0.2°, 12.7±0.2°, 13.3±0.2°, 14.24±0.2°, 15.3±0.2°, 16.4±0.2°, 17.1±0.2°, 17.8±0.2°, 18.1±0.2°, 19.2±0.2°, 19.5±0.2°, 20.2±0.2°, 21.7±0.2°, 22.1±0.2°, and 26.1±0.2°.

In some embodiments, the benzenesulfonate of the compound A in the crystalline form is a crystalline form C, an X-ray powder diffraction pattern of which, using Cu-Kα radiation and expressed as 2θ angles (°), has diffraction peaks with peak positions and relative peak intensities shown in Table 6 below.

**Table 6 Data of X-ray Powder Diffraction Pattern of Crystalline Form C**

| No. | 2θ Angle (°) | Relative Peak Intensity (%) | No. | 2θ Angle (°) | Relative Peak Intensity (%) |
|---|---|---|---|---|---|
| 1 | 8.496 | 47.30% | 12 | 17.817 | 40.70% |
| 2 | 11.687 | 14.10% | 13 | 18.111 | 32.90% |
| 3 | 12.704 | 20.00% | 14 | 19.167 | 70.40% |
| 4 | 12.915 | 16.40% | 15 | 19.487 | 27.60% |
| 5 | 13.327 | 16.60% | 16 | 20.23 | 22.10% |
| 6 | 13.887 | 17.70% | 17 | 20.583 | 10.10% |
| 7 | 14.182 | 17.70% | 18 | 21.742 | 54.00% |
| 8 | 15.335 | 38.60% | 19 | 22.126 | 100.00% |
| 9 | 15.784 | 12.00% | 20 | 22.794 | 14.10% |
| 10 | 16.358 | 32.50% | 21 | 26.101 | 32.10% |
| 11 | 17.116 | 30.60% | | | |

In some embodiments, the benzenesulfonate of the compound A in the crystalline form is a crystalline form C, an X-ray powder diffraction of which, using Cu-Kα radiation, is substantially as shown in FIG. 6-1.

In some embodiments, the benzenesulfonate of the compound A in the crystalline form is a crystalline form C, a DSC profile of which has an endothermic peak at 235±3 °C.

In some embodiments, the benzenesulfonate of the compound A in the crystalline form is a crystalline form C, a DSC profile of which is substantially as shown in FIG. 6-2.

In some embodiments, the crystalline form C is a benzenesulfonate of the compound A in the crystalline form, wherein a molar ratio of a free base of the compound A to benzenesulfonic acid is 1:1.

In some embodiments, the pharmaceutically acceptable salt of the compound A in the crystalline form is selected from p-toluenesulfonate. In some embodiments, the p-toluenesulfonate of the compound A in the crystalline form is a hydrate.

In some embodiments, the p-toluenesulfonate of the compound A in the crystalline form is a crystalline form D-I, an X-ray powder diffraction pattern of which, using Cu-Kα radiation and expressed as 2θ angles (°), has characteristic peaks at 9.1±0.2°, 13.3±0.2°, 15.8±0.2°, 19.6±0.2°, and 20.9±0.2°.

In some embodiments, the p-toluenesulfonate of the compound A in the crystalline form is a crystalline form D-I, an X-ray powder diffraction pattern of which, using Cu-Kα radiation and expressed as 2θ angles (°), has characteristic peaks at 7.9±0.2°, 9.1±0.2°, 12.9±0.2°, 13.3±0.2°, 15.8±0.2°, 18.5±0.2°, 19.6±0.2°, and 20.9±0.2°.

In some embodiments, the p-toluenesulfonate of the compound A in the crystalline form is a crystalline form D-I, an X-ray powder diffraction pattern of which, using Cu-Kα radiation and expressed as 2θ angles (°), has diffraction peaks with peak positions and relative peak intensities shown in Table 7 below.

**Table 7 Data of X-ray Powder Diffraction Pattern of Crystalline Form D-I**

| No. | 2θ Angle (°) | Relative Peak Intensity (%) |
|---|---|---|
| 1 | 7.866 | 20.60% |
| 2 | 9.126 | 100.00% |
| 3 | 12.982 | 26.70% |
| 4 | 13.299 | 33.00% |
| 5 | 15.817 | 29.10% |
| 6 | 18.538 | 34.60% |
| 7 | 19.565 | 35.30% |
| 8 | 20.942 | 87.80% |

In some embodiments, the p-toluenesulfonate of the compound A in the crystalline form is a crystalline form D-I, an X-ray powder diffraction of which, using Cu-Kα radiation, is substantially as shown in FIG. 7-1.

In some embodiments, the p-toluenesulfonate of the compound A in the crystalline form is a crystalline form D-I, TGA of which shows a weight loss of approximately 4.2% at a temperature of from room temperature to 150 °C.

In some embodiments, the p-toluenesulfonate of the compound A in the crystalline form is a crystalline form D-I, a DSC profile of which has a relatively broad endothermic peak at 167±3 °C. In some embodiments, the p-toluenesulfonate of the compound A in the crystalline form is a crystalline form D-I, DSC of which shows two relatively broad endothermic peaks at 49±3 °C and 167±3 °C.

In some embodiments, the p-toluenesulfonate of the compound A in the crystalline form is a crystalline form D-I, a DSC profile of which is substantially as shown in FIG. 7-2.

In some embodiments, the crystalline form D-I is a p-toluenesulfonate monohydrate of the compound A in the crystalline form, wherein a molar ratio of the compound A, p-toluenesulfonic acid, and water is 1:1:1.

In some embodiments, the pharmaceutically acceptable salt of the compound A in the crystalline form is selected from fumarate. In some embodiments, a molar ratio of fumaric acid to a free base of the compound A in the fumarate of the compound A in the crystalline form is 1:1. In some embodiments, the fumarate of the compound A in the crystalline form is a hydrate. In some embodiments, the fumarate of the compound A in the crystalline form is a monohydrate.

In some embodiments, the fumarate of the compound A in the crystalline form is a crystalline form E, an X-ray powder diffraction pattern of which, using Cu-Kα radiation and expressed as 2θ angles (°), has characteristic peaks at 4.1 ±0.2°, 13.2±0.2°, 16.6±0.2°, 19.9±0.2°, and 21.5±0.2°.

In some embodiments, the fumarate of the compound A in the crystalline form is a crystalline form E, an X-ray powder diffraction pattern of which, using Cu-Kα radiation and expressed as 2θ angles (°), has characteristic peaks 4.1 ±0.2°, 8.4±0.2°, 12.5±0.2°, 13.2±0.2°, 13.9±0.2°, 16.6±0.2°, 19.9±0.2°, 21.5±0.2°, and 22.1±0.2°.

In some embodiments, the fumarate of the compound A in the crystalline form is a crystalline form E, an X-ray powder diffraction pattern of which, using Cu-Kα radiation and expressed as 2θ angles (°), has diffraction peaks with peak positions and relative peak intensities shown in Table 8 below.

**Table 8 Data of X-ray Powder Diffraction Pattern of Crystalline Form E**

| No. | 2θ Angle (°) | Relative Peak Intensity (%) |
|---|---|---|
| 1 | 4.148 | 100.00% |
| 2 | 8.35 | 9.20% |
| 3 | 12.474 | 14.70% |
| 4 | 13.196 | 20.30% |
| 5 | 13.917 | 7.20% |
| 6 | 16.608 | 26.30% |
| 7 | 19.92 | 34.30% |
| 8 | 21.488 | 20.10% |
| 9 | 22.113 | 19.30% |

In some embodiments, the fumarate of the compound A in the crystalline form is a crystalline form E, an X-ray powder diffraction of which, using Cu-Kα radiation, is substantially as shown in FIG. 9-1.

In some embodiments, the fumarate of the compound A in the crystalline form is a crystalline form E, a DSC profile of which has endothermic peaks at 222±3°C, 230±3°C, and 240±3°C. In some embodiments, the fumarate of the compound A in the crystalline form is a crystalline form E, a DSC profile of which has an exothermic peak at 159±3 °C and endothermic peaks at 32±3 °C, 222±3 °C, 230±3 °C, and 240±3 °C.

In some embodiments, the fumarate of the compound A in the crystalline form is a crystalline form E, a DSC profile of which is substantially as shown in FIG. 9-2.

In some embodiments, the crystalline form E is a fumarate monohydrate of the compound A in the crystalline form, wherein a molar ratio of the compound A, fumaric acid, and water is 1:1:1.

In some embodiments, the pharmaceutically acceptable salt of the compound A in the crystalline form is selected from citrate. In some embodiments, a molar ratio of a free base of the compound A and citric acid in the citrate of the compound A in the crystalline form is 1:1. In some embodiments, the citrate of the compound A in the crystalline form is a hydrate.

In some embodiments, the citrate of the compound A in the crystalline form is a crystalline form F, an X-ray powder diffraction pattern of which, using Cu-Kα radiation and expressed as 2θ angles (°), has characteristic peaks at 10.1±0.2°, 15.5±0.2°, 17.1±0.2°, 19.4±0.2°, 20.0±0.2°, and 21.5±0.2°.

In some embodiments, the citrate of the compound A in the crystalline form is a crystalline form F, an X-ray powder diffraction pattern of which, using Cu-Kα radiation and expressed as 2θ angles (°), has characteristic peaks at 10.1±0.2°, 11.1±0.2°, 12.5±0.2°, 15.5±0.2°, 17.1±0.2°, 18.4±0.2°, 19.4±0.2°, 20.0±0.2°, 21.5±0.2°, 23.0±0.2°, and 24.2±0.2°.

In some embodiments, the citrate of the compound A in the crystalline form is a crystalline form F, an X-ray powder diffraction pattern of which, using Cu-Kα radiation and expressed as 2θ angles (°), has characteristic peaks at 10.1+0.2°, 11.1±0.2°, 12.5±0.2°, 13.0±0.2°, 14.1±0.2°, 15.5±0.2°, 16.6±0.2°, 17.1±0.2°, 17.5±0.2°, 18.4±0.2°, 19.4±0.2°, 20.010.2', 20.5±0.2°, 21.5+0.2°, 23.0±0.2°, 24.2±0.2°, and 24.7±0.2°.

In some embodiments, the citrate of the compound A in the crystalline form is a crystalline form F, an X-ray powder diffraction pattern of which, using Cu-Kα radiation and expressed as 2θ angles (°), has diffraction peaks with peak positions and relative peak intensities shown in Table 9 below.

**Table 9 Data of X-ray Powder Diffraction Pattern of Crystalline Form F**

| No. | 2θ Angle (°) | Relative Peak Intensity (%) | No. | 2θ Angle (°) | Relative Peak Intensity (%) |
|---|---|---|---|---|---|
| 1 | 10.072 | 58.50% | 10 | 18.429 | 41.70% |
| 2 | 11.124 | 44.60% | 11 | 19.391 | 87.00% |
| 3 | 12.471 | 31.90% | 12 | 19.982 | 100.00% |
| 4 | 12.973 | 20.20% | 13 | 20.49 | 31.20% |
| 5 | 14.126 | 14.40% | 14 | 21.462 | 51.10% |
| 6 | 15.51 | 89.80% | 15 | 23.024 | 60.40% |
| 7 | 16.617 | 28.20% | 16 | 23.326 | 37.60% |
| 8 | 17.052 | 97.00% | 17 | 24.198 | 51.40% |
| 9 | 17.536 | 19.50% | 18 | 24.681 | 35.10% |

In some embodiments, the citrate of the compound A in the crystalline form is a crystalline form F, an X-ray powder diffraction of which, using Cu-Kα radiation, is substantially as shown in FIG. 10-1.

In some embodiments, the citrate of the compound A in the crystalline form is a crystalline form F, a DSC profile of which has three endothermic peaks at 30±3 °C, 198±3 °C, and 248±3 °C.

In some embodiments, the citrate of the compound A in the crystalline form is a crystalline form F, a DSC profile of which is substantially as shown in FIG. 10-2.

In some embodiments, the crystalline form F is a hydrate of a citrate of the compound A in the crystalline form, wherein a molar ratio of the compound A to citric acid is 1:1.

In some embodiments, the pharmaceutically acceptable salt of the compound A in the crystalline form is selected from malate. In some embodiments, a molar ratio of malic acid to a free base of the compound A in the malate of the compound A in the crystalline form is 1:1. In some embodiments, the malate of the compound A in the crystalline form is a hydrate. In some embodiments, the malate of the compound A in the crystalline form is a monohydrate.

In some embodiments, the malate of the compound A in the crystalline form is a crystalline form G, an X-ray

powder diffraction pattern of which, using Cu-Kα radiation and expressed as 2θ angles (°), has characteristic peaks at 4.0 ±0.2°, 11.4±0.2°, 12.4±0.2°, 19.6±0.2°, 20.4±0.2°, 21.1±0.2°, and 22.5+0.2°.

In some embodiments, the malate of the compound A in the crystalline form is a crystalline form G, an X-ray powder diffraction pattern of which, using Cu-Kα radiation and expressed as 2θ angles (°), has characteristic peaks at 4.0 ±0.2°, 9.8±0.2°, 10.5±0.2°, 11.4±0.2°, 12.4±0.2°, 13.2±0.2°, 14.3±0.2°, 15.9±0.2°, 17.0±0.2°, 19.0±0.2°, 19.6 ±0.2°, 20.4±0.2°, 21.1±0.2°, 22.5±0.2°, 24.1±0.2°, and 25.4±0.2°.

In some embodiments, the malate of the compound A in the crystalline form is a crystalline form G, an X-ray powder diffraction pattern of which, using Cu-Kα radiation and expressed as 2θ angles (°), has diffraction peaks with peak positions and relative peak intensities shown in Table 10 below.

**Table 10 Data of X-ray Powder Diffraction Pattern of Crystalline Form G**

| No. | 2θ Angle (°) | Relative Peak Intensity (%) | No. | 2θ Angle (°) | Relative Peak Intensity (%) |
|---|---|---|---|---|---|
| 1 | 4.045 | 55.60% | 11 | 19.028 | 17.30% |
| 2 | 9.802 | 15.50% | 12 | 19.636 | 50.40% |
| 3 | 10.498 | 16.90% | 13 | 20.411 | 43.70% |
| 4 | 11.449 | 35.40% | 14 | 21.052 | 100.00% |
| 5 | 12.395 | 34.60% | 15 | 22.536 | 74.30% |
| 6 | 13.225 | 9.40% | 16 | 22.664 | 45.60% |
| 7 | 14.271 | 12.20% | 17 | 24.081 | 20.50% |
| 8 | 15.889 | 20.20% | 18 | 25.386 | 22.80% |
| 9 | 16.96 | 27.40% | 19 | 25.704 | 20.40% |
| 10 | 17.278 | 27.90% | 20 | 26.08 | 18.60% |

In some embodiments, the malate of the compound A in the crystalline form is a crystalline form G, an X-ray powder diffraction pattern of which, using Cu-Kα radiation, is substantially as shown in FIG. 11-1.

In some embodiments, the malate of the compound A in the crystalline form is a crystalline form G, a DSC profile of which has endothermic peaks at 57±3 °C, 175±3 °C, 191±3 °C, and 238 ±3 °C.

In some embodiments, the malate of the compound A in the crystalline form is a crystalline form G, a DSC profile of which is substantially as shown in FIG. 11-2.

In some embodiments, the crystalline form G is a malate monohydrate of the compound A, wherein a molar ratio of the compound A, malic acid, and water is 1:1:1.

In a second aspect, the present application further provides a pharmaceutical composition comprising the compound A or the pharmaceutically acceptable salt thereof in the crystalline form as described above. In some embodiments, the present application provides a pharmaceutical composition comprising one or more of the crystalline form A-I, the crystalline form A-II, the crystalline form A-III, the crystalline form A-IV, the crystalline form B, the crystalline form C, the crystalline form D-I, the crystalline form E, the crystalline form F, and the crystalline form G as described above, and optionally one or more pharmaceutically acceptable excipients. Preferably, the pharmaceutical composition comprises one or more of the crystalline form A-I, the crystalline form B, and the crystalline form C as described above, and optionally one or more pharmaceutically acceptable excipients.

In a third aspect, the present application further provides a use of the compound A or the pharmaceutically acceptable salt thereof in the crystalline form as described above in the preparation of an anti-tumor drug. In some embodiments, the present application provides a use of the crystalline form A-I, the crystalline form A-II, the crystalline form A-III, the crystalline form A-IV, the crystalline form B, the crystalline form C, the crystalline form D-I, the crystalline form E, the crystalline form F, the crystalline form G, or the pharmaceutical composition as described above in the preparation of an anti-tumor drug.

In a fourth aspect, the present application further provides a method for treating tumors with the compound A or the pharmaceutically acceptable salt thereof in the crystalline form as described above, comprising administering a therapeutically effective amount of the compound A or the pharmaceutically acceptable salt thereof in the crystalline form to a subject or patient in need of treatment. In some embodiments, the present application further provides a method for treating tumors, comprising administering a therapeutically effective amount of the crystalline form A-I, the crystalline form A-II, the crystalline form A-III, the crystalline form A-IV, the crystalline form B, the crystalline form C, the crystalline form D-I, the crystalline form E, the crystalline form F, the crystalline form G, or the pharmaceutical composition as described above to a subject or patient in need of treatment.

In a fifth aspect, the present application further provides the compound A or the pharmaceutically acceptable salt thereof in the crystalline form as described above for use in the treatment of tumors. In some embodiments, the present application further provides the crystalline form A-I, the crystalline form A-II, the crystalline form A-III, the crystalline form A-IV, the crystalline form B, the crystalline form C, the crystalline form D-I, the crystalline form E, the crystalline form F, the crystalline form G, or the pharmaceutical composition as described above for use in the treatment of tumors.

In a sixth aspect, the present application further provides a use of the compound A or the pharmaceutically acceptable salt thereof in the crystalline form as described above in the treatment of tumors. In some embodiments, the present application provides a use of the crystalline form A-I, the crystalline form A-II, the crystalline form A-III, the crystalline form A-IV, the crystalline form B, the crystalline form C, the crystalline form D-I, the crystalline form E, the crystalline form F, the crystalline form G, or the pharmaceutical composition as described above in the treatment of tumors.

Within the above aspects:

In some embodiments, the tumor is selected from a sensitive tumor or a drug-resistant tumor. Preferably, the sensitive tumor or the drug-resistant tumor is selected from a solid tumor or a hematological tumor. Preferably, the solid tumor is selected from fibrosarcoma, salivary gland carcinoma, liver cancer, colorectal cancer, bladder cancer, laryngeal cancer, breast cancer, prostate cancer, glioma, ovarian cancer, head and neck squamous cell carcinoma, cervical cancer, esophageal cancer, renal cancer, pancreatic cancer, skin cancer, lymphoma, gastric cancer, multiple myeloma, brain tumors, lung cancer, or melanoma; and the hematological tumor is selected from acute myeloid leukemia. More preferably, the sensitive tumor or the drug-resistant tumor is selected from breast cancer, lung cancer, colorectal cancer, liver cancer, ovarian cancer, melanoma, or acute myeloid leukemia. In some embodiments, the breast cancer is selected from HR+/HER2-breast cancer, triple-negative breast cancer, or HR-/HER2+ breast cancer; the acute myeloid leukemia is selected from myelomonocytic leukemia; and the lung cancer is selected from non-small cell lung cancer.

In some embodiments, the tumor is selected from the drug-resistant tumor. The drug-resistant tumor is selected from tumors with primary drug resistance or tumors with acquired drug resistance.

In some embodiments, the drug-resistant tumor is selected from CDK4/6 inhibitor-resistant tumors, further selected from tumors with primary CDK4/6 inhibitor resistance or tumors with acquired CDK4/6 inhibitor resistance. The CDK4/6 inhibitor is selected from Palbociclib, Abemaciclib, Ribociclib, or Dalpiciclib, preferably Palbociclib.

In some embodiments, the CDK4/6 inhibitor-resistant tumor is selected from breast cancer or liver cancer, further preferably Palbociclib-resistant breast cancer or liver cancer, and further preferably breast cancer or liver cancer with primary Palbociclib resistance, or breast cancer or liver cancer with acquired Palbociclib resistance. The breast cancer with primary Palbociclib resistance or the breast cancer with acquired Palbociclib resistance is selected from HR+/HER2-breast cancer, triple-negative breast cancer, or HR-/HER2+ breast cancer with primary or acquired Palbociclib resistance.

The compound A or the pharmaceutically acceptable salt thereof in the crystalline form as described herein may be administered as monotherapy or in combination with other clinically/pharmaceutically acceptable anti-tumor agents, biological therapies, radiation therapies, and/or traditional Chinese medicine therapies. In some embodiments, the other anti-tumor agents are selected from endocrine therapy drugs, such as Anastrozole, Letrozole, Exemestane, Tamoxifen, Toremifene, Fulvestrant, Megestrol, Fluoxymesterone, and Ethinylestradiol, preferably Fulvestrant.

Accordingly, the present application provides a use of the compound A or the pharmaceutically acceptable salt thereof in the crystalline form in combination with an additional clinically/pharmaceutically acceptable drug for the preparation of an anti-tumor drug. Also, the present application provides an anti-tumor drug comprising the compound A or the pharmaceutically acceptable salt thereof in the crystalline form and an additional clinically/pharmaceutically acceptable drug. The additional drug and the compound A or the pharmaceutically acceptable salt thereof in the crystalline form may be formulated in the same dosage unit or in different dosage units that are packaged as a combination, such as a kit product. In some embodiments, the additional drug is selected from endocrine therapy drugs, such as Anastrozole, Letrozole, Exemestane, Tamoxifen, Toremifene, Fulvestrant, Megestrol, Fluoxymesterone, and Ethinylestradiol, preferably Fulvestrant.

The inventors conducted studies on the crystalline state of the compound A and the pharmaceutically acceptable salt thereof. The results demonstrated that the compound A failed to form crystalline salts with maleic acid or phosphoric acid; salt samples formed with sulfuric acid, methanesulfonic acid, and tartaric acid exhibited poor crystallinity; the compound A did not fully react with succinic acid; the hydrochloride salt, p-toluenesulfonate, citrate, fumarate, and malate of the compound A were solvates/hydrates; and only the benzenesulfonate of the compound A was an anhydrous crystalline form. Based on solid state characterization, crystalline form B (a hydrate) of the hydrochloride salt of the compound A and crystalline form C (an anhydrous crystalline form) of the benzenesulfonate of the compound A were the preferred salt forms.

Further comparisons of solubility and stability among crystalline form B (a hydrate) of the hydrochloride salt of the compound A, crystalline form C (an anhydrous crystalline form) of the benzenesulfonate of the compound A, and crystalline form A-I of the free base of the compound A revealed that: crystalline form A-I of the free base of the compound A exhibited low solubility in water, whereas crystalline forms of the two salts exhibited significantly higher solubility in water; crystalline form A-I of the free base of the compound A and crystalline forms of the two salts exhibited insignificant

difference of solubility in other three bio-relevant medium, and exhibited a solubility of more than 5 mg/mL in SGF. Solid stability results indicated that crystalline form C of the benzenesulfonate of the compound A may transform into a hydrate under 92.5% RH condition; crystalline form A-I of the free base of the compound A, crystalline form B of the hydrochloride salt of the compound A, and crystalline form C of the benzenesulfonate of the compound A all demonstrated certain physical and chemical stability after being placed for 7 days under the following three conditions: 60 °C, 92.5% RH, and 40 °C/75% RH.

Preclinical studies demonstrated that the compound A or the pharmaceutically acceptable salt thereof in the crystalline form can significantly inhibit the proliferation of various tumor cells such as breast cancer, colorectal cancer, ovarian cancer, melanoma, liver cancer, lung cancer, acute myeloid leukemia cells, etc., and also exhibited potent inhibitory effects on Palbociclib-resistant (including both primary and acquired Palbociclib resistant) breast cancer and liver cancer.

Therefore, the compound A or the pharmaceutically acceptable salt thereof in the crystalline form has good clinical application prospects for treating advanced malignancies and provides a novel drug option for the treatment of CDK4/6 inhibitor-resistant tumors.

Definitions and Explanations

Unless otherwise stated, the following terms and phrases used herein are intended to have the meanings set forth below. A specific phrase or term that is not explicitly defined should not be considered to be indefinite or unclear but should be interpreted according to the ordinary meaning thereof.

The compound A or the pharmaceutically acceptable salt thereof in the crystalline form as described herein includes anhydrous and solvent-free forms, hydrate forms, solvate forms, and eutectic crystalline forms of the compound A or the pharmaceutically acceptable salt thereof.

The term "room temperature" refers to the room temperature conventionally understood in the art, generally ranging from 10 °C to 30 °C, preferably 25±5 °C.

The term "substantially" or "substantially as shown in figure", when describing the X-ray powder diffraction (XRPD) pattern, means that at least 50%, at least 60%, at least 70%, at least 80%, at least 90%, at least 95%, at least 96%, at least 97%, at least 98%, or at least 99% of the peaks in the XRPD pattern of a substantially pure certain crystalline form appear in the given pattern. Further, as the content of a certain crystalline form in a product decreases, some diffraction peaks attributed to that crystalline form in the XRPD pattern may diminish due to the detection sensitivity of the instrument. Additionally, for whatever given crystalline form, minor positional deviations in peaks may be appeared, and are well-recognized in crystallography. For example, due to temperature variations, sample displacement, or instrument calibration, etc. during sample analysis, peak positions may shift, and the measurement deviation for $2\theta$ values is sometimes approximately ±0.3°, and typically ±0.2°. Therefore, when determining the structure of each crystalline form, such deviations must be taken into consideration. The term "substantially" or "substantially as shown in figure" is also intended to encompass such variations in peak positions, i.e., ±0.3°, preferably ±0.2°.

The term "substantially" or "substantially as shown in figure", when describing the DSC profile or the TGA profile, means that the deviation in the onset temperature of the thermal transition, the peak temperature of a endothermic peak, the peak temperature of an exothermic peak, the melting point, the weight loss start temperature, or the weight loss end temperature, etc. is typically ± 5 °C, and commonly ± 3 °C during continuous analysis for the same crystalline form of the same compound.

The term "tumor" includes benign tumors, malignant tumors, and borderline tumors. The malignant tumors are collectively referred to as cancers.

The term "preventing" used herein means that the compound or drug (e.g., the combination product claimed herein), when used for a disease or disorder (e.g., cancer), can reduce the frequency of a symptom of a medical condition or delays the onset thereof in a subject compared to a subject to which the compound or drug is not administered.

The term "treating/treatment" used herein refers to alleviating, mitigating, or improving symptoms of a disease or disorder, improving underlying symptoms caused by metabolism, or inhibiting a disease or disorder, e.g., preventing progression of a disease or disorder, alleviating a disease or disorder, inducing remission of a disease or disorder, mitigating conditions caused by a disease or disorder, or preventing symptoms of a disease or disorder.

The term "pharmaceutically acceptable carrier" or "pharmaceutically acceptable excipient" refers to carriers or excipients that do not cause significant irritation to an organism and do not impair the biological activity or performance of the active compound.

The terms "subject" and "patient" include, but are not limited to, mammals (e.g., mice, rats, cats, monkeys, dogs, horses, pigs) and human beings.

The term "pharmaceutical composition" refers to a mixture consisting of one or more compounds of the present application or salts thereof and pharmaceutically acceptable excipients. The pharmaceutical composition described herein may be prepared by combining the compound of the present application with suitable pharmaceutically acceptable

excipients. The pharmaceutical composition of the present application may be prepared using methods well-known in the art, such as conventional mixing process, dissolving process, granulation process, sugar coating process for tablets/pills, fine grinding process, emulsification process, lyophilization process, etc.

For a drug or pharmacologically active agent, the term "therapeutically effective amount" refers to a sufficient amount of a drug or agent that can achieve the intended effect without toxicity. The determination of the effective amount varies among individuals and depends on factors such as the recipient's age, general condition, and the specific active substance. Appropriate effective amount for individual cases may be determined by a person skilled in the art through routine experimentation.

All solvents used in the present application are commercially available and can be used without further purification.

To provide concise description, the term "about/approximately" is not used to modify some quantitative data herein. It should be understood that every numerical value provided, whether or not modified by the term "about/approximately", includes not only the explicitly stated value (the given value) but also approximations of the given value reasonably inferred by a person of ordinary skill in the art, encompassing equivalents and approximations of the given value arising from experimental and/or measurement conditions. Such approximations preferably fall within the given value ±20%, 15%, ±10%, 18%, ±6%, ±5%, ±4%, ±3%, ±2%, or ±1%.

## BRIEF DESCRIPTION OF DRAWINGS

FIG. 1-1 XRPD pattern of crystalline form A-I of free base of compound A.
FIG. 1-2 TGA profile of crystalline form A-I of free base of compound A.
FIG. 1-3 DSC profile of crystalline form A-I of free base of compound A.
FIG. 2-1 XRPD pattern of crystalline form A-II of free base of compound A.
FIG. 2-2 TGA profile of crystalline form A-II of free base of compound A.
FIG. 2-3 DSC profile of crystalline form A-II of free base of compound A.
FIG. 3-1 XRPD pattern of crystalline form A-III of free base of compound A.
FIG. 3-2 TGA profile of crystalline form A-III of free base of compound A.
FIG. 3-3 DSC profile of crystalline form A-III of free base of compound A.
FIG. 4-1 XRPD pattern of crystalline form A-IV of free base of compound A.
FIG. 4-2 TGA profile of crystalline form A-IV of free base of compound A.
FIG. 4-3 DSC profile of crystalline form A-IV of free base of compound A.
FIG. 5-1 XRPD pattern of crystalline form B of hydrochloride salt of compound A.
FIG. 5-2 TGA and DSC profiles of crystalline form B of hydrochloride salt of compound A.
FIG. 6-1 XRPD pattern of crystalline form C of benzenesulfonate of compound A.
FIG. 6-2 TGA and DSC profiles of crystalline form C of benzenesulfonate of compound A.
FIG. 7-1 XRPD pattern of crystalline form D-I of p-toluenesulfonate of compound A.
FIG. 7-2 TGA and DSC profiles of crystalline form D-I of p-toluenesulfonate of compound A.
FIG. 8-1 XRPD pattern of crystalline form D-II of p-toluenesulfonate of compound A.
FIG. 8-2 TGA and DSC profiles of crystalline form D-II of p-toluenesulfonate of compound A.
FIG. 9-1 XRPD pattern of crystalline form E of fumarate of compound A.
FIG. 9-2 TGA and DSC profiles of crystalline form E of fumarate of compound A.
FIG. 10-1 XRPD pattern of crystalline form F of citrate of compound A.
FIG. 10-2 TGA and DSC profiles of crystalline form F of citrate of compound A.
FIG. 11-1 XRPD pattern of crystalline form G of malate of compound A.
FIG. 11-2 TGA and DSC profiles of crystalline form G of malate of compound A.

## DESCRIPTION OF EMBODIMENTS

The drugs, reagents, etc. involved in the following specific implementations are commercially available or prepared by conventional methods of the related art.

Hereinafter, the present application will be further explained with conjunction of specific examples. It should be understood that these examples are only used to illustrate the present application but not to limit the scope of the present application. The experimental methods without specific conditions specified in the following examples are typically carried out under conventional conditions or conditions recommended by the manufacturer. Unless otherwise defined, all professional and scientific terms used herein have the same meanings as those familiar to those skilled in the art. In addition, any methods and materials that are similar or equivalent to the content recorded herein can be applied to the

method of the present application. The preferred implementations and materials illustrated herein are for exemplary purposes only.

Detection Method

1. X-ray Powder Diffraction (XRPD)

Instrument: Bruker D8 Advance Diffractometer

Detection method: the sample was spread on a non-reflective sample pan, gently pressed flat, and detected under the following specific conditions: Cu-Kα radiation; Tube Voltage: 40 kV; Tube Current: 40 mA; 2θ Scan Range: 3° to 40° or 3° to 30°; Scan Step: 0.02°; Exposure Time: 0.2 second.

2. Differential Scanning Calorimetry (DSC)

Instrument: TA Instruments Q200 DSC

Detection method: 0.5 to 5 mg of the sample was placed in a perforated aluminum pan and heated at a rate of 10°C/min to the final temperature, with purging nitrogen at 50 mL/min in the furnace.

3. Thermogravimetric Analysis (TGA)

Instrument: TA Instruments Q500 TGA

Detection method: 1 to 10 mg of the sample was placed in a tared open aluminum sample pan and automatically weighed in the TGA furnace. The sample was heated at a rate of 10°C/min to the final temperature, with purging nitrogen at a rate of 25 mL/min in the sample chamber and 40 mL/min in the balance chamber.

4. Dynamic Vapor Sorption (DVS)

Instrument: TA Instruments Q5000 SA

Detection method: 20 to 50 mg of the sample was placed in a tared sample basket and automatically weighed, and DVS testing was performed according to the parameters in Table 7: Step Time: 60 min; Equilibration Time per RH%: 1 hour; Total Gas Flow Rate: 200 sccm; Gradient mode: 0%-90%-0% humidity variation, with a humidity variation amount of 10% for each gradient level between 0% and 90%, and gradient endpoint was determined by dm/dt < 0.01% maintained for 10 minutes.

5. Nuclear Magnetic Resonance (1H-NMR)

5.1 Studies on crystalline form of free base

Detection method: 1H-NMR spectrum was measured by nuclear magnetic resonance spectrometer: Bruker Ascend 500 or WNMR-I 400 with Full-frequency excitation, 30 ppm spectral width, single pulse, 30° flip angle, 16 scans, digital quadrature detection, temperature controlled at 298 K, and deuterated chloroform agent.

5.2 Salt formation assay

Detection method: 1H-NMR spectrum was measured by nuclear magnetic resonance spectrometer: Bruker 400MHz with DMSO-d6 solvent, full-frequency excitation, 30 ppm spectral width, single pulse, 30° flip angle, 4 scans, digital quadrature detection, and temperature controlled at 295 K.

6. Solubility assay

At room temperature, a solvent was added in portions into a known amount of sample. Stirring or sonication was applied until the sample was dissolved to reach visual clarification. The amount of the solvent consumed was recorded. If the sample remained undissolved at a specific concentration, its solubility was indicated as "<" the specific concentration.

7. Preparation of biological media

| Name | Preparation Method |
|---|---|
| Simulated gastric fluid (SGF) | 7.65 mL of concentrated hydrochloric acid was taken, and thereto were added about 800 mL of water, 10 g of pepsin, and 2.0 g of sodium chloride, the resulting mixture was shaked well, and then diluted with water to 1000 mL. |
| Fasted-state simulated intestinal fluid (FaSSIF) | 3.95 g of potassium dihydrogen phosphate, 1.61 g of sodium taurocholate, 0.56 g of lecithin, and 7.7 g of potassium chloride were placed in a 1000 mL volumetric flask, dissolved with water, and diluted to a volume of 1000 mL, the pH value of the resulting mixture was measured with a pH meter, and adjusted to 6.5 with 10% phosphoric acid and 1% sodium hydroxide. |
| Fed-state simulated intestinal fluid (FeSSIF) | 8.65 g of glacial acetic acid, 8.06 g of sodium taurocholate, 2.81 g of lecithin, and 15.2 g of potassium chloride were placed in a 1000 mL volumetric flask, dissolved with water, and diluted to a volume of 1000 mL, the pH value of the resulting mixture was measured with a pH meter, and adjusted to 5.0 with 10% acid and 1% sodium hydroxide. |

**Preparation Example 1: Preparation of compound A**

Compound A was prepared according to the method described in Example 2 of WO2021139817A1. The specific method is as follows:

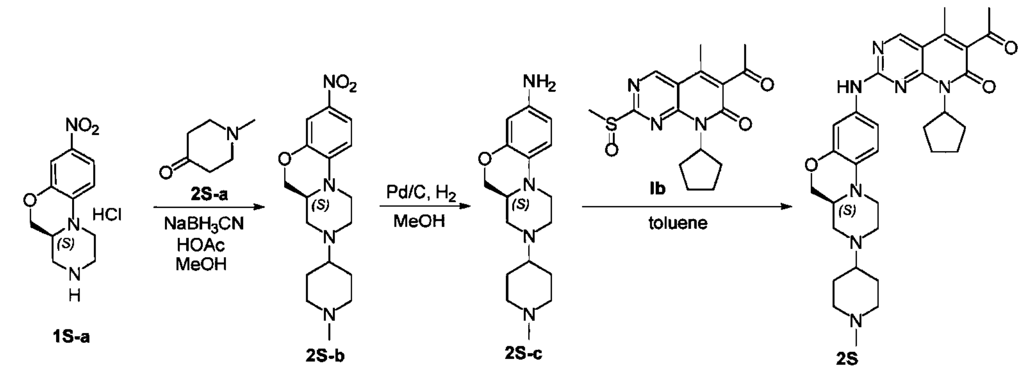


Compound 1S-a (500 mg, 1.84 mmol), N-methylpiperidone (2S-a, 416 mg, 3.68 mmol), and 2 drops of acetic acid were added to methanol (15 mL). The mixture was stirred at room temperature for 1 hour. Then, sodium cyanoborohydride (231 mg, 3.68 mmol) was added, and the reaction mixture was stirred overnight at room temperature. The completion of the reaction was monitored by TLC. Methanol in the reaction mixture was removed under reduced pressure. Then, water (10 mL) was added, and the mixture was extracted with dichloromethane (3×20 mL). The combined organic phase was washed with saturated brine, dried over anhydrous sodium sulfate, and filtered, and the filtrate was concentrated under reduced pressure. The resulting crude product was separated and purified via silica gel column chromatography (dichloromethane: methanol = 30:1) to give a yellow solid compound 2S-b (500 mg), which was directly used in the next reaction step.

Compound 2S-b (500 mg, 1.50 mmol) and palladium-on-carbon catalyst (10%, 70 mg) were added to methanol (15 mL) at room temperature. The reaction mixture was stirred for 3 hours under a hydrogen atmosphere at 1 atm and room temperature. The completion of the reaction was monitored by TLC. The reaction mixture was filtered through diatomite,

and the filtrate was concentrated under reduced pressure to obtain the crude product. This crude product was separated and purified via silica gel column chromatography (dichloromethane: methanol = 20:1) to give a brown solid compound 2S-c (280 mg, with a yield of 62%).

Compound 2S-c (280 mg, 0.93 mmol) and compound Ib (310 mg, 0.93 mmol) were dissolved in toluene (8 mL). The reaction mixture was stirred at 90°C for 3 hours. The completion of the reaction was monitored by TLC. After the reaction solution was cooled to room temperature, it was concentrated under reduced pressure. The resulting crude product was separated and purified via silica gel column chromatography (dichloromethane: methanol = 20:1) to give a yellow solid compound 2S (180 mg, with a yield of 34%).

$^1H$ NMR(500MHz,DMSO-$d_6$) δ 9.86 (bs,1H), 8.90(s,1H), 7.16(d,J=2.1Hz,1H), 7.06(d,J=8.9 Hz,1H), 6.81(d,J=8.9Hz,1H), 5.91-5.70 (m,1H), 4.23(dd,J=10.5,2.4Hz,1H), 3.89(dd,J=10.5, 9.0Hz,1H), 3.69(d,J=11.3Hz,1H),3.00-2.88(m,3H), 2.79(d,J=11.2Hz,2H), 2.59-2.53(m,1H), 2.41 (s,3H), 2.34-2.16(m,7H), 2.14(s,3H), 1.94-1.73(m,9H), 1.62-1.51(m,2H), 1.48-1.36(m,2H)ppm. MS m/z 572.8 $[M+H]^+$.

The compound 2S is the compound A of the present application. Detection shows that the aforementioned yellow solid compound is amorphous.

**Example 1: Preparation of Crystalline Form A-I of Free Base of Compound A**

100 mL of n-butanol and 10 mL of anisole were added to approximately 5 g of the sample obtained from Preparation Example 1. The mixture was warmed to 105±5 °C and stirred until dissolved. The solution was cooled to 85±5 °C for crystallization for 1.5±0.5 hours, and further cooled to 15±5 °C for crystallization for 2.0±0.5 hours. The resulting solid was filtered with suction, washed with 10 mL of n-butanol and then with 10 mL of n-heptane. The filter cake was vacuum-dried at 60±5 °C for 4±2 hours to obtain the product.

The solid was taken for XRPD characterization, and this crystalline solid was designated as crystalline form A-I. The XRPD pattern thereof is as shown in FIG. 1-1, with characterization data provided in Table 11. TGA results (see FIG. 1-2) indicate a weight loss of 0.30% from room temperature to 150 °C, demonstrating that this crystalline form is an anhydrate; and the decomposition temperature of this crystalline form is approximately 342 °C. The DSC profile (see FIG. 1-3) shows a melting point at approximately 256 °C. DVS testing results demonstrate that the crystalline form exhibits a weight change of approximately 0.17% across 0%-90% RH, indicating non-hygroscopic properties.

**Table 11 Data of X-ray Powder Diffraction Pattern of Crystalline Form A-I**

| No. | 2θ angle (°) | D value | Relative peak intensity (%) | Peak intensity |
|---|---|---|---|---|
| 1 | 5.24 | 16.8382 | 62.6 | 6301 |
| 2 | 9.62 | 9.1897 | 12.5 | 1253 |
| 3 | 10.44 | 8.4701 | 11.7 | 1177 |
| 4 | 11.46 | 7.7134 | 27.1 | 2729 |
| 5 | 12.89 | 6.8645 | 14.1 | 1420 |
| 6 | 14.19 | 6.2382 | 10 | 1002 |
| 7 | 15.77 | 5.615 | 39.1 | 3938 |
| 8 | 16.36 | 5.4139 | 34.1 | 3427 |
| 9 | 18.69 | 4.7447 | 14.6 | 1471 |
| 10 | 20.28 | 4.3748 | 100 | 10061 |
| 11 | 20.63 | 4.3024 | 91.3 | 9184 |
| 12 | 22.07 | 4.0241 | 47.2 | 4744 |
| 13 | 25.89 | 3.439 | 25.6 | 2577 |

The inventors conducted systematic studies on the preparation method of the crystalline form A-I of the free base. Other exemplary preparation methods include:

| No. | Method | Solvent 1 | Solvent 2 | Solvent 1/ Solvent 2 (mL) | Result Analysis |
|---|---|---|---|---|---|
| 1 | Approximately 30 mg of the sample obtained in Preparation Example 1 was taked, and thereto was added the corresponding solvent, the resulting mixture was warmed to 70 °C to dissolve until a clear solution is obtained, the solution was stirred at room temperature for 2 hours to precipitate a solid (if no solid was precipitated, then the solution was cooled to 4 °C and further stirred until a solid was precipitated), followed by centrifuging, and vacuum-drying at 40 °C for 1 day. | Methanol | - | 8 | crystalline form A-I |
| 2 | | Tetrahydrofuran | - | 1 | crystalline form A-I |
| 3 | | Tetrahydrofuran | Ethyl acetate | 2.0/1.0 | crystalline form A-I |
| 4 | | Dichloromethane | Acetonitrile | 1.0/0.6 | crystalline form A-I |
| 5 | | Chloroform | Butanone | 1.6/2.0 | crystalline form A-I |
| 6 | Approximately 30 mg of the sample obtained in Preparation Example 1 was taken, thereto was added Solvent 1, and dissolved in a 70 °C water bath until a clear solution is obtained, Solvent 2 was added dropwise into Solvent 1, and stirred to precipitate a solid (if no solid was precipitated, then the solution was cooled to 4 °C and stirred overnight until a solid was precipitated), followed by centrifuging, and vacuum-drying at 40 °C overnight. | Methanol | Methyl tert-butyl ether | 8.0/8.0 | crystalline form A-I |
| 7 | | Tetrahydrofuran | n-Heptane | 1.0/3.0 | crystalline form A-I |
| 8 | | Dichloromethane | Butyl acetate | 0.5/1.0 | crystalline form A-I |
| 9 | Approximately 30 mg of the sample obtained in Preparation Example 1 was taken, thereto was added | Tetrahydrofuran | Isopropyl ether | 1.0/3.0 | crystalline form A-I |
| 10 | Solvent 1, and dissolved in a 70°C water bath to obtain a clear solution, the resulting solution was added dropwise into Solvent 2, and stirred to precipitate a solid (if no solid was precipitated, then the solution was cooled to 4 °C and stirred overnight until a solid was precipitated), followed by centrifuging and vacuum-drying at 40 °C overnight. | Dichloromethane | Methyl tert-butyl ether | 0.5/2.0 | crystalline form A-I |

**Example 2: Preparation of crystalline form A-II of Free Base of Compound A (Trifluoroethanol Solvate of Compound A)**

Approximately 20 mg of the sample obtained from Preparation Example 1 was taken. 1.0 mL of trifluoroethanol and 0.5 mL of tetrahydrofuran were added to dissolve the sample, a clear solution was obtained, and the solution was rapidly dried by rotary evaporation at room temperature to obtain the product.

A sample was taken for XRPD characterization (as shown in FIG. 2-1), and the characterization data are shown in Table 12. The obtained crystalline solid is designated as a crystalline form A-II of the free base. TGA analysis (as shown in FIG. 2-2) indicated that this crystalline form has a weight loss of 16.9% before 150 °C and a decomposition temperature at 343°C, demonstrating that this crystalline form is a trifluoroethanol solvate of compound A (containing one trifluoroethanol molecule, with a theoretical proportion of 14.9%). The DSC profile (FIG. 2-3) shows that this crystalline form has a desolvation peak at 100 to 130 °C and a melting point at approximately 256 °C (the melting point of crystalline form A-I).

**Table 12 Data of X-ray Powder Diffraction Pattern of crystalline form A-II**

| No. | 2θ angle (°) | D value | Relative peak intensity (%) | Peak intensity |
|---|---|---|---|---|
| 1 | 4.08 | 21.6154 | 100 | 5894 |

(continued)

| No. | 2θ angle (°) | D value | Relative peak intensity (%) | Peak intensity |
|---|---|---|---|---|
| 2 | 16.17 | 5.4771 | 37.8 | 2230 |
| 3 | 16.79 | 5.2746 | 21.4 | 1262 |
| 4 | 17.65 | 5.0196 | 5.2 | 305 |
| 5 | 20.13 | 4.4072 | 47 | 2773 |
| 6 | 20.68 | 4.2909 | 70.9 | 4176 |

**Example 3: Preparation of Crystalline Form A-III of Free Base of compound A (Dimethyl Sulfoxide Solvate of Compound A)**

Approximately 30 mg of the sample obtained from Preparation Example 1 was taken. 5.0 mL of dimethyl sulfoxide was added, and the sample was dissolved at 70°C, and a clear solution was obtained. The solution was transferred to room temperature and stirred for 2 hours to precipitate a solid, which was centrifuged and vacuum-dried at 40°C overnight to obtain the product.

A sample was taken for XRPD characterization (as shown in FIG. 3-1), and the characterization data are shown in Table 13. The obtained crystalline solid was designated as crystalline form A-III. The TGA test (FIG. 3-2) shows that this crystalline form has a weight loss of 12.4% before 150 °C and a decomposition temperature at 342 °C, indicating that crystalline form A-III was a dimethyl sulfoxide solvate of compound A (containing one dimethyl sulfoxide molecule, with a theoretical proportion of 12.0%). The DSC study (-FIG. 33) shows that this crystalline form has a desolvation peak at 100 to 150 °C and a melting point at approximately 256 °C (the melting point of crystalline form A-I of the free base).

**Table 13 Data of X-ray Powder Diffraction Pattern of Crystalline Form A-III**

| No. | 2θ angle (°) | D value | Relative peak intensity (%) | Peak intensity |
|---|---|---|---|---|
| 1 | 6.58 | 13.4138 | 49.9 | 2214 |
| 2 | 13.19 | 6.7043 | 27 | 1195 |
| 3 | 15.2 | 5.8259 | 13.3 | 589 |
| 4 | 16.53 | 5.3592 | 28.8 | 1275 |
| 5 | 20.08 | 4.4188 | 100 | 4434 |
| 6 | 23.01 | 3.8618 | 30.8 | 1364 |
| 7 | 28.17 | 3.1649 | 11.9 | 528 |

**Example 4: Crystalline Form A-IV of Free Base of Compound A**

An appropriate amount of the amorphous sample was taken, heated up to 160°C on a hot stage, and maintained at the temperature for 5 minutes, and then the temperature was lowered back to room temperature to obtain the product.

A sample was taken for XRPD characterization (as shown in FIG. 4-1), and the characterization data are shown in Table 14. The obtained crystalline solid is a crystalline form A-IV. The TGA profile (FIG. 4-2) shows that this crystalline form has a weight loss of 0.8% before 150 °C and a decomposition temperature at 348 °C. The DSC profile (FIG. 4-3) shows that the melting point is approximately 255 °C.

**Table 14 Data of X-ray Powder Diffraction Pattern of Crystalline Form A-IV**

| No. | 2θ angle (°) | D value | Relative peak intensity (%) | Peak intensity |
|---|---|---|---|---|
| 1 | 4.1 | 21.5074 | 11.4 | 2139 |
| 2 | 5.36 | 16.4669 | 43.4 | 8143 |
| 3 | 9.52 | 9.2846 | 20.4 | 3825 |
| 4 | 12.08 | 7.3229 | 20.1 | 3775 |
| 5 | 15.9 | 5.5685 | 40.7 | 7635 |

(continued)

| No. | 2θ angle (°) | D value | Relative peak intensity (%) | Peak intensity |
|---|---|---|---|---|
| 6 | 17.8 | 4.9783 | 19.3 | 3621 |
| 7 | 19.26 | 4.6055 | 8.4 | 1580 |
| 8 | 19.87 | 4.4655 | 17.8 | 3334 |
| 9 | 20.44 | 4.3424 | 100 | 18761 |
| 10 | 20.72 | 4.2828 | 89.1 | 16725 |

**Example 5: Hydrochloride Monohydrate of Compound A**

Approximately 350 mg of compound A was weighed and placed in a reaction flask. 7 mL of methanol was added, and the mixture was stirred at room temperature to obtain a suspension. After adding 1.05 equivalents (eq.) of a 1M aqueous hydrochloric acid solution, the reaction solution remained a suspension, and the stirring was continued overnight. The resulting solid was filtered and vacuum-dried at 50 °C for 6 hours to obtain the product (yield: 75%, product purity: 99.31%).

IC analysis was conducted on the product (Table 15). The results shows that the molar ratio of the free base of compound A to hydrochloric acid was 1:1 (the theoretical content of chloride ions in the monohydrochloride salt is 5.8%). Therefore, the hydrochloride salt of compound A in the crystalline form (designated as crystalline form B) was successfully prepared in this experiment.

**Table 15 IC Results of crystalline form B of Hydrochloride salt of Compound A**

| Parallel samples | Cl-content result (% mass/mass) | Average content (% mass/mass) |
|---|---|---|
| Sample 1 | 5.564 | 5.6 |
| Sample 2 | 5.728 | |

The PLM and XRPD results (FIG. 5-1) show that the sample consists of irregular crystals with a small particle size and an agglomeration phenomenon, but has high crystallinity. The characterization data are shown in Table 16 below. TGA shows a weight loss of approximately 4.4% from room temperature to 130 °C. DSC has two endothermic peaks at 27 °C and 253 °C, which were the desolvation peak and the melting/decomposition peak, respectively (see FIG. 5-2). No significant residual organic solvent was detected by NMR. Therefore, the weight loss was caused by the loss of water, and the hydrochloride salt crystal was a monohydrate (the theoretical water content of the monohydrate is 2.9%), which is defined as a crystalline form B of hydrochloride salt of compound A.

**Table 16 Data of X-ray Powder Diffraction Pattern of Crystalline Form B**

| No. | 2θ angle (°) | D value | Relative peak intensity (%) | Peak intensity | No. | 2θ angle (°) | D value | Relative peak intensity (%) | Peak intensity |
|---|---|---|---|---|---|---|---|---|---|
| 1 | 4.071 | 21.6852 | 27.30% | 451.038 | 11 | 17.441 | 5.08077 | 11.90% | 196.379 |
| 2 | 8.246 | 10.71345 | 20.60% | 340.399 | 12 | 19.104 | 4.642 | 15.30% | 253.567 |
| 3 | 8.906 | 9.92116 | 100.00% | 1654.61 | 13 | 19.866 | 4.46565 | 39.90% | 660.634 |
| 4 | 9.522 | 9.2803 | 15.60% | 258.096 | 14 | 20.641 | 4.29963 | 17.30% | 286.409 |
| 5 | 11.142 | 7.93506 | 7.00% | 116.237 | 15 | 21.57 | 4.11647 | 10.10% | 167.666 |
| 6 | 12.318 | 7.17974 | 23.80% | 394.539 | 16 | 25.229 | 3.52717 | 29.10% | 480.964 |
| 7 | 14.263 | 6.20463 | 58.90% | 973.886 | 17 | 25.531 | 3.48615 | 32.50% | 536.965 |
| 8 | 14.982 | 5.9085 | 23.20% | 383.746 | 18 | 26.574 | 3.35161 | 12.00% | 198.652 |
| 9 | 15.387 | 5.75395 | 33.50% | 554.096 | 19 | 29.057 | 3.0706 | 16.10% | 265.71 |
| 10 | 16.788 | 5.2769 | 31.40% | 519.17 | | | | | |

The DVS test was carried out on the crystalline form B of the hydrochloride salt. The results shows that the DVS curve of the sample is reversible, and the water content changes with the humidity. The moisture absorption of the sample ranged from 2.4% to 5.0% within 10%-90% RH. Moreover, after the DVS test, the XRPD pattern of the sample did not change.

In different solvent systems, attempts were made to prepare the hydrochloride salt by adding different acid equivalents. The details are shown in Table 17. Except that the sample obtained in the acetone/water (4/1) system contained some crystalline form A-I of the free base, all other experiments obtained the same XRPD pattern (as shown in FIG. 5-1). Among them, the patterns of the samples obtained by adding 2.1 eq. and 5 eq. of hydrochloric acid were consistent with that of the sample obtained by adding 1 eq. of the acid. However, the crystallinity of the sample obtained by adding 5 eq. of the acid was slightly poorer, and some impurity peaks were detected in the NMR spectrum.

Therefore, the free base of compound A forms a salt with hydrochloric acid in a 1:1 ratio in the methanol, acetone, and acetone/water systems. According to the results of adding 5 eq. of the acid, increasing the proportion of the acid failed to obtain hydrochloride salts of other ratios, and had a risk of degradation.

**Table 17 Preparation of Hydrochloride Salt Samples under Different Conditions**

<table>
<tr><th>No.</th><th>Solvent</th><th>Hydrochloric Acid</th><th>Crystallization Process</th><th>Results</th></tr>
<tr><td>1</td><td>Methanol (20V)</td><td>1M aqueous solution, 1 eq.</td><td>Methanol was added to the compound A at room temperature to obtain a suspension, thereto was added the acid to obtain a clear solution rapidly, and the solution was stirred at room temperature for 5 minutes to precipitate a solid, stirred for another 3 hours, and then filtered.</td><td>crystalline form B</td></tr>
<tr><td>2</td><td>Acetone (20V)</td><td rowspan="2">3M aqueous solution, 1 eq.</td><td rowspan="2">The solvent was added to the compound A at room temperature to obtain a suspension, thereto was added the acid to obtain still a suspension, the suspension was heated up to 50 °C, stirred for 1 hour without obtaining a clear solution, cooled to room temperature, and stirred overnight.</td><td>crystalline form B</td></tr>
<tr><td>3</td><td>Acetone/Water (4/1, 20V)</td><td>crystalline form B +crystalline form A-I of the free base</td></tr>
<tr><td>4</td><td>Methanol (20V)</td><td>1M aqueous solution, 2.1 eq.</td><td>Methanol was added to the compound A at room temperature to obtain a suspension, thereto was added the acid to obtain a clear solution immediately, the solution was stirred for 2 minutes to precipitate a large amount of solid, thereto was added 20V of methanol, stirred overnight at room temperature, and then filtered.</td><td>crystalline form B</td></tr>
<tr><td>5</td><td>Acetone/Water (4/1, 20V)</td><td>3M aqueous solution, 5 eq.</td><td>The solvent was added to to the compound A at 50 °C to obtain a suspension, thereto was added the acid to obtain still a suspension, the suspension was stirred at 50 °C for 5 hours, and then filtered.</td><td>crystalline form B</td></tr>
<tr><td>6</td><td>Methanol (20V)</td><td>1M aqueous solution, 1 eq.</td><td>Methanol was added to the compound A at room temperature to obtain a suspension, thereto was added the acid to obtain still a suspension, the suspension was stirred overnight, and then filtered.</td><td>crystalline form B</td></tr>
<tr><td>7</td><td>Methanol (20V)</td><td>4M ethyl acetate, 1 eq.</td><td>Methanol was added to the compound A at room temperature to obtain a suspension, thereto was added the acid to obtain a clear solution, after 5 minutes, a solid was precipitated.</td><td>crystalline form B</td></tr>
</table>

**Example 6: Benzenesulfonate of Compound A**

A crystalline form C of the benzenesulfonate was prepared with methanol as the solvent. The specific experimental procedure and characterization results are as follows.

Approximately 300 mg of compound A was weighed and placed in a reaction flask. 6 mL (20V) of methanol was added, and stirred at room temperature to obtain a suspension. 1.05 eq. of benzenesulfonic acid solid was added, and the reaction solution became clear immediately. After stirring for 1 hour, no solid was precipitated. Then 30 mL of ethyl acetate was added, the mixture was stirred overnight at room temperature, and a solid was precipitated out. The resulting solid was filtered and vacuum-dried at 50 °C for 6 hours to obtain the product (yield: approximately 56%, product purity: 98.79%).

PLM and XRPD (FIG. 6-1) show that the sample consists of irregular crystals with a small particle size and agglomeration. The XRPD characterization data are shown in Table 18 below. FIG. 6-2 shows that the TGA test had a weight loss of approximately 0.4% from room temperature to 100 °C, which was caused by the loss of the solvent. DSC has an endothermic peak at 235 °C, which is the melting peak. NMR shows that the molar ratio of the free base to benzenesulfonic acid in the sample is 1:1. Therefore, the crystalline form C of the benzenesulfonate of compound A is an anhydrous crystalline form.

**Table 18 Data of X-ray Powder Diffraction Pattern of Crystalline** Form C

| No. | 2θ angle (°) | D value | Relative peak intensity (%) | Peak intensity | No. | 2θ angle (°) | D value | Relative peak intensity (%) | Peak intensity |
|---|---|---|---|---|---|---|---|---|---|
| 1 | 8.496 | 10.39859 | 47.30% | 766.096 | 12 | 17.817 | 4.97432 | 40.70% | 659.5 |
| 2 | 11.687 | 7.56597 | 14.10% | 228.17 | 13 | 18.111 | 4.89418 | 32.90% | 533.431 |
| 3 | 12.704 | 6.96254 | 20.00% | 323.292 | 14 | 19.167 | 4.62679 | 70.40% | 1140.54 |
| 4 | 12.915 | 6.84921 | 16.40% | 266.03 | 15 | 19.487 | 4.55163 | 27.60% | 446.469 |
| 5 | 13.327 | 6.63812 | 16.60% | 269.363 | 16 | 20.23 | 4.38607 | 22.10% | 358.079 |
| 6 | 13.887 | 6.37183 | 17.70% | 286.439 | 17 | 20.583 | 4.31163 | 10.10% | 162.951 |
| 7 | 14.182 | 6.24002 | 17.70% | 286.961 | 18 | 21.742 | 4.08431 | 54.00% | 874.193 |
| 8 | 15.335 | 5.77348 | 38.60% | 625.522 | 19 | 22.126 | 4.01433 | 100.00% | 1619.16 |
| 9 | 15.784 | 5.60994 | 12.00% | 194.698 | 20 | 22.794 | 3.89812 | 14.10% | 228.903 |
| 10 | 16.358 | 5.41436 | 32.50% | 525.645 | 21 | 26.101 | 3.4113 | 32.10% | 520.008 |
| 11 | 17.116 | 5.17647 | 30.60% | 496.221 | | | | | |

The results of the DVS test show that the moisture absorption of the sample was only 1.2% at 80% RH; however, the moisture absorption increased rapidly from 1.2% to 7% within 80%-90% RH; during the desorption process, the moisture began to be removed again in an environment of 60% RH and was basically removed until the humidity reached 40% RH. Therefore, under high humidity conditions, a hydrate crystalline form of the benzenesulfonate exists, and the crystalline form C may transform into the hydrate; and the hydrate may transform into the anhydrous crystalline form again during the desorption process. In addition, it can be seen from the DVS data that the hydrate formed by the transformation of the crystalline form C of the benzenesulfonate will undergo dehydration when the humidity is 60% RH or below, and can be completely dehydrated to become the anhydrous crystalline form of the benzenesulfonate when the humidity reaches 40% RH. Therefore, this hydrate stabilizes under a humidity range of 60-90% RH, but cannot exist stably under low environmental humidity conditions.

### Example 7: p-Toluenesulfonate of Compound A

Two solvent systems, methanol and acetone/water, were selected, and 1 eq. of p-toluenesulfonic acid was added for the preparation of the p-toluenesulfonate. The specific experimental information and results are shown in Table 19.

According to the characterization results, the ratio of p-toluenesulfonic acid to the free base in the product obtained by adding 1 eq. of the acid was 1:1, and two types of p-toluenesulfonates were obtained: (1) Sample 1 obtained in methanol (crystalline form D-I of the p-toluenesulfonate of compound A) was a monohydrate (the XRPD characterization data are shown in FIG. 7-1, and the characterization data are shown in Table 20 below); (2) Sample 2 obtained in acetone/water (19/1) was a mixed crystal of a new crystalline form (crystalline form D-II) of the p-toluenesulfonate of compound A and the free base (the XRPD characterization data are shown in FIG. 8-1).

**Table 19 Preparation of p-Toluenesulfonate of Compound A**

| No. | Solvent | Crystallization Process | Results |
|---|---|---|---|
| 1 | Methanol (4V) | The acid was added to the compound A, stirred for 2 hours to obtain still a clear solution, thereto was added 32V of ethyl acetate to precipitate a viscous solid, stirrred for another 1 hour, then 16V of ethyl acetate was added, the resulting mixture was stirred at room temperature for two days to precipitate a large amount of solid, and filtered. | crystalline form D-I |
| 2 | Acetone/water (19/1, 20V) | The acid was added to the compound A at 50°C to obtain a suspension with partial solid being dissolved, the suspension was stirred for 1 hour, then cooled to room temperature, 20V of ethyl acetate was added thereto, the resulting mixture was stirred overnight, and then filtered. | crystalline form D-II + a small amount of the free base |

**Table 20 Data of X-ray Powder Diffraction Pattern of Crystalline Form D-I**

| No. | 2θ angle (°) | D value | Relative peak intensity (%) | Peak intensity |
|---|---|---|---|---|
| 1 | 7.866 | 11.23064 | 20.60% | 91.6201 |
| 2 | 9.126 | 9.68308 | 100.00% | 444.817 |
| 3 | 12.982 | 6.81386 | 26.70% | 118.829 |
| 4 | 13.299 | 6.65222 | 33.00% | 146.949 |
| 5 | 15.817 | 5.59837 | 29.10% | 129.254 |
| 6 | 18.538 | 4.78238 | 34.60% | 153.973 |
| 7 | 19.565 | 4.53356 | 35.30% | 156.845 |
| 8 | 20.942 | 4.23847 | 87.80% | 390.735 |

The crystalline form D-I of p-toluenesulfonate of compound A has a low crystallinity, and consists of irregular crystals with a relatively small particle size. The results of thermal analysis are shown in FIG. 7-2. The TGA profile shows a weight loss of approximately 4.2% from room temperature to 150 °C. The DSC profile exhibits two broad endothermic peaks at 49 °C and 167 °C, among which the former is caused by desolvation and the latter is probably the melting peak. NMR shows that no residual organic solvent was detected in the sample, and the molar ratio of the free base to p-toluenesulfonic acid is 1:1. In conclusion, the crystalline form D-I of the p-toluenesulfonate of compound A is a monohydrate (the theoretical water content of the monohydrate is 2.3%).

The characterization results of Sample 2 of the p-toluenesulfonate of compound A indicate that this sample has a high crystallinity and consists of irregular crystals. However, the XRPD pattern (FIG. 8-1) shows that the sample contains a small amount of the free base, making it a mixed crystal of the crystalline form D-II and the free base. The characterization data are shown in Table 21 below. The TGA profile shows a weight loss of approximately 3.8% from room temperature to 130 °C, which should be caused by the loss of the solvent. The DSC profile exhibits three endothermic peaks at 70 °C, 145 °C, and 160 °C, which are respectively caused by desolvation and melting (FIG. 8-2). The NMR results show that the sample contains approximately 0.1% residual acetone solvent, and the ratio of the free base to the acid is 1:0.9. In conclusion, the crystalline form D-II of the p-toluenesulfonate of compound A should be a monohydrate. Since this sample contains a small amount of the free base, the characterization data for a pure crystalline form D-II could not be obtained.

**Table 21 Data of X-ray Powder Diffraction Pattern of crystalline form D-II**

| No. | 2θ angle (°) | D value | Relative peak intensity (%) | Peak intensity | No. | 2θ angle (°) | D value | Relative peak intensity (%) | Peak intensity |
|---|---|---|---|---|---|---|---|---|---|
| 1 | 7.443 | 11.86842 | 100.00% | 1126.32 | 14 | 19.886 | 4.46111 | 31.80% | 358.361 |
| 2 | 8.49 | 10.40613 | 24.60% | 277.494 | 15 | 20.163 | 4.40044 | 20.10% | 226.279 |
| 3 | 9.268 | 9.53413 | 32.20% | 362.394 | 16 | 20.543 | 4.32002 | 33.80% | 380.402 |

(continued)

| No. | 2θ angle (°) | D value | Relative peak intensity (%) | Peak intensity | No. | 2θ angle (°) | D value | Relative peak intensity (%) | Peak intensity |
|---|---|---|---|---|---|---|---|---|---|
| 4 | 10.61 | 8.33132 | 88.60% | 997.43 | 17 | 21.19 | 4.18955 | 16.70% | 188.208 |
| 5 | 12.135 | 7.28742 | 17.30% | 195.364 | 18 | 21.401 | 4.14864 | 13.20% | 148.868 |
| 6 | 12.752 | 6.93659 | 13.70% | 154.82 | 19 | 21.931 | 4.04955 | 17.10% | 192.488 |
| 7 | 14.297 | 6.19002 | 35.70% | 401.631 | 20 | 22.36 | 3.97275 | 11.50% | 129.329 |
| 8 | 14.532 | 6.09068 | 62.70% | 705.677 | 21 | 24.588 | 3.61771 | 28.50% | 321.373 |
| 9 | 15.002 | 5.90083 | 33.80% | 380.981 | 22 | 24.775 | 3.59074 | 29.20% | 329.261 |
| 10 | 15.627 | 5.66595 | 34.20% | 384.739 | 23 | 25.741 | 3.45815 | 24.60% | 276.908 |
| 11 | 18.42 | 4.81275 | 34.50% | 389.029 | 24 | 26.275 | 3.38905 | 19.50% | 219.595 |
| 12 | 19.056 | 4.65363 | 51.20% | 577.096 | 25 | 29.294 | 3.04629 | 14.60% | 164.389 |
| 13 | 19.58 | 4.53023 | 11.70% | 132.171 | | | | | |

**Example 8: Fumarate of Compound A**

The compound A obtained from Preparation Example 1 was taken, and 20V of methanol was added for dissolving the compound A. 1 eq. of fumaric acid was added to obtain a suspension. Then 12V of methanol solution was added, heated to 50°C and stirred for 3 hours, the resulting mixture still remained a suspension. The suspension was cooled to room temperature, stirred overnight, filtered, and dried at 50°C for 20 hours to obtain the product. This crystalline solid is defined as crystalline form E.

The crystalline form E of the fumarate of compound A consists of irregular crystals with low crystallinity. The XRPD characterization is shown in FIG. 9-1, and the characterization data are shown in Table 22 below. The results of thermal analysis are shown in FIG. 9-2. The TGA profile shows a weight loss of approximately 3.8% from room temperature to 150 °C. The DSC profile indicates the presence of multiple thermal events, including a desolvation peak at 32 °C, an exothermic crystal-transformation peak at 159 °C, and endothermic peaks at 222 °C, 230 °C, and 240 °C that should be caused by melting or decomposition. NMR detected that there was no residual organic solvent in the sample, and the ratio of fumaric acid to the free base was 1:1. Therefore, the crystalline form E of the fumarate is a monohydrate (the theoretical water content of the monohydrate is 2.5%), but it has low crystallinity and may lose water at a relatively low temperature.

**Table 22 Data of X-ray Powder Diffraction Pattern of Crystalline Form E**

| No. | 2θ angle (°) | D value | Relative peak intensity (%) | Peak intensity |
|---|---|---|---|---|
| 1 | 4.148 | 21.28427 | 100.00% | 1093.55 |
| 2 | 8.35 | 10.58036 | 9.20% | 100.783 |
| 3 | 12.474 | 7.09007 | 14.70% | 161.147 |
| 4 | 13.196 | 6.7039 | 20.30% | 221.679 |
| 5 | 13.917 | 6.35829 | 7.20% | 78.8464 |
| 6 | 16.608 | 5.33359 | 26.30% | 287.579 |
| 7 | 19.92 | 4.45356 | 34.30% | 374.764 |
| 8 | 21.488 | 4.13208 | 20.10% | 219.281 |
| 9 | 22.113 | 4.0167 | 19.30% | 211.278 |

**Example 9: Citrate of Compound A**

The compound A obtained from Preparation Example 1 was taken, and 20V of methanol was added for dissolving the compound A. After 1 eq. of citric acid was added, a suspension was obtained. Then 12V of methanol solution was

added, stirred at 50°C for 3 hours, the resulting mixture still remained a suspension. The suspension was cooled to room temperature, stirred overnight, filtered, and dried at 50°C for 20 hours to obtain Sample 1.

The compound A obtained from Preparation Example 1 was taken, 20V of acetone/water (4/1) was added, and the mixture was heated to 50°C for dissolution. After 2 eq. of citric acid was added, a clear solution was obtained. After the solution was stirred for 1.5 hours, no solid was precipitated. After the solution was cooled to room temperature and stirred overnight at room temperature, a solid was precipitated, followed by filtering and drying at 50°C for 6 hours to obtain Sample 2.

According to the XRPD characterization (see FIG. 10-1), both Sample 1 and Sample 2 above are of the same crystalline form, i.e., the crystalline form F, and the characterization data are shown in Table 23 below. This crystalline form consists of irregular crystals with high crystallinity. The results of thermal analysis are shown in FIG. 10-2. The TGA profile shows a weight loss of approximately 1.6% from room temperature to 150 °C. The DSC profile exhibits three endothermic peaks at 30 °C, 198 °C, and 248 °C, which should be caused by desolvation, melting, and sample decomposition respectively. The 1H-NMR results show that the ratio of the free base to citric acid in the samples is 1:1. The results of the DVS test show that the crystalline form F had a moisture absorption of approximately 3.8% under 80% RH, and the crystalline form of the samples did not change before and after the DVS test. The citrate may be a channel hydrate, with the water content changing with the humidity.

**Table 23 Data of X-ray Powder Diffraction Pattern of Crystalline Form F**

| No. | 2θ angle (°) | D value | Relative peak intensity (%) | Peak intensity | No. | 2θ angle (°) | D value | Relative peak intensity (%) | Peak intensity |
|---|---|---|---|---|---|---|---|---|---|
| 1 | 10.072 | 8.77501 | 58.50% | 808.381 | 10 | 18.429 | 4.81039 | 41.70% | 576.772 |
| 2 | 11.124 | 7.94772 | 44.60% | 617.218 | 11 | 19.391 | 4.57382 | 87.00% | 1202.93 |
| 3 | 12.471 | 7.09223 | 31.90% | 441.258 | 12 | 19.982 | 4.44002 | 100.00% | 1382.99 |
| 4 | 12.973 | 6.81879 | 20.20% | 278.697 | 13 | 20.49 | 4.33109 | 31.20% | 430.964 |
| 5 | 14.126 | 6.26454 | 14.40% | 199.289 | 14 | 21.462 | 4.137 | 51.10% | 706.619 |
| 6 | 15.51 | 5.70869 | 89.80% | 1241.67 | 15 | 23.024 | 3.85979 | 60.40% | 835.007 |
| 7 | 16.617 | 5.33077 | 28.20% | 389.945 | 16 | 23.326 | 3.81051 | 37.60% | 520.33 |
| 8 | 17.052 | 5.1957 | 97.00% | 1342.09 | 17 | 24.198 | 3.67511 | 51.40% | 711.305 |
| 9 | 17.536 | 5.05341 | 19.50% | 269.225 | 18 | 24.681 | 3.60424 | 35.10% | 485.508 |

**Example 10: Malate of Compound A**

The compound A obtained from Preparation Example 1 was taken, 20V of methanol was added, and stirring was conducted for dissolving the compound A. After 1 eq. of malic acid was added, a suspension was obtained. Then 12V of methanol was added, and the mixture was heated to 50°C and stirred to obtain a clear solution. 3 hours later, the solution was cooled to room temperature and stirred overnight to precipitate a solid, followed by filtering and drying at 50°C for 20 hours to obtain the product.

XRPD characterization was carried out on the obtained product (see FIG. 11-1), which is designated as a crystalline form G. The characterization data are shown in Table 24 below. The crystalline form G of the malate has good crystallinity, and consists of irregular crystals with agglomeration. According to the results of thermal analysis (see FIG. 11-2), the TGA profile shows a weight loss of approximately 4.2% from room temperature to 120 °C; and the DSC profile exhibits four endothermic peaks at 57 °C, 175 °C, 191 °C, and 238 °C, among which the former one endothermic peak is caused by desolvation, and the latter three endothermic peaks are melting and decomposition peaks. NMR shows that the ratio of malic acid to the free base in the sample is 1:1, and the sample contains no residual organic solvent. Therefore, this sample is a monohydrate (the theoretical water content of the monohydrate is 2.3%).

**Table 24: Data of X-ray Powder Diffraction Pattern of Crystalline Form G**

| No. | 2θ angle (°) | D value | Relative peak intensity (%) | Peak intensity | No. | 2θ angle (°) | D value | Relative peak intensity (%) | Peak intensity |
|---|---|---|---|---|---|---|---|---|---|
| 1 | 4.045 | 21.82651 | 55.60% | 733.745 | 11 | 19.028 | 4.66028 | 17.30% | 227.695 |
| 2 | 9.802 | 9.01655 | 15.50% | 203.877 | 12 | 19.636 | 4.51728 | 50.40% | 665.201 |
| 3 | 10.498 | 8.42025 | 16.90% | 222.736 | 13 | 20.411 | 4.34768 | 43.70% | 576.113 |
| 4 | 11.449 | 7.72285 | 35.40% | 466.317 | 14 | 21.052 | 4.21653 | 100.00% | 1318.66 |
| 5 | 12.395 | 7.13519 | 34.60% | 455.647 | 15 | 22.536 | 3.94228 | 74.30% | 980.256 |
| 6 | 13.225 | 6.6895 | 9.40% | 123.356 | 16 | 22.664 | 3.92026 | 45.60% | 601.302 |
| 7 | 14.271 | 6.20121 | 12.20% | 161.044 | 17 | 24.081 | 3.6927 | 20.50% | 270.001 |
| 8 | 15.889 | 5.57333 | 20.20% | 266.109 | 18 | 25.386 | 3.50573 | 22.80% | 300.473 |
| 9 | 16.96 | 5.22355 | 27.40% | 361.695 | 19 | 25.704 | 3.46302 | 20.40% | 269.627 |
| 10 | 17.278 | 5.12827 | 27.90% | 368.362 | 20 | 26.08 | 3.41394 | 18.60% | 245.34 |

**Example 11: Succinate of Compound A**

The compound A obtained from Preparation Example 1 was taken, 20V of acetone/water (9/1) was added, and the mixture was heated to 50 °C. After 1 eq. of succinic acid was added, the resulting mixture still remained a suspension, but part of the solid was dissolved. The suspension was stirred for 1 hour and then cooled to room temperature, 20V of ethyl acetate was then added, and stirred overnight, the resulting mixture was filtered, and dried at 50°C for 20 hours to obtain the product.

The XRPD pattern exhibits diffraction peaks of the free base. NMR shows that the molar ratio of the free base to succinic acid in the sample is 3:2, and the melting peak of the free base can be observed in the DSC profile. This indicates that the free base and succinic acid did not react completely, or the succinate tended to dissociate readily.

**Example 12: Other Salts of Compound A**

The present application further attempted the reactions between the free base and other acids (sulfuric acid, methanesulfonic acid, tartaric acid, maleic acid, and phosphoric acid). The specific experimental procedures and results are shown in Table 25 below. The results show that no crystalline samples were obtained from the reactions between the free base of compound A and maleic acid or phosphoric acid; and a total of 4 crystal samples were obtained from the reactions with sulfuric acid, methanesulfonic acid, and tartaric acid, while the obtained samples all had low crystallinity and no detailed characterization was carried out on them. Therefore, when the free base reacted with sulfuric acid, methanesulfonic acid, tartaric acid, maleic acid, and phosphoric acid, no crystal salts with acceptable crystallinity could be obtained.

**Table 25 Preparation Methods of Other Salts of Compound A**

| No. | Acid | Solvent | Crystallization Process | Result |
|---|---|---|---|---|
| 1 | A solution of 3M Sulfuric acid in methanol | Methanol (20V) | The acid was added to the compound A, stirred for 3 hours to still obtain a clear solution, 100V of ethyl acetate was then added, the resulting mixture was a clear solution, and then stirred at room temperature overnight to precipitate a solid, and filtered. | Low crystallinity, and low yield |
| 2 | | Methanol (8V) | The acid was added to the compound A, stirred for 2 hours to still obtain a clear solution, 72V of ethyl acetate was added to precipitate a solid, the resulting mixture was stirred for two days, and then filtered. | Low crystallinity |
| 3 | | Acetone (20V) | The acid was added to the compound A at room temperature to still obtain a suspension, the suspension was heated to 50°C, stirred for 1 hour to get viscous, cooled to room temperature, and then stirred overnight to precipitate a solid. | Amorphous |
| 4 | Methanesulfonic acid | Methanol (4V) | The acid was added to the compound A, stirred for 2 hours to still obtain a clear solution, 20V of ethyl acetate was added to precipitate viscous substance, the resulting mixture was stirred for 1 hour, then 20V of ethyl acetate was added, stirred at room temperature for two days to precipitate a large amount of solid, and filtered. | Low crystallinity |
| 5 | Maleic acid | Methanol (20V) | The acid was added to the compound A to get clear rapidly, after stirring at room temperature for 3 hours, a solid began to precipitate, the resulting mixture was stirred overnight, and then filtered. | Amorphous |
| 6 | | Acetone/water (19/1, 20V) | The acid was added to the compound A at 50°C to obtain a clear solution, the solution was stirred for 1 hour, then cooled to room temperature to precipitate a small amount of solid, 20V of ethyl acetate was added, the resulting mixture was stirred overnight, and filtered. | Amorphous |
| 7 | Phosphoric acid | Methanol (20V) | The acid was added to the compound A to obtain a suspension, 12V of methanol was added, and stirred at 50°C to obtain a clear solution. The solution was stirred for 3 hours, cooled to room temperature and then stirred overnight, but no solid was precipitated. After adding 40V of ethyl acetate, a solid was precipitated, stirred for 6 hours, and filtered. | Amorphous |
| 8 | | Acetone/water (4/1, 20V) | The acid was added to the compound A at 50°C to get a viscous substance. 1 hour later, the resulting substance was cooled to room temperature, and stirred overnight to still obtain a viscous substance. | Viscous substance |

(continued)

| No. | Acid | Solvent | Crystallization Process | Result |
|---|---|---|---|---|
| 9 | Tartaric acid | Methanol (20V) | The acid was added to the compound A to obtain a suspension, 12V of methanol was added, heated to 50°C and stirred for 3 hours to still obtain a suspension, then the suspension was cooled to room temperature, stirred overnight, and filtered. | Low crystallinity |
| 10 | | Acetone/water (4/1, 20V) | The acid was added to the compound A at 50°C to obtain a clear solution, the solution was stirred for 1 hour, then cooled to room temperature, it was stirred overnight at room temperature to precipitate a solid, which was then filtered. | Amorphous |

**Test Example 1: crystalline form Transformation Test** of Free **Base** of Compound **A**

1. Anhydrous crystalline form transformation test

1) Preparation of initial mixed sample: equal amounts of the crystalline form A-I and the crystalline form A-IV of the free base of compound A were mixed evenly, and sampled for XRPD characterization.

2) 1 mL of the corresponding solvent was added to the sample to form a suspension, the suspension was stirred at the corresponding temperature, and sampled for XRPD characterization.

**Table 26 Anhydrous crystalline form Transformation Test of Free Base of Compound A**

| Experimental conditions | Solvent | n-Butanol | | n-Butanol/Anisole (v:v=1:1) | |
|---|---|---|---|---|---|
| | Temperature | 25°C±2°C | 37°C±2°C | 25°C±2°C | 37°C±2°C |
| Experimental results | | crystalline form A-I | crystalline form A-I | crystalline form A-I | crystalline form A-I |

2. Competition experiment between anhydrates and solvates and solvent activity experiment

1) A mixed sample formed by mixing evenly equal amounts of different crystalline forms of the free base of compound A was taken, and sampled for XRPD characterization.

2) 1 mL of the corresponding solvent was added to the mixed sample to form a suspension, the suspension was stirred at room temperature, and sampled for XRPD characterization.

**Table 27 Competition Experiment between Anhydrates and Solvates and Solvent Activity Experiment**

| Tests | Mixed Samples | Solvent | Test Results |
|---|---|---|---|
| Competition experiment between anhydrates and solvates | crystalline form A-I<br>crystalline form A-II<br>crystalline form A-III | n-Butanol | crystalline form A-I |
| | | n-Butanol/Anisole (v:v = 1:1) | crystalline form A-I |

(continued)

| Tests | Mixed Samples | Solvent | Test Results |
|---|---|---|---|
| Solvent activity experiment with trifluoroethanol | | 20% (v/v) Trifluoroethanol | Transformation to crystalline form A-I |
| | crystalline form A-I crystalline form A-II | 50% (v/v) Trifluoroethanol | Transformation to crystalline form A-I |
| | | 80% (v/v) Trifluoroethanol | Transformation to crystalline form A-II |
| Solvent activity experiment with dimethyl sulfoxide | crystalline form A-I crystalline form A-III | 20% Dimethyl sulfoxide (v/v) | crystalline form A-I |
| | | 50% Dimethyl sulfoxide (v/v) | crystalline form A-I |
| | | 80% Dimethyl sulfoxide (v/v) | crystalline form A-I |

**Test Example 2: Stability Test**

Appropriate amounts of the crystalline form A-I of the free base of compound A, the crystalline form B of the hydrochloride monohydrate of compound A, and the crystalline form C of the benzenesulfonate of compound A were respectively placed open under three conditions: 60 °C, 92.5% RH and 40 °C/75% RH for 7 days. Subsequently, the samples after 7 days were respectively detected by HPLC and XRPD to determine their chemical and physical stabilities.

The test results show that after being placed under the conditions of 60 °C, 92.5% RH and 40 °C/75% RH for 7 days, the crystalline form A-I of the free base of compound A, the crystalline form B of the hydrochloride monohydrate of compound A, and the crystalline form C of the benzenesulfonate of compound A were chemically stable, and no significant degradation was found. The XRPD results show that after 7 days under the three conditions, the crystalline form A-I of the free base of compound A and the crystalline form B of the hydrochloride monohydrate of compound A remained unchanged, while after 7 days under the high-humidity condition of 92.5% RH, the crystalline form C of the benzenesulfonate of compound A changed into a new crystalline form, which might be a hydrate, and the crystalline form C remained unchanged under the other two conditions.

In addition, the present application also investigated the crystalline form obtained after grinding or crushing the crystalline form A-I of the free base of compound A, and found that the crystalline form of the sample remained unchanged.

**Table 28 Results of Stability Test**

| Free base/Salt | Experimental Conditions | Purity (Peak area%) | | XRPD-7days |
|---|---|---|---|---|
| | | 0 day | 7 days | |
| crystalline form A-I of the free base of | 60°C | 99.16 | 99.24 | Unchanged |
| | 40°C /75%RH | | 99.20 | Unchanged |
| compound A | 92.5%RH | | 99.24 | Unchanged |
| crystalline form B of the hydrochloride salt of compound A | 60°C | 99.31 | 99.38 | Unchanged |
| | 40°C /75%RH | | 99.33 | Unchanged |
| | 92.5%RH | | 99.36 | Unchanged |
| crystalline form C of the benzenesulfonate of compound A | 60°C | 98.79 | 98.89 | Unchanged |
| | 40°C /75%RH | | 98.79 | Unchanged |
| | 92.5%RH | | 98.84 | Changed |

**Test Example 3: Solubility comparison among crystalline form A-I of the free base of compound A, crystalline form B of hydrochloride monohydrate of compound A, and crystalline form C of benzenesulfonate of compound A**

The crystalline form B of the hydrochloride monohydrate of compound A, the crystalline form C of the benzenesulfonate of compound A, and the crystalline form A-I of the free base of compound A were respectively added to water, SGF, FaSSIF, and FeSSIF to prepare 5 mg/mL sample solutions/suspensions. Two samples were prepared in parallel for each medium. All samples were shaken at 200 rpm at 37°C for 24 h. At 0.5 h, 2 h, and 24 h, appropriate amounts of the suspensions were taken and filtered. The obtained filtrates were analyzed by HPLC and their pH values were measured, and the filter cakes were analyzed by XRPD.

The results showed that the solubilities of the free base and the two salts in SGF were all greater than 5 mg/mL; the free base had a relatively low solubility (approximately 0.003 mg/mL) in water, while the two salts had relatively high solubilities in water, and the benzenesulfonate had the highest solubility, which was greater than 5 mg/mL at 24 h. In FaSSIF and FeSSIF, the solubility of the hydrochloride salt was slightly higher than that of the free base. But in FaSSIF, due to the crystalline form transformation of the benzenesulfonate, the solubility of the benzenesulfonate within two hours was lower than those of the free base and the hydrochloride salt.

The XRPD test on the filter cakes show that during the solubility determination process, the free base and the benzenesulfonate became viscous in FeSSIF. While the benzenesulfonate transformed to the crystalline form I of the hydrochloride salt in the FaSSIF medium after 0.5 h, the XRPD patterns of the remaining solids remained unchanged.

In summary, while the crystalline form A-I of the free base of compound A exhibited significantly lower water solubility than the two salts, it had little difference in solubility from the two salts in the other three bio-relevant media.

**Table 29 Solubility Comparison among crystalline form A-I of the free base of compound A, crystalline form B of hydrochloride monohydrate of compound A, and crystalline form C of benzenesulfonate of compound A**

| Sample | Medium | Solubility - 0.5 h (mg/mL) | Solubility -2 h (mg/mL) | Solubility -24 h (mg/mL) | XRPD-24h |
|---|---|---|---|---|---|
| crystalline form A-I of the free base of compound A | Water | 0.002 | 0.004 | 0.003 | Unchanged |
| | SGF | >5 | >5 | >5 | Clear |
| | FeSSIF | 1.06 | 1.00 | 1.10 | Viscous solid |
| | FaSSIF | 0.17 | 0.17 | 0.20 | Unchanged |
| crystalline form B of hydrochloride monohydrate of compound A | Water | 3.32 | 3.39 | 3.45 | Unchanged |
| | SGF | >5 | >5 | >5 | Clear |
| | FeSSIF | 1.63 | 1.68 | 1.37 | Unchanged |
| | FaSSIF | 0.24 | 0.21 | 0.21 | Unchanged |
| crystalline form C of benzenesulfonate of compound A | Water | 3.77 | 3.73 | >5 | Clear |
| | SGF | >5 | >5 | >5 | Clear |
| | FeSSIF | 1.43 | 1.48 | 1.37 | Viscous solid |
| | FaSSIF* | 0.04 | 0.06 | 0.18 | transformation to crystalline form B of the hydrochloride salt after 0.5 h |
| Note: * Particle breakthrough occurred during the filtration of the crystalline form C of the benzenesulfonate of compound A in the FaSSIF solution. The measured data are the HPLC results of the supernatant obtained after centrifugation. | | | | | |

**Test Example 4: In vitro Tumor Cell Proliferation Inhibition Test of crystalline form A-I of Free Base of Compound A**

**1. Test Method**

The tumor cell strains were cultured in an incubator at 37 °C with 5% $CO_2$. The cells were subcultured regularly,

and those in the logarithmic growth phase were taken for plating. The cells were stained with trypan blue and the viable cells were counted. The cell concentration was adjusted to an appropriate level. The cell suspension was added to each well of the culture plate, and the culture medium without cells was added to the blank control wells. The culture plate was incubated overnight in an incubator at 37 °C, 5% $CO_2$, and 100% RH. The compound A (the crystalline form A-I of the free base) was diluted in a gradient from the highest concentration to the lowest concentration with DMSO (starting from 20 $\mu$M, diluted at a ratio of 1:3, with 8 concentration gradients, and 3 replicate wells for each concentration gradient). The compound working solution was added to the cell culture plate, and DMSO at the same final concentration was added to the solvent control wells. Then the plate was returned to the incubator for 4-7 days of culture. The cell viability was detected according to the instructions of the Promega CellTiter-Glo (CTG) Luminescent Cell Viability Assay Kit (Promega-G7573).

The inhibition rate (IR) of the test compound was calculated using the following equation:

$$IR\ (\%) = [1-(RLU_{compound} - RLU_{blank\ control}) / (RLU_{vehicle\ control} - RLU_{blank\ control})] \times 100\%$$

The inhibition rates of the compound at different concentrations were calculated in Excel, then the inhibition curve was plotted using GraphPad Prism software and relevant parameters, including the minimum inhibition rate, maximum inhibition rate, and $IC_{50}$ were calculated. The $IC_{50}$ was calculated using the following equation:

Y = minimum inhibition rate + (maximum inhibition rate - minimum inhibition rate) / (1 + 10^((Log$IC_{50}$-X)*HillSlope))

X: log(concentration)
Y: response value, negatively correlated with X
HillSlope: slope factor
Note: When the maximum inhibition rate is < 50%, the result is expressed as $IC_{50}$ > the maximum starting concentration.

### 2. Test Results

The results show that compound A had good inhibitory activity against the 7 tested breast cancer cell strains and had marked superiority over the CDK4/6 selective inhibitor Palbociclib. Wherein, compound A had better inhibitory activity than Palbociclib against the strain MDA-MB-468 (Rb-) with primary resistance to Palbociclib and the strain xMCF-7/Palbo-R with acquired resistance to Palbociclib. Especially for the strain MDA-MB-468 (Rb-) with primary resistance to Palbociclib, the therapeutic advantage of compound A was more evident, with a maximum inhibition rate exceeding 99%, while the maximum inhibition rate of Palbociclib was only 34%. Compound A had better inhibitory activity than Palbociclib against the HR+/HER2- breast cancer cells T47D, the triple-negative breast cancer cells HCC1187, and MDA-MB-231, and comparable inhibitory activity to Palbociclib against the HR+/HER2- breast cancer cells MCF-7. For the HR-/HER2+ breast cancer cells MDA-MB-453, although compound A had an $IC_{50}$ value that was about twice that of Palbociclib, it had a maximum inhibition rate of nearly 100% against this cell strain, while the maximum inhibition rate of Palbociclib could only reach about 74% even with an increased dose. In other breast cancer cell strains, compound A also showed a higher maximum inhibition rate. Thus, compound A is superior to Palbociclib in terms of the maximum effect of inhibiting tumor cell proliferation.

As the direct downstream target of CDK4/6, Rb protein is considered to be the main biomarker for evaluating the sensitivity of CDK4/6 inhibitors. The absence of the Rb protein (Rb-) indicates that the tumor is insensitive to the CDK4/6 inhibitor (primary resistance). The results in Table 31 show that compound A had a significant inhibitory effect on the Hep3B(Rb-) liver cancer cells, with a maximum inhibition rate exceeding 99%, suggesting that compound A also has a good therapeutic effect on CDK4/6 inhibitor-resistant liver cancer.

Compound A has a good inhibitory activity against the Rb+ colorectal cancer cells COLO205, HT-29, and HCT116, the Rb+ liver cancer cells Huh-7 and JHH-7, the hematological tumor cells MV-4-11 and MOLM-13, the ovarian cancer cells A2780, the small cell lung cancer cells NCI-H211, and the melanoma SK-MEL-5.

**Table 30 In vitro Proliferation Inhibition $IC_{50}$ Values of Compound A against Multiple Breast Cancer Cell Strains (CTG Test)**

| Indications | Cell Strains | $IC_{50}$ (μM) | | Maximum Inhibition Rate (%) | |
|---|---|---|---|---|---|
| | | Compound A | Palbociclib | Compound A | Palbociclib |
| Breast cancer (acquired resistance to Palbociclib) | xMCF-7/ Palbo-R | 3.093 | 7.699 | 99.677 | 98.367 |
| Breast cancer (primary resistance to Palbociclib) | MDA-MB-468 (Rb-) | 0.440 | >5 | 99.693 | 34.044 |
| HR+/HER2- Breast cancer | MCF-7 | 0.076 | 0.052 | 99.937 | 96.983 |
| | T47D | 1.499 | >5 | 99.910 | 48.850 |
| Triple-negative breast cancer | HCC1187 | 1.112 | >5 | 99.777 | 11.183 |
| | MDA-MB-231 | 3.451 | >5 | 99.567 | 41.943 |
| HR-/HER2+ Breast cancer | MDA-MB-453 | 0.384 | 0.188 | 102.600 | 73.980 |

**Table 31 In vitro Proliferation Inhibition $IC_{50}$ Values of Compound A against Human Tumor Cells (CTG Test)**

| Indications | Cell Strains | IC50 (μM) | Maximum Inhibition Rate (%) |
|---|---|---|---|
| Colorectal cancer | COLO 205 | 0.734 | 99.093 |
| | HT-29 | 0.113 | 95.622 |
| | HCT 116 | 0.134 | 99.244 |
| Liver cancer | Huh-7 | 0.107 | 99.447 |
| | Hep3B (Rb-) | 3.326 | 99.055 |
| | JHH-7 | 0.107 | 99.394 |
| Hematological tumor | MV-4-11 | 0.102 | 99.897 |
| | MOLM-13 | 0.370 | 99.993 |
| Melanoma | SK-MEL-5 | 9.499 | 99.213 |
| Ovarian cancer | A2780 | 0.438 | 98.887 |
| Small cell lung cancer | NCI-H211 | 0.201 | 99.883 |

**Test Example 5: Effect of particle size of crystalline form A-I of the free base of compound A on dissolution of formulations**

The crystalline form A-I of the free base of compound A is a drug with a low solubility. The particle size of the active ingredient may affect the dissolution and content uniformity of the formulation. Therefore, it is necessary to investigate the particle size of the active ingredient.

Using the crystalline form A-I of the free base of compound A as the active ingredient, different particle sizes of the active ingredient were selected, and conventional excipients such as fillers, disintegrants, etc., were added thereto, the resulting mixture was compressed into tablets. The effect of the particle size of the active ingredient on the dissolution of the formulation was investigated, and the results are shown in the table below.

**Table 32 Effect of Particle Size of Active Ingredient on Dissolution of Formulation**

| Active ingredient No. | | Active ingredient-1 | Active ingredient-2 |
|---|---|---|---|
| Particle size of active ingredient | *d* (0.9) | 28.0μm | 94.9μm |
| | *d (0.5)* | 11.6μm | 30.8μm |
| | *d (0.1)* | 4.1μm | 8.2μm |

(continued)

| Formulation No. | | Formulation-1 | | Formulation-2 | |
|---|---|---|---|---|---|
| Cumulative dissolution in phosphate medium with pH of 4.5 | Time | AVE (%) | RSD (%) | AVE (%) | RSD (%) |
| | 5min | 36 | 8.6 | 47 | 1.7 |
| | 10min | 54 | 8.2 | 66 | 12 |
| | 15min | 71 | 6.4 | 91 | 5.3 |
| | 30min | 94 | 2.0 | 98 | 0.6 |
| | 45min | 96 | 0.8 | 97 | 2.5 |
| Note: Formulation-1 and Formulation-2 are compositionally identical except for the particle size of the active ingredient. | | | | | |

**Test Example 6: Investigation on the stability of crystalline form A-I of the free base of compound A**

The factors influencing the stability of the crystalline form A-I of the free base of compound A were investigated, and the accelerated stability testing and long-term stability testing were completed. The test results are shown in the following table.

(1) Stress Testing

**Table 33 Results of stress testing of crystalline form A-I of the free base of compound A**

| Investigation Items | | 0 Day (%) | Illumination Testing 4500lx±500lx | | | High Temperature (60°C) | | | High Humidity (92.5% RH) | | |
|---|---|---|---|---|---|---|---|---|---|---|---|
| | | | 7 (day) | 14 (days) | 30 (days) | 7 (days) | 14 (days) | 30 (days) | 7 (days) | 14 (days) | 30 (days) |
| Description | Appearance | Yellow solid | Yellow solid | Yellow solid | Yellow solid | Yellow solid | Yellow solid | Yellow solid | Yellow solid | Yellow solid | Yellow solid |
| Hygroscopic weight gain (%) | | N/A | N/A | N/A | N/A | N/A | N/A | N/A | 0.03 | 0.05 | 0.09 |
| loss on drying (%) | | 0.03 | 0.07 | 0.06 | 0.07 | 0.05 | 0.05 | 0.03 | 0.06 | 0.07 | 0.05 |
| crystalline form | | crystalline form A-I of free base | N/A | N/A | Unchanged | N/A | N/A | Unchanged | N/A | N/A | Unchanged |
| Content (%) | | 99.5 | 101.0 | 100.2 | 100.2 | 100.4 | 100.6 | 100.1 | 100.2 | 99.6 | 101.0 |
| Total impurities (%) | | 0.84 | 0.76 | 0.79 | 0.84 | 0.76 | 0.83 | 0.81 | 0.76 | 0.78 | 0.87 |
| Notes: "N/A" indicates not tested. | | | | | | | | | | | |

Conclusion: The results of the investigation of the influencing factors show that there was no significant change in the description of the crystalline form A-I of compound A under the above test conditions. Under the high-temperature conditions, there was no significant change in relevant substances. Under the high-humidity conditions, there was no significant change in the hygroscopic weight gain, and the relevant substances remained unchanged compared with 0 month. Under the illumination conditions (illuminance: 4500 lx±500 lx, the total illuminance of the light source was not less than $1.2\times10^6$ lux·hr, and the energy of the near-ultraviolet lamp was not less than 200 W·hr/$m^2$), the crystalline form A-I of compound A was relatively stable, and no significant degradation impurities were found. It demonstrates that the crystalline form A-I of compound A has good stability.

(2) Accelerated Stability Testing

Packaging: Simulated the commercial packaging (using a pharmaceutical-grade low-density polyethylene bag as the inner packaging and a polyester/aluminum/polyethylene composite bag for pharmaceutical packaging as the outer packaging)
Investigation conditions: 40°C±2°C/75%±5%RH

**Table 34 Results of Accelerated Stability Testing of Crystalline Form A-I of Free Base of Compound A**

| Investigation Items | | Time (Months) | | | | |
|---|---|---|---|---|---|---|
| | | 0 | 1 | 2 | 3 | 6 |
| Description | Appearance | Yellow solid | Yellow solid | Yellow solid | Yellow solid | Yellow solid |
| loss on drying (%) | | 0.03 | 0.04 | 0.02 | 0.02 | 0.08 |
| crystalline form | | crystalline form A-I of free base | N/A | N/A | Unchanged | Unchanged |
| Measured content (%) | | 99.5 | 100.4 | 100.8 | 100.6 | 100.9 |
| Total impurities (%) | | 0.84 | 0.80 | 0.82 | 0.85 | 0.77 |
| Notes: "N/A" indicates not tested. | | | | | | |

(3) Long-term Stability Testing

Packaging: Simulated the commercial packaging (using a pharmaceutical-grade low-density polyethylene bag as the inner packaging and a polyester/aluminum/polyethylene composite bag for pharmaceutical packaging as the outer packaging)
Investigation conditions: 25°C±2°C/60%±5%RH

**Table 35 Results of Long-term Stability Testing of Crystalline Form A-I of Free Base of Compound**

| Investigation Items | | Time (Months) | | | | | |
|---|---|---|---|---|---|---|---|
| | | 0 | 3 | 6 | 9 | 12 | 18 |
| Description | Appearance | Yellow solid | Yellow solid | Yellow solid | Yellow solid | Yellow solid | Yellow solid |
| loss on drying (%) | | 0.03 | 0.02 | 0.05 | 0.05 | 0.05 | 0.03 |
| crystalline form | | crystalline form A-I of free base | Unchanged | Unchanged | Unchanged | Unchanged | Unchanged |

(continued)

| Investigation Items | Time (Months) | | | | | |
|---|---|---|---|---|---|---|
| | 0 | 3 | 6 | 9 | 12 | 18 |
| Measured content (%) | 99.5 | 100.8 | 100.6 | 99.6 | 100.8 | 98.7 |
| Total impurities (%) | 0.84 | 0.89 | 0.75 | 0.73 | 0.78 | 0.71 |
| Note: "N/A" indicates not tested. | | | | | | |

Conclusion: After being placed for 6 months under the accelerated stability testing conditions (40°C±2°C/75% ±5%RH) and for 18 months under the long-term stability testing conditions (25°C±2°C/60%±5%RH), the samples showed basically the same description, loss on drying, relevant substances, crystalline form, and content as compared with 0 month, and no significant increasing trend was observed. It demonstrates that the crystalline form A-I of compound A has an excellent stability.

## Claims

1. A compound A or a pharmaceutically acceptable salt thereof in a crystalline form, the compound A having a structure represented by Formula (I) below:

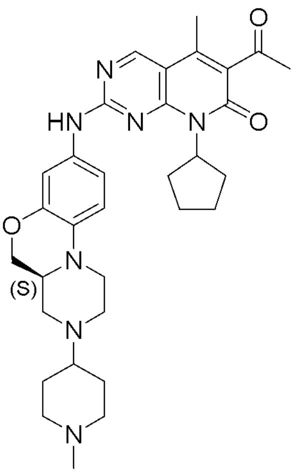

(I).

2. The compound A or the pharmaceutically acceptable salt thereof in the crystalline form according to claim 1, wherein a DSC profile of the compound A in the crystalline form has an endothermic peak at 256 ± 3 °C.

3. The compound A or the pharmaceutically acceptable salt thereof in the crystalline form according to claim 1 or 2, wherein the compound A in the crystalline form is an anhydrate.

4. The compound A or the pharmaceutically acceptable salt thereof in the crystalline form according to any one of claims 1 to 3, wherein the compound A in the crystalline form is a crystalline form A-I, an X-ray powder diffraction pattern of which, using Cu-Kα radiation and expressed as 2θ angles, has characteristic peaks at 5.2 ± 0.2°, 11.4 ± 0.2°, 15.7 ± 0.2°, 20.2 ± 0.2°, and 22.0 ± 0.2°; or

   the compound A in the crystalline form is a crystalline form A-I, an X-ray powder diffraction pattern of which, using Cu-Kα radiation and expressed as 2θ angles, has characteristic peaks at 5.2 ± 0.2°, 9.5 ± 0.2°, 11.4 ± 0.2°, 15.7 ± 0.2°, 16.3 ± 0.2°, 20.2 ± 0.2°, and 22.0 ± 0.2°; or
   the compound A in the crystalline form is a crystalline form A-I, an X-ray powder diffraction pattern of which, using Cu-Kα radiation and expressed as 2θ angles, has characteristic peaks at 5.2 ± 0.2°, 9.5 ± 0.2°, 10.4 ± 0.2°, 11.4 ± 0.2°, 12.8 ± 0.2°, 14.1 ± 0.2°, 15.7 ± 0.2°, 16.3 ± 0.2°, 18.6 ± 0.2°, 20.2 ± 0.2°, 22.0 ± 0.2°, and 25.8 ± 0.2°; or
   the compound A in the crystalline form is a crystalline form A-I, an X-ray powder diffraction pattern of which, using Cu-Kα radiation, is substantially as shown in FIG. 1-1.

5. The compound A or the pharmaceutically acceptable salt thereof in the crystalline form according to any one of claims 1 to 4, wherein the compound A in the crystalline form is a crystalline form A-I, and a DSC profile of the crystalline form A-I has an endothermic peak at 256 ± 3 °C; or
the DSC profile of the crystalline form A-I is substantially as shown in FIG. 1-3.

6. The compound A or the pharmaceutically acceptable salt thereof in the crystalline form according to claim 1, wherein the pharmaceutically acceptable salt of the compound A in the crystalline form is selected from the group consisting of sulfate, methanesulfonate, tartrate, benzenesulfonate, hydrochloride, p-toluenesulfonate, fumarate, citrate, and malate; preferably benzenesulfonate, hydrochloride, p-toluenesulfonate, fumarate, citrate, and malate; more preferably hydrochloride and benzenesulfonate;
preferably, the hydrochloride is a hydrate; more preferably, the hydrochloride is a hydrochloride monohydrate, wherein a molar ratio of the compound A, hydrochloric acid, and water is 1:1:1.

7. The compound A or the pharmaceutically acceptable salt thereof in the crystalline form according to claim 6, wherein the hydrochloride salt of the compound A in the crystalline form is a crystalline form B, an X-ray powder diffraction pattern of which, using Cu-K$\alpha$ radiation and expressed as 2$\theta$ angles, has characteristic peaks at 4.1 ± 0.2°, 8.9 ± 0.2°, 14.3 ± 0.2°, and 19.9 ± 0.2°; or

the hydrochloride salt of the compound A in the crystalline form is a crystalline form B, an X-ray powder diffraction pattern of which, using Cu-K$\alpha$ radiation and expressed as 2$\theta$ angles, has characteristic peaks at 4.1 ± 0.2°, 8.9 ± 0.2°, 12.3 ± 0.2°, 14.3 ± 0.2°, 15.4 ± 0.2°, 16.8 ± 0.2°, and 19.9 ± 0.2°; or
the hydrochloride salt of the compound A in the crystalline form is a crystalline form B, an X-ray powder diffraction pattern of which, using Cu-K$\alpha$ radiation and expressed as 2$\theta$ angles, has characteristic peaks at 4.1 ± 0.2°, 8.3 ± 0.2°, 8.9 ± 0.2°, 9.5 ± 0.2°, 12.3 ± 0.2°, 14.3 ± 0.2°, 15.0 ± 0.2°, 15.4 ± 0.2°, 16.8 ± 0.2°, 19.1 ± 0.2°, 19.9 ± 0.2°, 20.6 ± 0.2°, and 25.5 ± 0.2°; or
the hydrochloride salt of the compound A in the crystalline form is a crystalline form B, an X-ray powder diffraction pattern of which, using Cu-K$\alpha$ radiation and expressed as 2$\theta$ angles, has characteristic peaks at 4.1 ± 0.2°, 8.3 ± 0.2°, 8.9 ± 0.2°, 9.5 ± 0.2°, 11.1 ± 0.2°, 12.3 ± 0.2°, 14.3 ± 0.2°, 15.0 ± 0.2°, 15.4 ± 0.2°, 16.8 ± 0.2°, 17.4 ± 0.2°, 19.1 ± 0.2°, 19.9 ± 0.2°, 20.6 ± 0.2°, 21.6 ± 0.2°, 25.2 ± 0.2°, 25.5 ± 0.2°, 26.6 ± 0.2°, and 29.1 ± 0.2°; or
the hydrochloride salt of the compound A in the crystalline form is a crystalline form B, an X-ray powder diffraction pattern of which, using Cu-K$\alpha$ radiation, is substantially as shown in FIG. 5-1.

8. The compound A or the pharmaceutically acceptable salt thereof in the crystalline form according to claim 6 or 7, wherein the hydrochloride salt of the compound A in the crystalline form is a crystalline form B, a TGA profile of which shows a weight loss of approximately 4.4% from room temperature to 130 °C.

9. The compound A or the pharmaceutically acceptable salt thereof in the crystalline form according to any one of claims 6 to 8, wherein the hydrochloride salt of the compound A in the crystalline form is a crystalline form B, a DSC profile of which has an endothermic peak at 253 °C, or two endothermic peaks at 27 °C and 253 °C.

10. The compound A or the pharmaceutically acceptable salt thereof in the crystalline form according to claim 6, wherein the pharmaceutically acceptable salt of the compound A in the crystalline form is selected from benzenesulfonate; preferably, the benzenesulfonate is an anhydrate; preferably, a molar ratio of the free base of the compound A to benzenesulfonic acid in the benzenesulfonate is 1:1.

11. The compound A or the pharmaceutically acceptable salt thereof in the crystalline form according to claim 10, wherein the benzenesulfonate of the compound A in the crystalline form is a crystalline form C, an X-ray powder diffraction pattern of which, using Cu-K$\alpha$ radiation and expressed as 2$\theta$ angles, has characteristic peaks at 8.5 ± 0.2°, 15.3 ± 0.2°, 19.2 ± 0.2°, 21.7 ± 0.2°, 22.1 ± 0.2°, and 26.1 ± 0.2°; or

the benzenesulfonate of the compound A in the crystalline form is a crystalline form C, an X-ray powder diffraction pattern of which, using Cu-K$\alpha$ radiation and expressed as 2$\theta$ angles, has characteristic peaks at 8.5 ± 0.2°, 15.3 ± 0.2°, 16.4 ± 0.2°, 17.1 ± 0.2°, 17.8 ± 0.2°, 19.2 ± 0.2°, 21.7 ± 0.2°, 22.1 ± 0.2°, and 26.1 ± 0.2°; or
the benzenesulfonate of the compound A in the crystalline form is a crystalline form C, an X-ray powder diffraction pattern of which, using Cu-K$\alpha$ radiation and expressed as 2$\theta$ angles, has characteristic peaks at 8.5 ± 0.2°, 15.3 ± 0.2°, 16.4 ± 0.2°, 17.1 ± 0.2°, 17.8 ± 0.2°, 18.1 ± 0.2°, 19.2 ± 0.2°, 20.2 ± 0.2°, 21.7 ± 0.2°, 22.1 ± 0.2°, and 26.1 ± 0.2°; or
the benzenesulfonate of the compound A in the crystalline form is a crystalline form C, an X-ray powder diffraction

pattern of which, using Cu-Kα radiation and expressed as 2θ angles, has characteristic peaks at 8.5 ± 0.2°, 11.7 ± 0.2°, 12.7 ± 0.2°, 13.3 ± 0.2°, 14.2 ± 0.2°, 15.3 ± 0.2°, 16.4 ± 0.2°, 17.1 ± 0.2°, 17.8 ± 0.2°, 18.1 ± 0.2°, 19.2 ± 0.2°, 19.5 ± 0.2°, 20.2 ± 0.2°, 21.7 ± 0.2°, 22.1 ± 0.2°, and 26.1 ± 0.2°; or
the benzenesulfonate of the compound A in the crystalline form is a crystalline form C, an X-ray powder diffraction pattern of which, using Cu-Kα radiation, is substantially as shown in FIG. 6-1.

**12.** The compound A or the pharmaceutically acceptable salt thereof in the crystalline form according to claim 10 or 11, wherein a DSC profile of the benzenesulfonate of the compound A in the crystalline form has an endothermic peak at 235 ± 3 °C; or
a DSC profile of the benzenesulfonate of the compound A in the crystalline form is substantially as shown in FIG. 6-2.

**13.** A pharmaceutical composition, comprising the compound A or the pharmaceutically acceptable salt thereof in the crystalline form according to any one of claims 1 to 12, and optionally one or more pharmaceutically acceptable excipients.

**14.** Use of the compound A or the pharmaceutically acceptable salt thereof in the crystalline form according to any one of claims 1 to 12, or the pharmaceutical composition according to claim 13, in the manufacture of an anti-tumor medicement;

preferably, the tumor is selected from a sensitive tumor or a drug-resistant tumor;
preferably, the sensitive tumor or the drug-resistant tumor is selected from a solid tumor or a hematological tumor;
preferably, the solid tumor is selected from the group consisting of fibrosarcoma, salivary gland carcinoma, liver cancer, colorectal cancer, bladder cancer, throat cancer, breast cancer, prostate cancer, glioma, ovarian cancer, head and neck squamous cell carcinoma, cervical cancer, esophageal cancer, renal cancer, pancreatic cancer, skin cancer, lymphoma, gastric cancer, multiple myeloma, brain tumor, lung cancer, and melanoma, and the hematological tumor is selected from acute myeloid leukemia;
more preferably, the sensitive tumor or the drug-resistant tumor is selected from the group consisting of breast cancer, lung cancer, colorectal cancer, liver cancer, ovarian cancer, melanoma, and acute myeloid leukemia;
more preferably, the breast cancer is selected from the group consisting of HR+/HER2- breast cancer, triple-negative breast cancer, and HR-/HER2+ breast cancer; the acute myeloid leukemia is selected from myelomonocytic leukemia; and the lung cancer is selected from non-small cell lung cancer.

**15.** The use according to claim 14, wherein the tumor is selected from the drug-resistant tumor; the drug-resistant tumor is selected from the group consisting of tumors with primary drug resistance and tumors with acquired drug resistance;

preferably, the drug-resistant tumor is selected from a CDK4/6 inhibitor-resistant tumor, and further selected from the group consisting of tumors with primary CDK4/6 inhibitor resistance and tumors with acquired CDK4/6 inhibitor resistance; and the CDK4/6 inhibitor is selected from the group consisting of Palbociclib, Abemaciclib, Ribociclib, and Dalpiciclib, preferably Palbociclib;
more preferably, the CDK4/6 inhibitor-resistant tumor is selected from the group consisting of breast cancer and liver cancer, further preferably Palbociclib-resistant breast cancer or liver cancer, and further preferably breast cancer or liver cancer with primary resistance to Palbociclib, or breast cancer or liver cancer with acquired resistance to Palbociclib;
more preferably, the breast cancer with primary resistance to Palbociclib or the breast cancer with acquired resistance to Palbociclib is selected from the group consisting of HR+/HER2- breast cancer, triple-negative breast cancer, and HR-/HER2+ breast cancer, which have primary or acquired resistance to Palbociclib.

**16.** The use according to claim 14 or 15, wherein the compound A or the pharmaceutically acceptable salt thereof in the crystalline form is used in combination with an additional clinically/pharmaceutically acceptable drug for the preparation of the anti-tumor medicement;

the additional clinically/pharmaceutically acceptable drug is selected from endocrine therapy drugs, such as Anastrozole, Letrozole, Exemestane, Tamoxifen, Toremifene, Fulvestrant, Megestrol, Fluoxymesterone, Ethinylestradiol; preferably Fulvestrant;
preferably, the additional clinically/pharmaceutically acceptable drug and the compound A or the pharmaceutically acceptable salt thereof in the crystalline form may be included in a same dosage unit, or in different dosage units that are made into a combination package, such as a kit product.

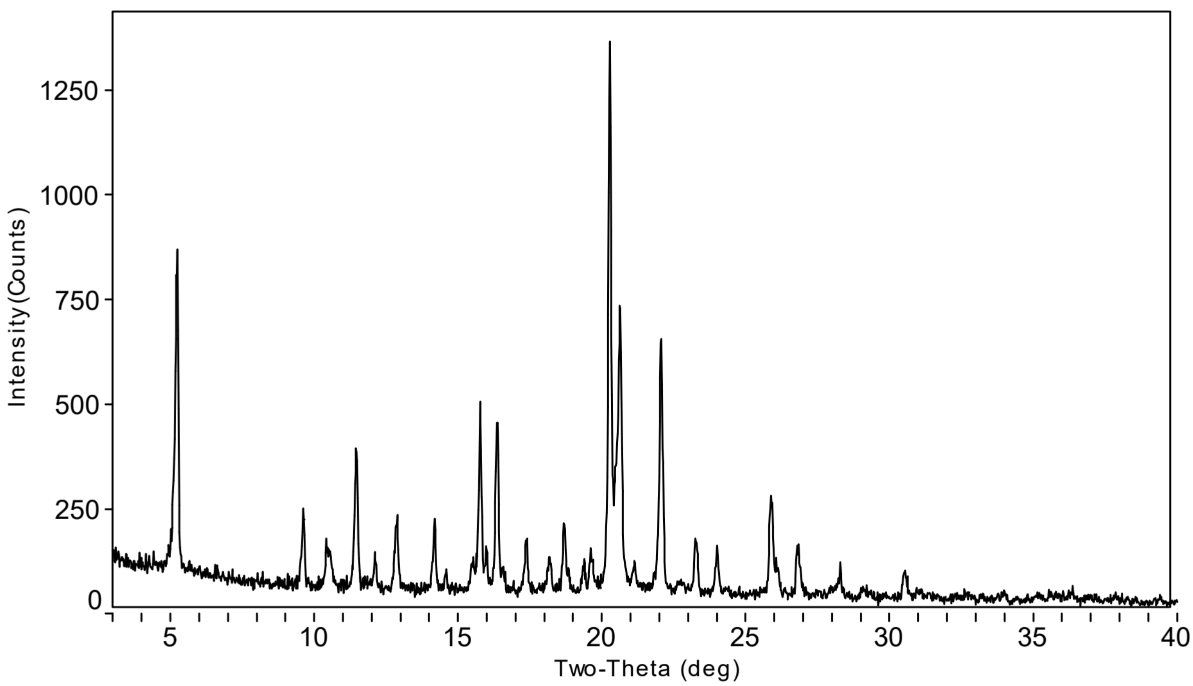
Intensity(Counts)
1250
1000
750
500
250
0
5
10
15
20
25
30
35
40
Two-Theta (deg)

FIG.1-1

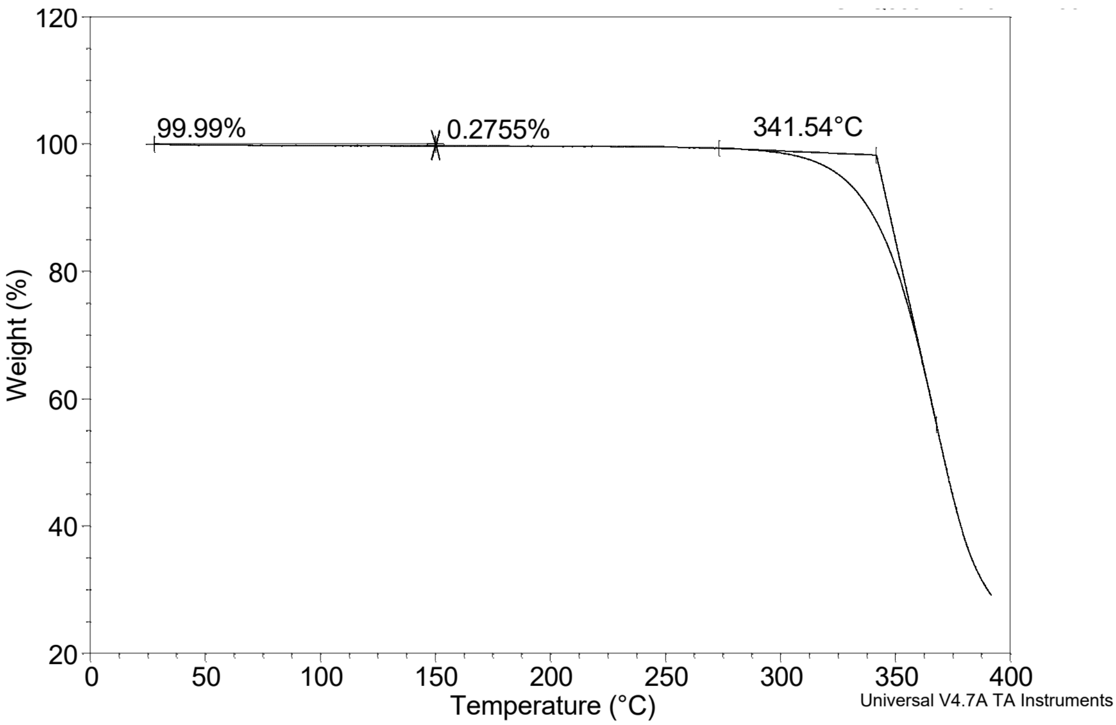
120
100
80
60
40
20
Weight (%)
99.99%
0.2755%
341.54°C
0
50
100
150
200
250
300
350
400
Temperature (°C)
Universal V4.7A TA Instruments

FIG.1-2

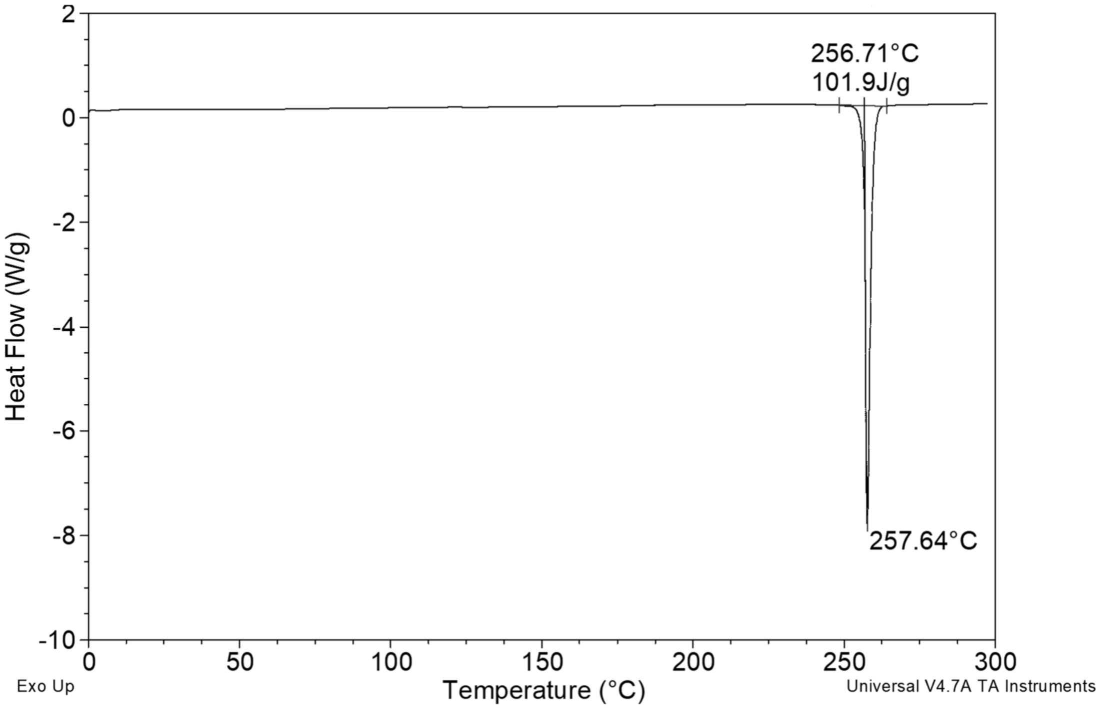
256.71°C
101.9J/g
257.64°C
Heat Flow (W/g)
Temperature (°C)
Exo Up
Universal V4.7A TA Instruments
2
0
-2
-4
-6
-8
-10
0
50
100
150
200
250
300

FIG.1-3

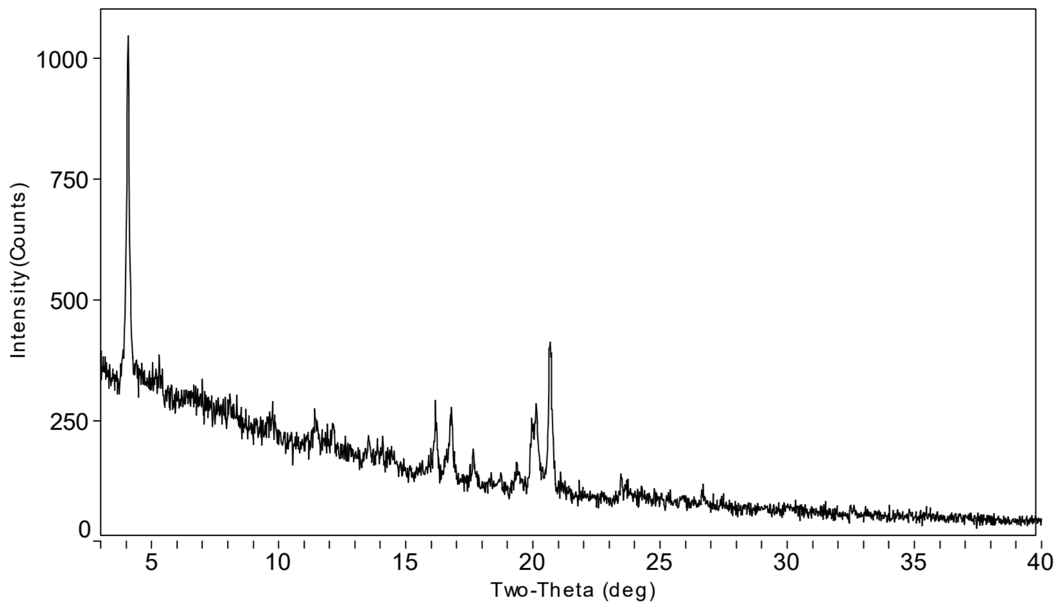
Intensity(Counts)
Two-Theta (deg)
1000
750
500
250
0
5
10
15
20
25
30
35
40

FIG.2-1

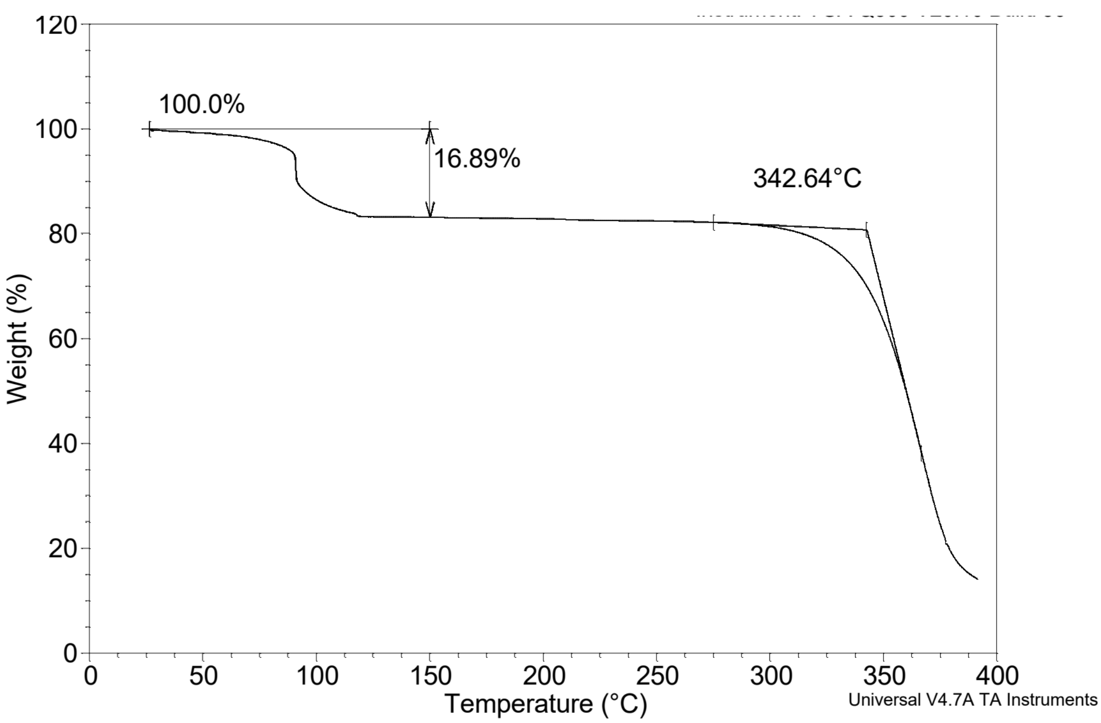

100.0%
16.89%
342.64°C
Weight (%)
Temperature (°C)
Universal V4.7A TA Instruments


FIG.2-2

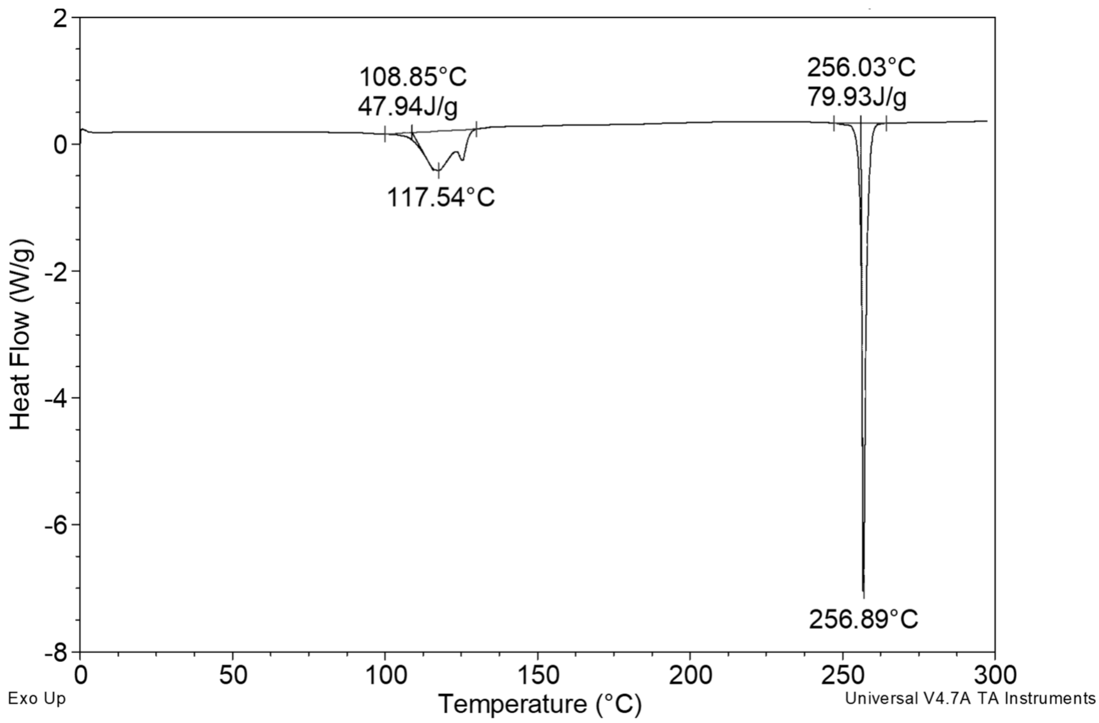

108.85°C
47.94J/g
117.54°C
256.03°C
79.93J/g
256.89°C
Heat Flow (W/g)
Exo Up
Temperature (°C)
Universal V4.7A TA Instruments


FIG.2-3

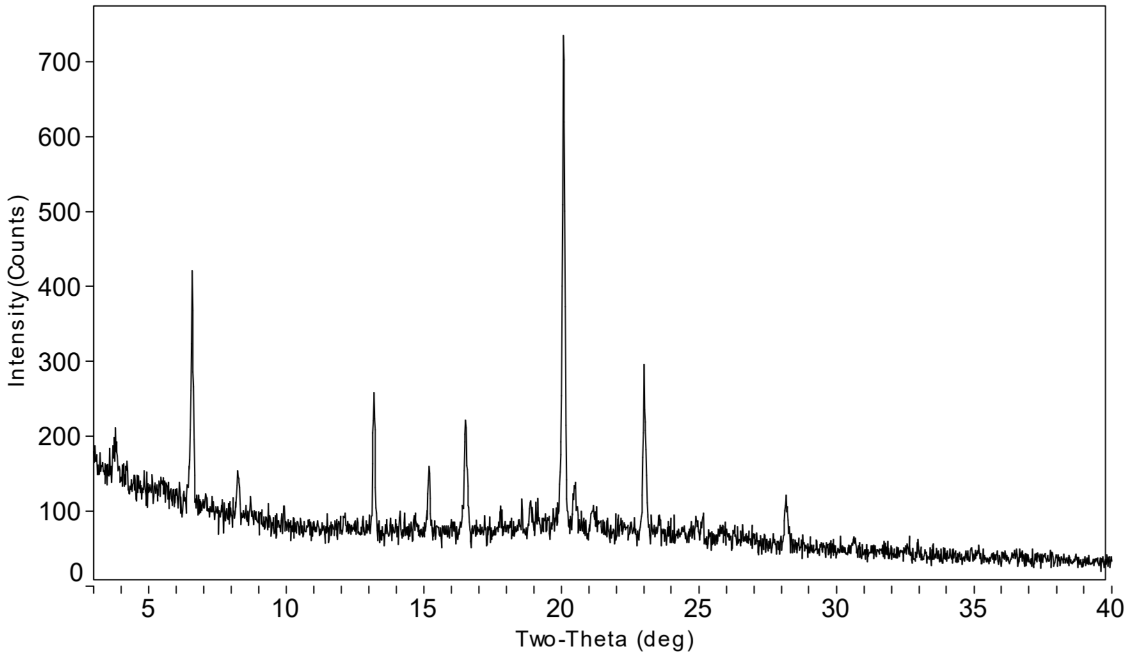

700
600
500
400
300
200
100
0
Intensity(Counts)
5
10
15
20
25
30
35
40
Two-Theta (deg)


FIG.3-1

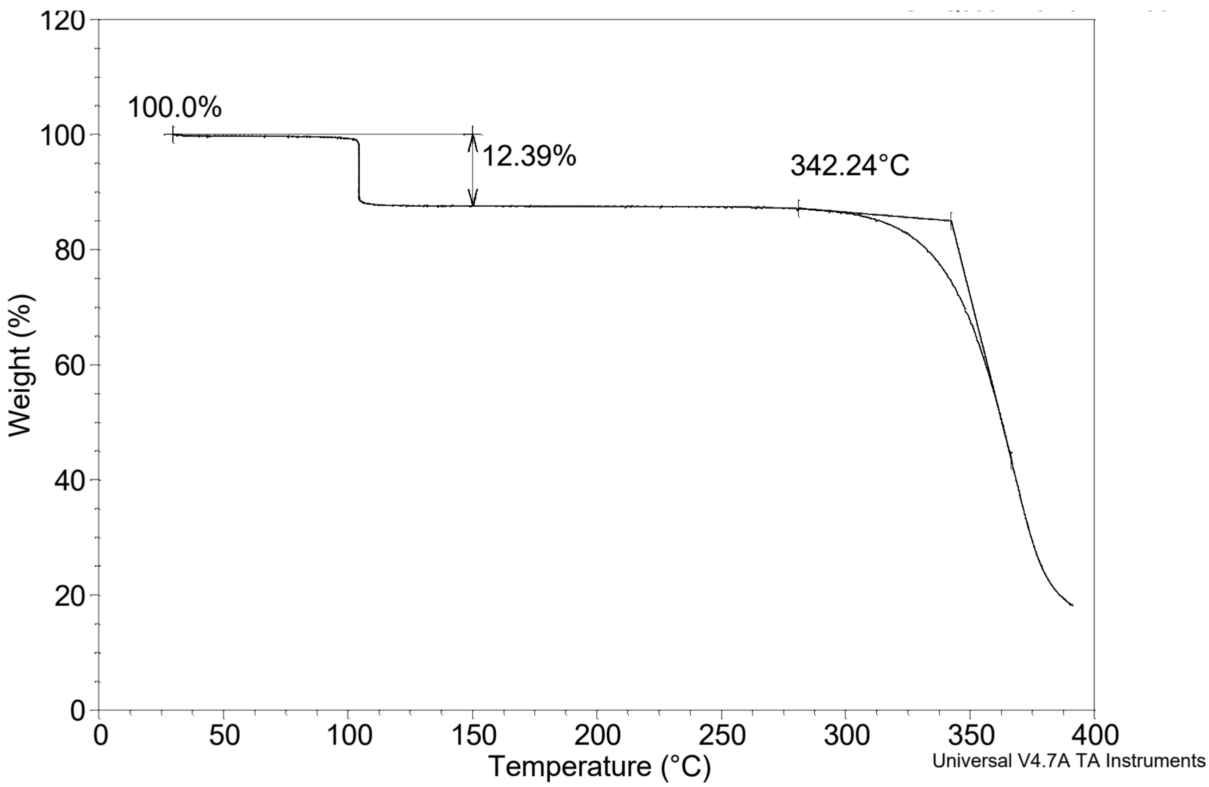

120
100
80
60
40
20
0
Weight (%)
100.0%
12.39%
342.24°C
0
50
100
150
200
250
300
350
400
Temperature (°C)
Universal V4.7A TA Instruments


FIG.3-2

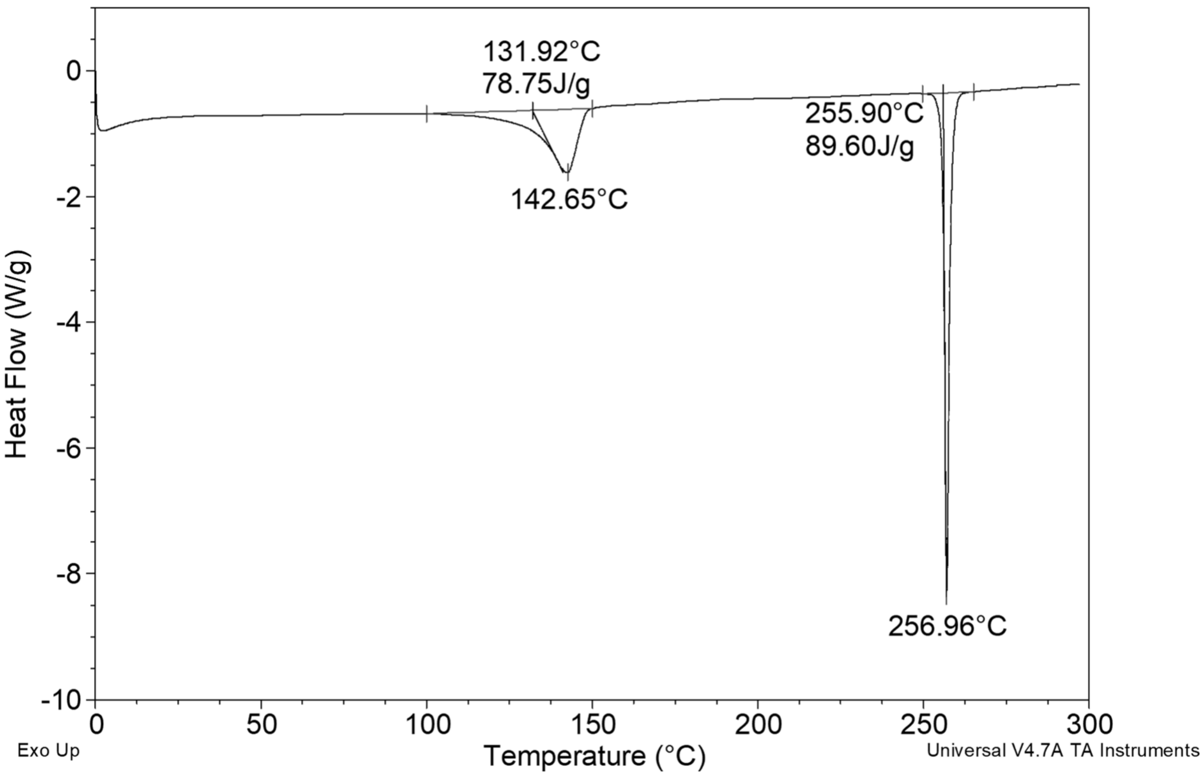
131.92°C
78.75J/g
142.65°C
255.90°C
89.60J/g
256.96°C
Heat Flow (W/g)
Temperature (°C)
Exo Up
Universal V4.7A TA Instruments

FIG.3-3

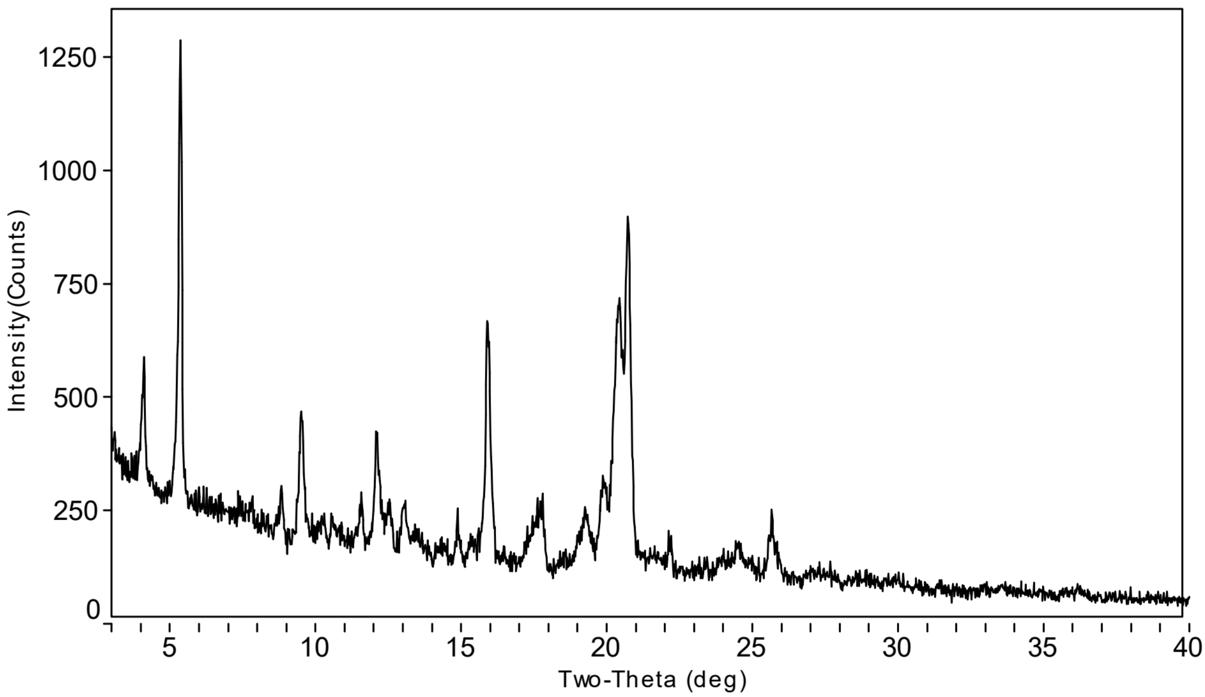
Intensity(Counts)
Two-Theta (deg)

FIG.4-1

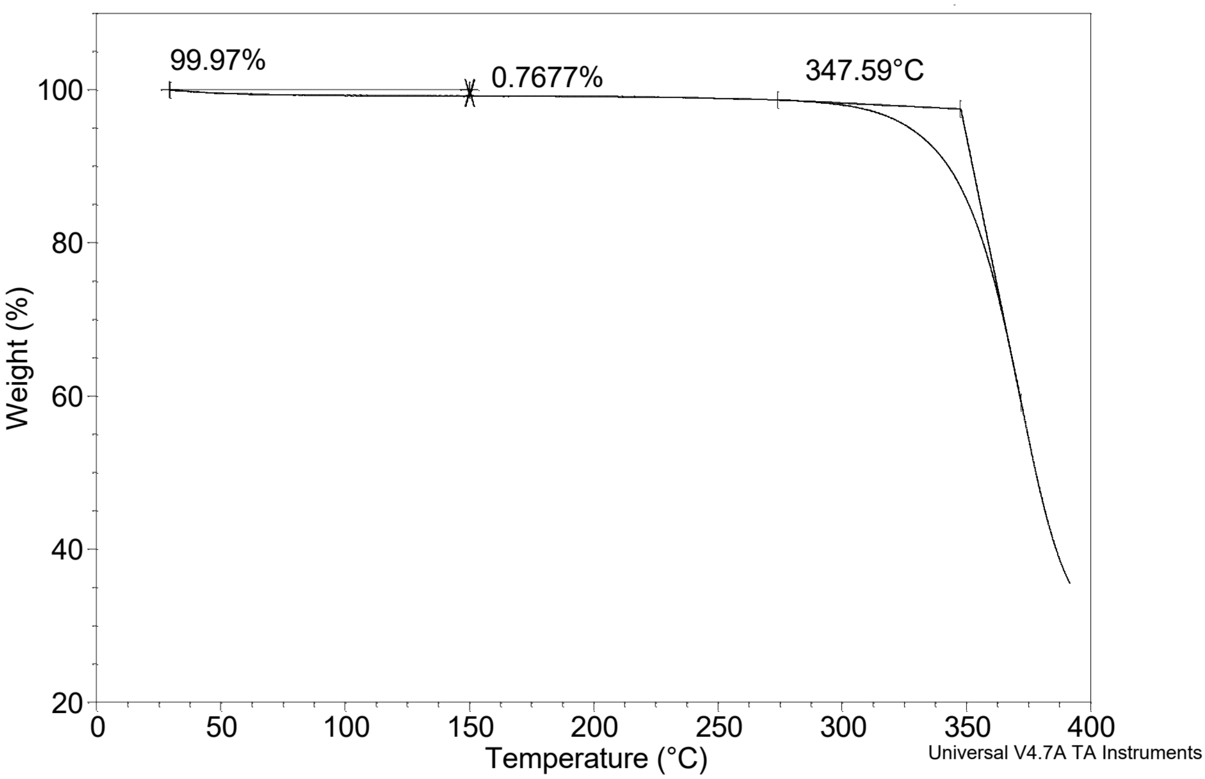

99.97%
0.7677%
347.59°C
Weight (%)
Temperature (°C)
Universal V4.7A TA Instruments


FIG.4-2

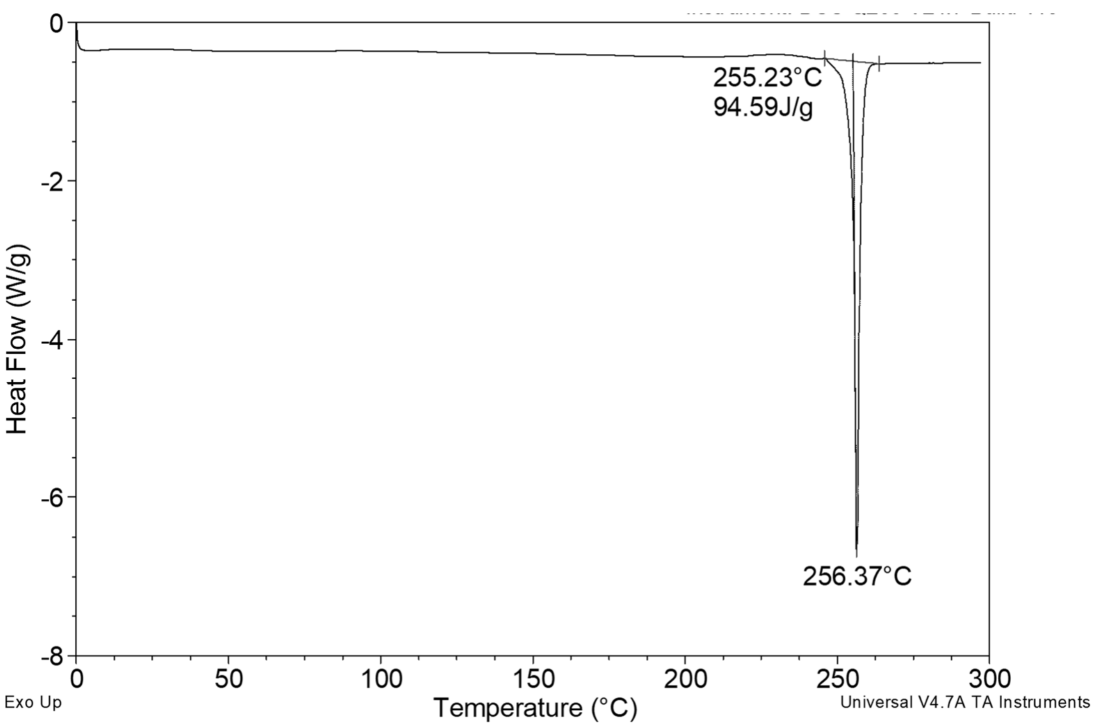

255.23°C
94.59J/g
256.37°C
Heat Flow (W/g)
Exo Up
Temperature (°C)
Universal V4.7A TA Instruments


FIG.4-3

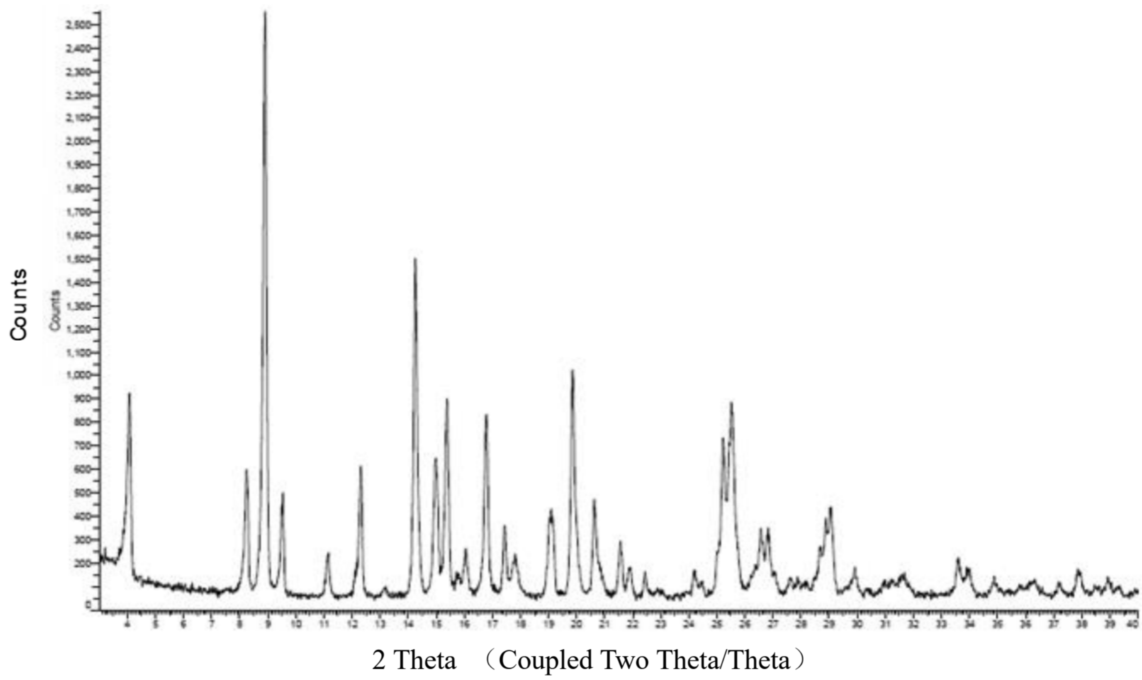
Counts
2 Theta （Coupled Two Theta/Theta）

FIG.5-1

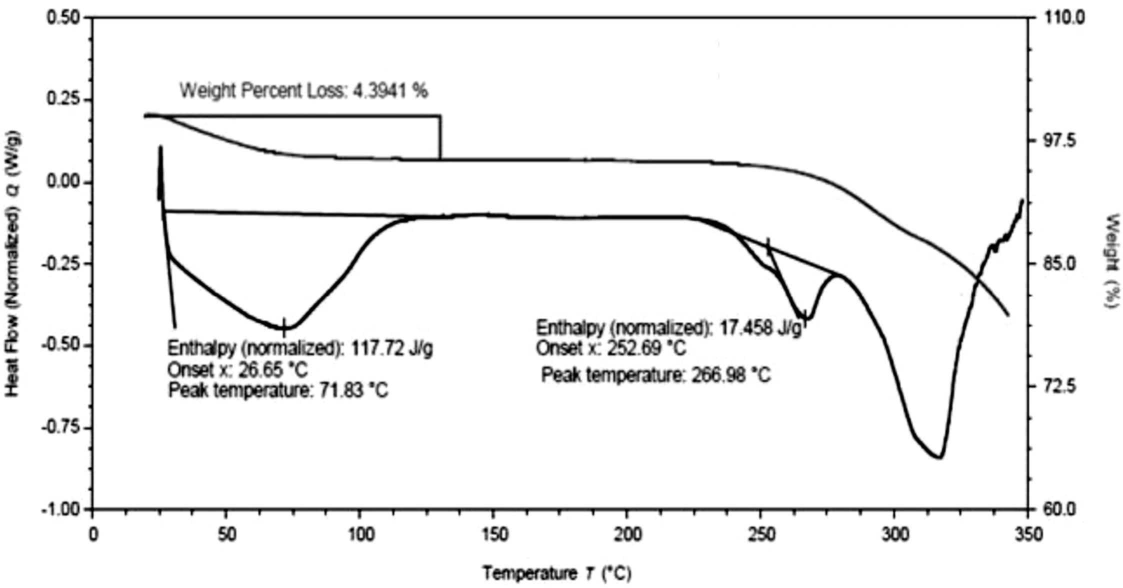
Weight Percent Loss: 4.3941 %
Enthalpy (normalized): 117.72 J/g
Onset x: 26.65 °C
Peak temperature: 71.83 °C
Enthalpy (normalized): 17.458 J/g
Onset x: 252.69 °C
Peak temperature: 266.98 °C
Heat Flow (Normalized) Q (W/g)
Weight (%)
Temperature T (°C)

FIG.5-2

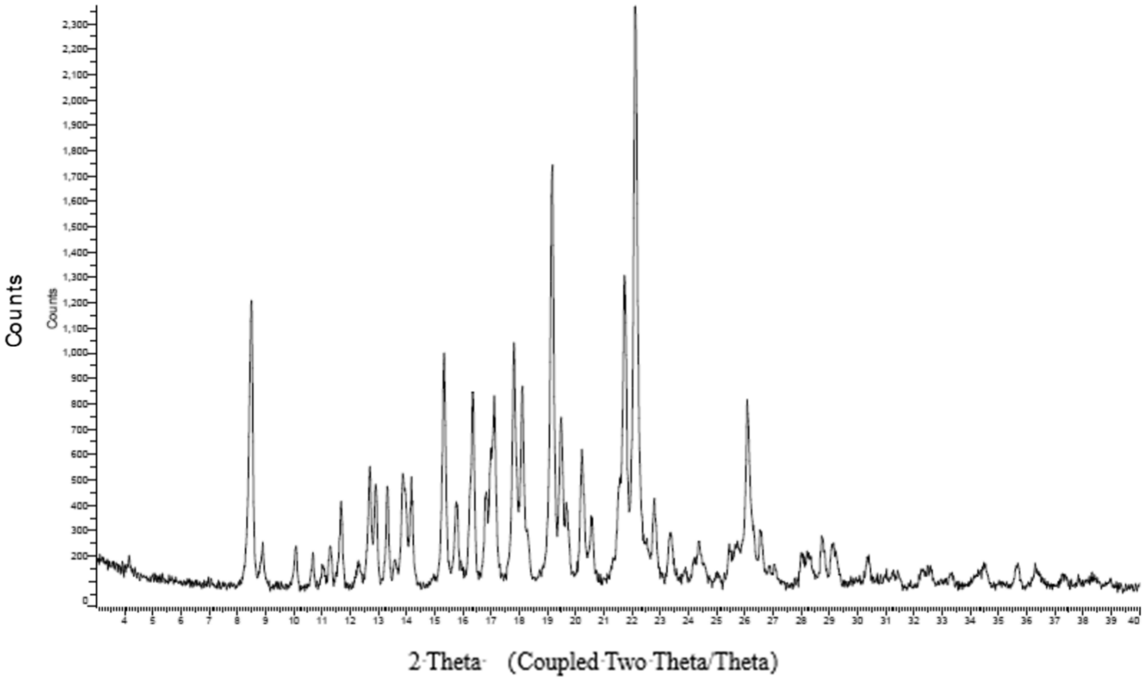
Counts
2-Theta (Coupled Two Theta/Theta)

FIG.6-1

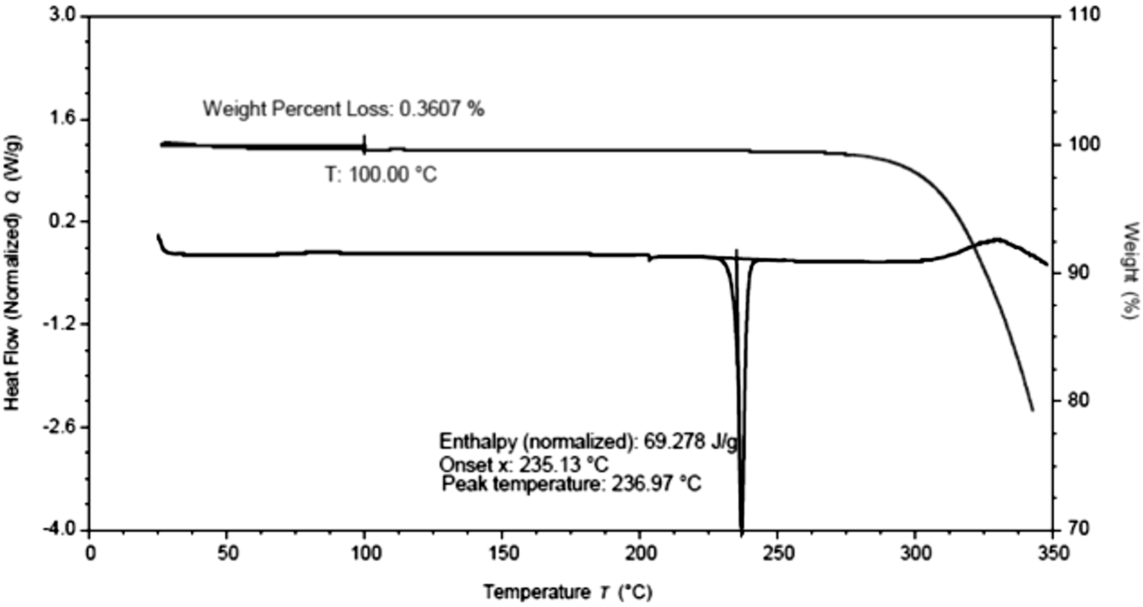
Weight Percent Loss: 0.3607 %
T: 100.00 °C
Enthalpy (normalized): 69.278 J/g
Onset x: 235.13 °C
Peak temperature: 236.97 °C
Heat Flow (Normalized) Q (W/g)
Weight (%)
Temperature T (°C)

FIG.6-2

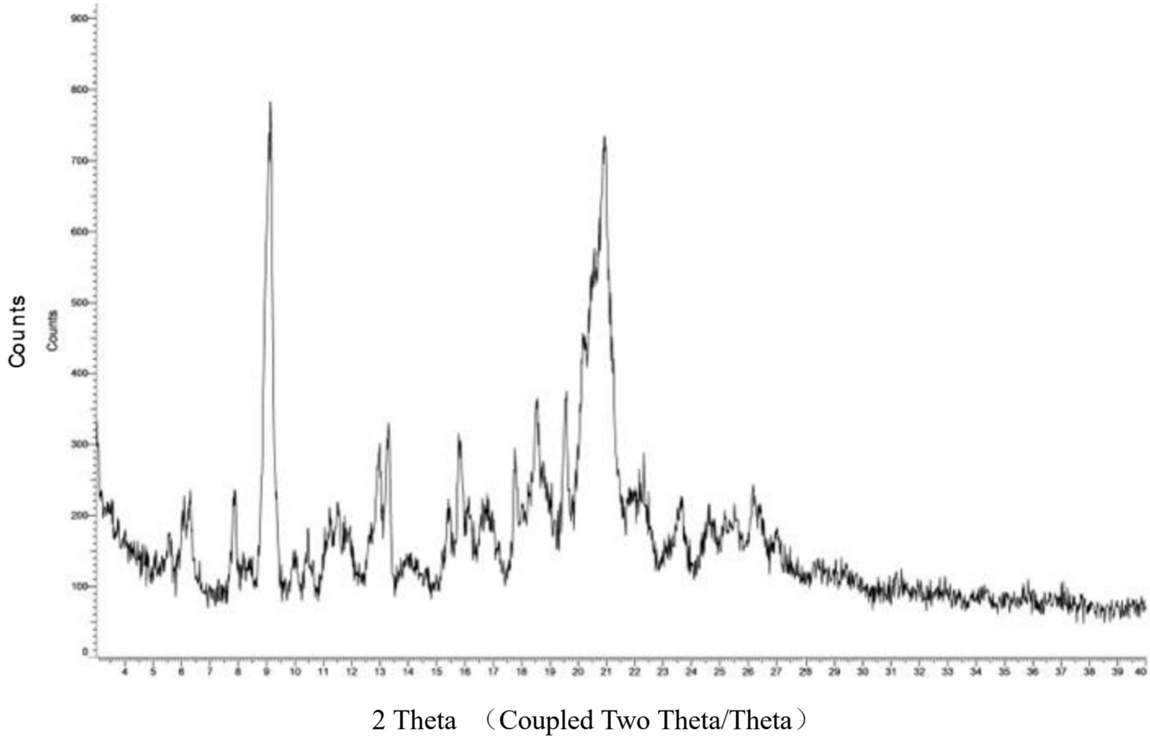
Counts
2 Theta （Coupled Two Theta/Theta）

FIG.7-1

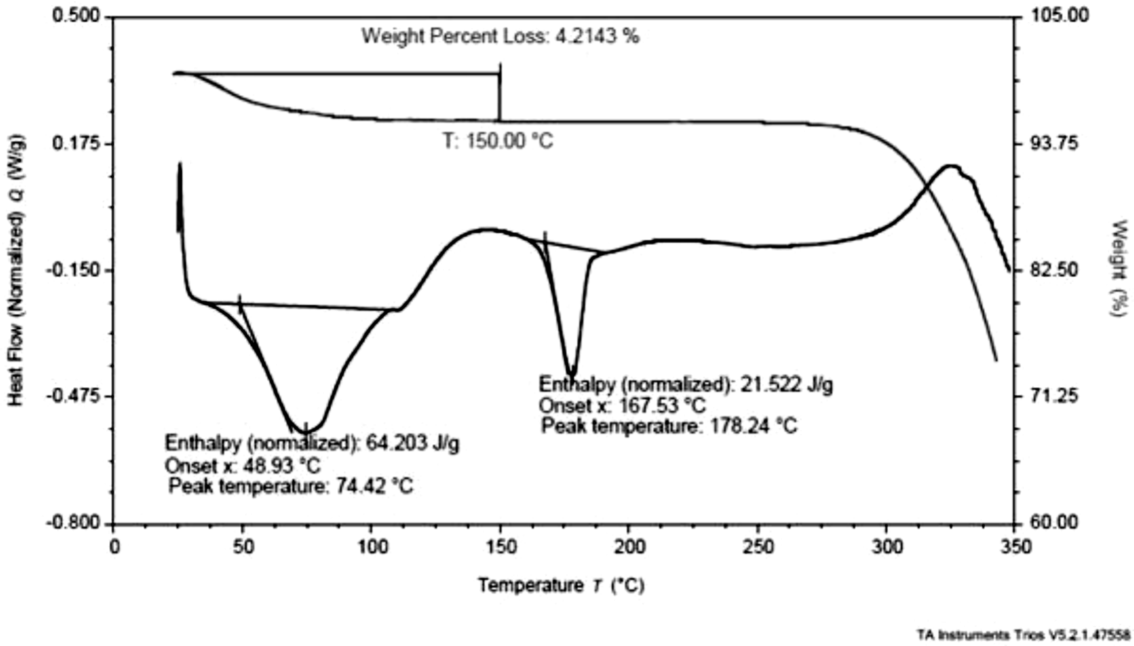
Weight Percent Loss: 4.2143 %
T: 150.00 °C
Enthalpy (normalized): 21.522 J/g
Onset x: 167.53 °C
Peak temperature: 178.24 °C
Enthalpy (normalized): 64.203 J/g
Onset x: 48.93 °C
Peak temperature: 74.42 °C
Heat Flow (Normalized) Q (W/g)
Weight (%)
Temperature T (°C)
TA Instruments Trios V5.2.1.47558

FIG.7-2

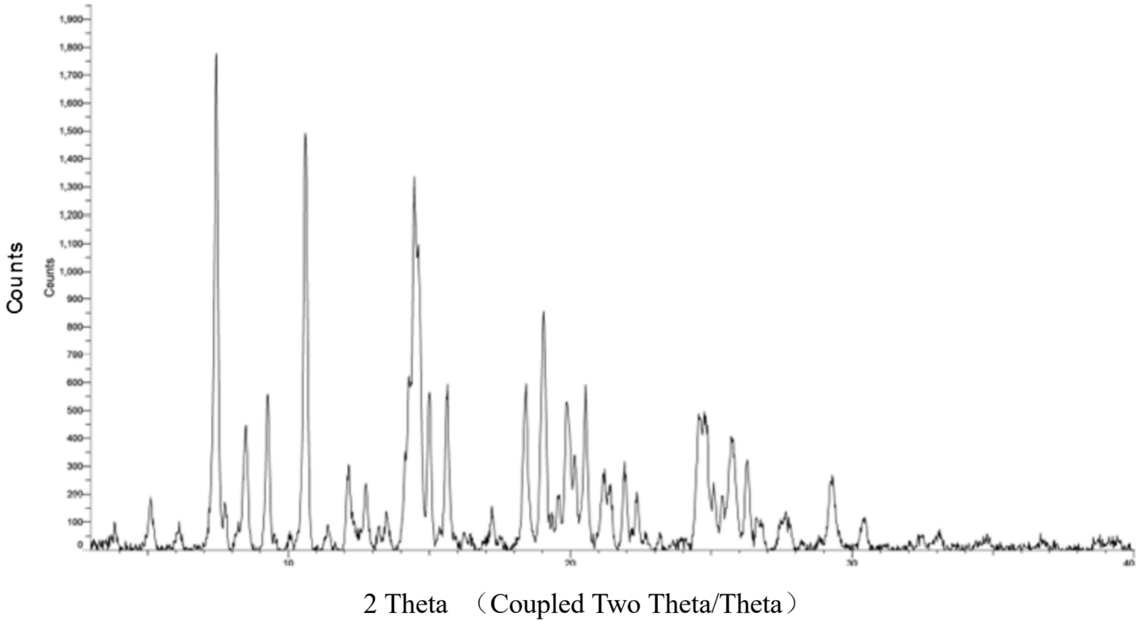
Counts
2 Theta （Coupled Two Theta/Theta）

FIG.8-1

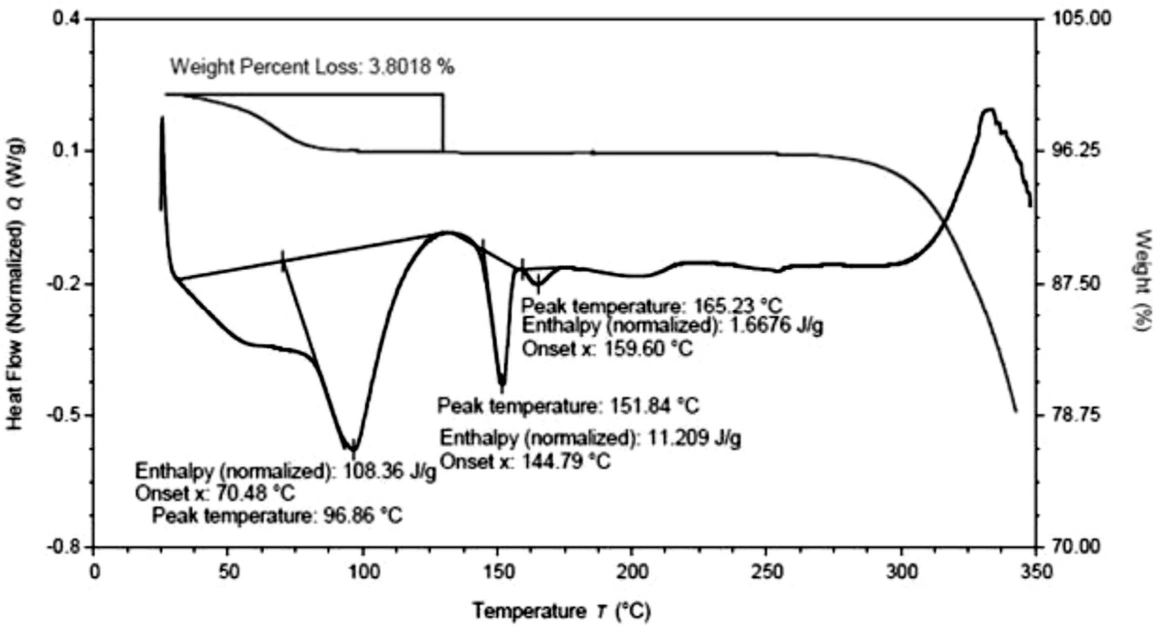
Weight Percent Loss: 3.8018 %
Peak temperature: 165.23 °C
Enthalpy (normalized): 1.6676 J/g
Onset x: 159.60 °C
Peak temperature: 151.84 °C
Enthalpy (normalized): 11.209 J/g
Onset x: 144.79 °C
Enthalpy (normalized): 108.36 J/g
Onset x: 70.48 °C
Peak temperature: 96.86 °C
Heat Flow (Normalized) Q (W/g)
Weight (%)
Temperature T (°C)

FIG.8-2

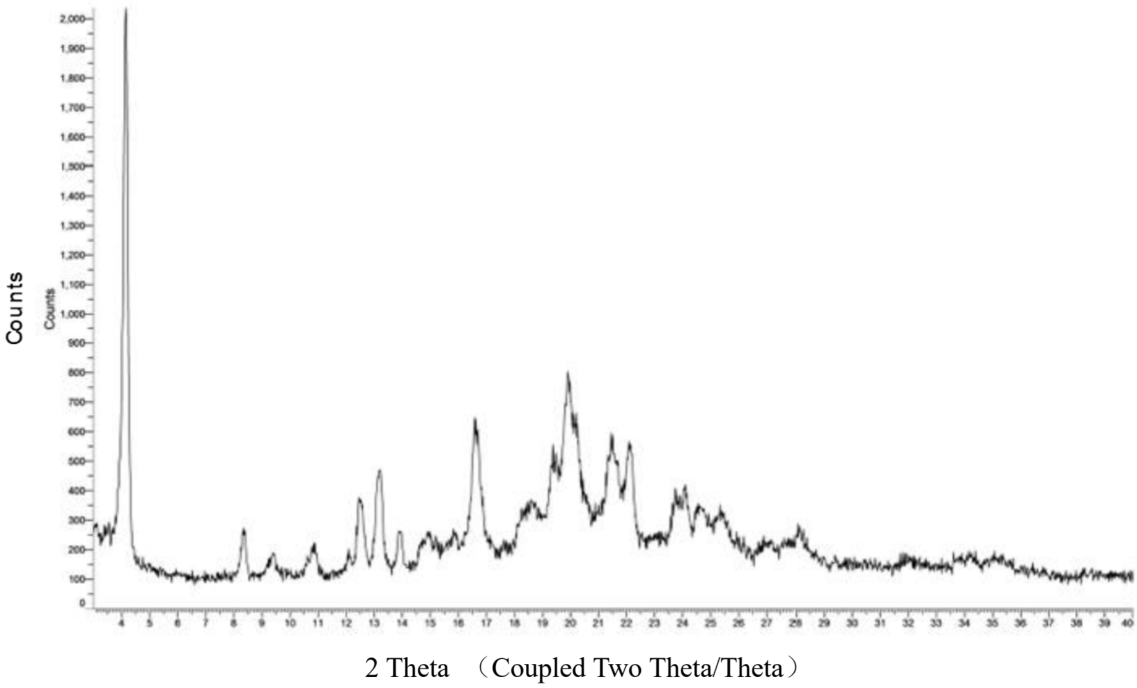
Counts
Counts
2 Theta （Coupled Two Theta/Theta）

FIG.9-1

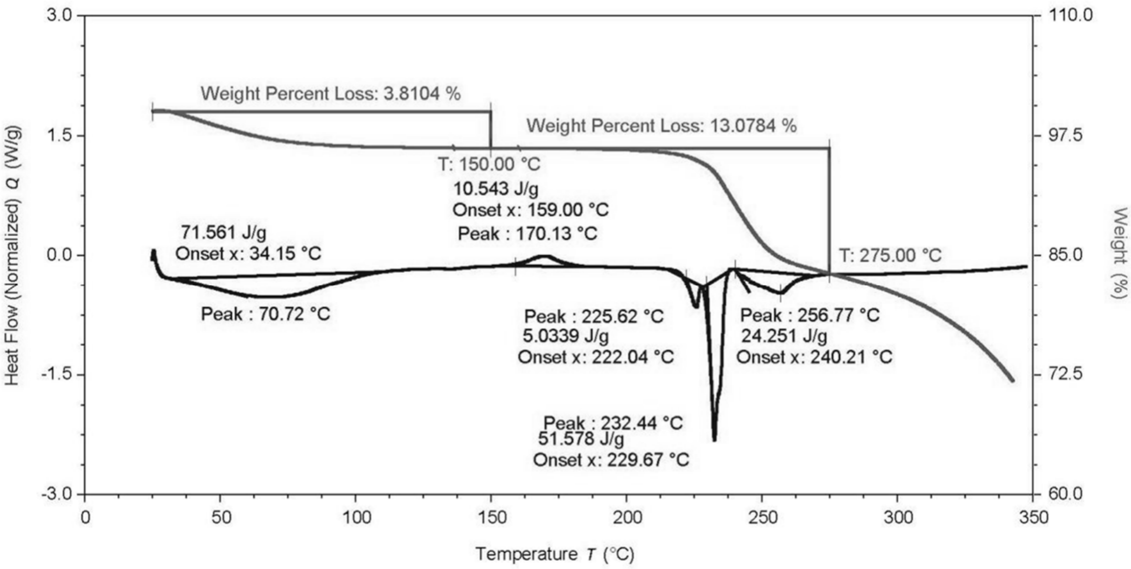
Weight Percent Loss: 3.8104 %
Weight Percent Loss: 13.0784 %
T: 150.00 °C
10.543 J/g
Onset x: 159.00 °C
Peak : 170.13 °C
71.561 J/g
Onset x: 34.15 °C
Peak : 70.72 °C
T: 275.00 °C
Peak : 225.62 °C
5.0339 J/g
Onset x: 222.04 °C
Peak : 256.77 °C
24.251 J/g
Onset x: 240.21 °C
Peak : 232.44 °C
51.578 J/g
Onset x: 229.67 °C
Heat Flow (Normalized) Q (W/g)
Weight (%)
Temperature T (°C)

FIG.9-2

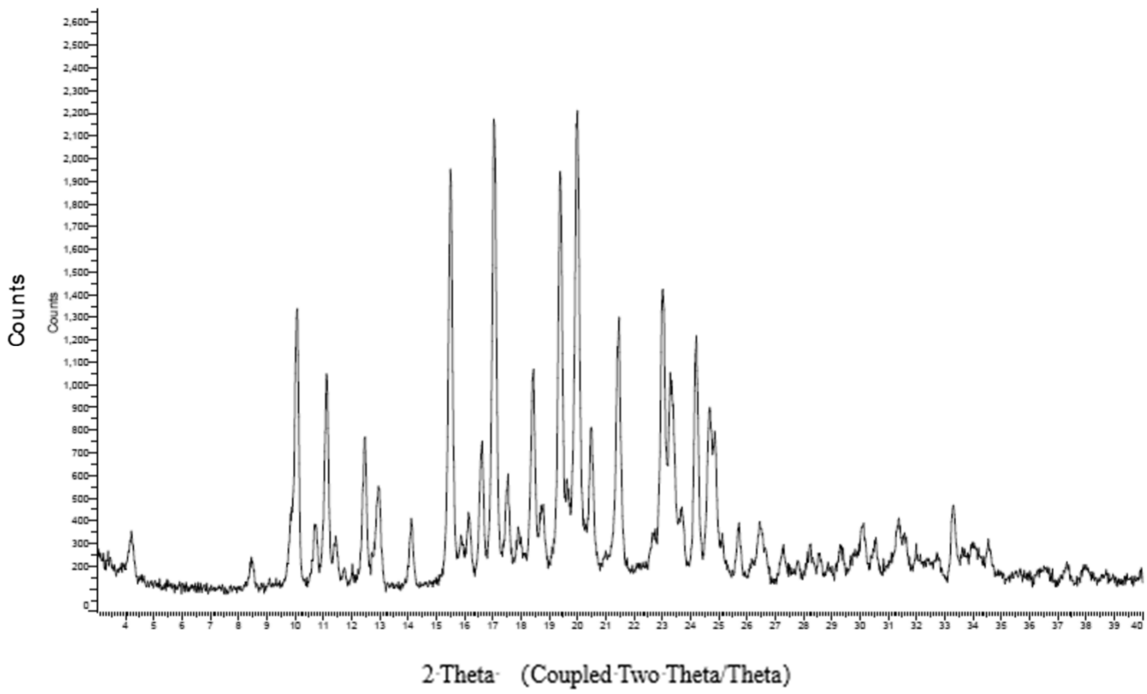


FIG.10-1

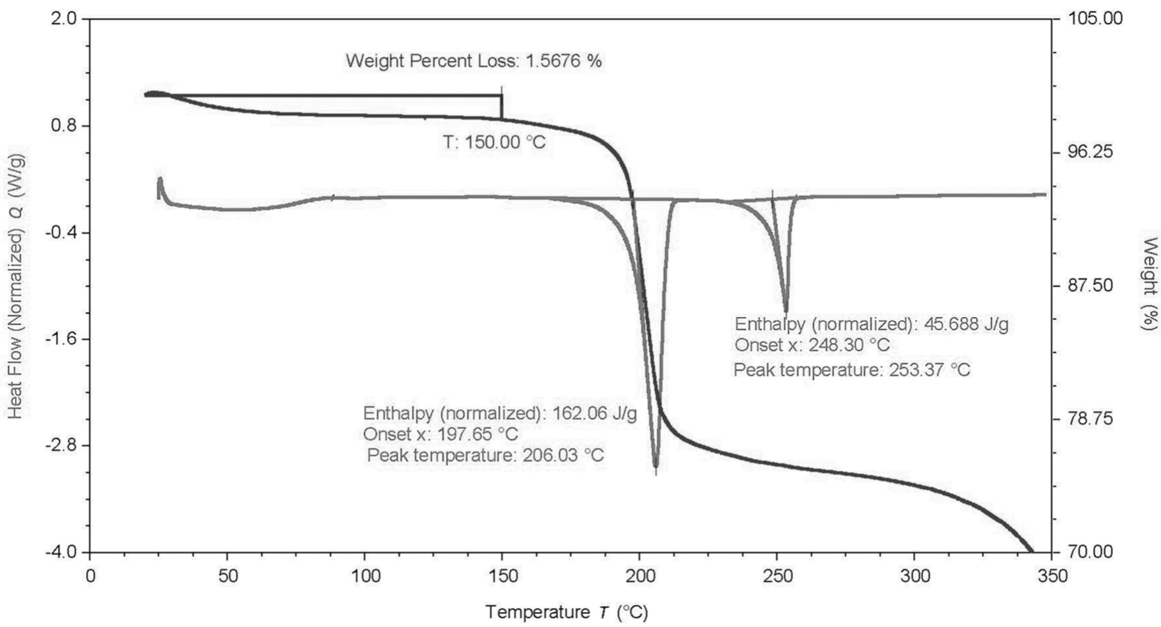


FIG.10-2

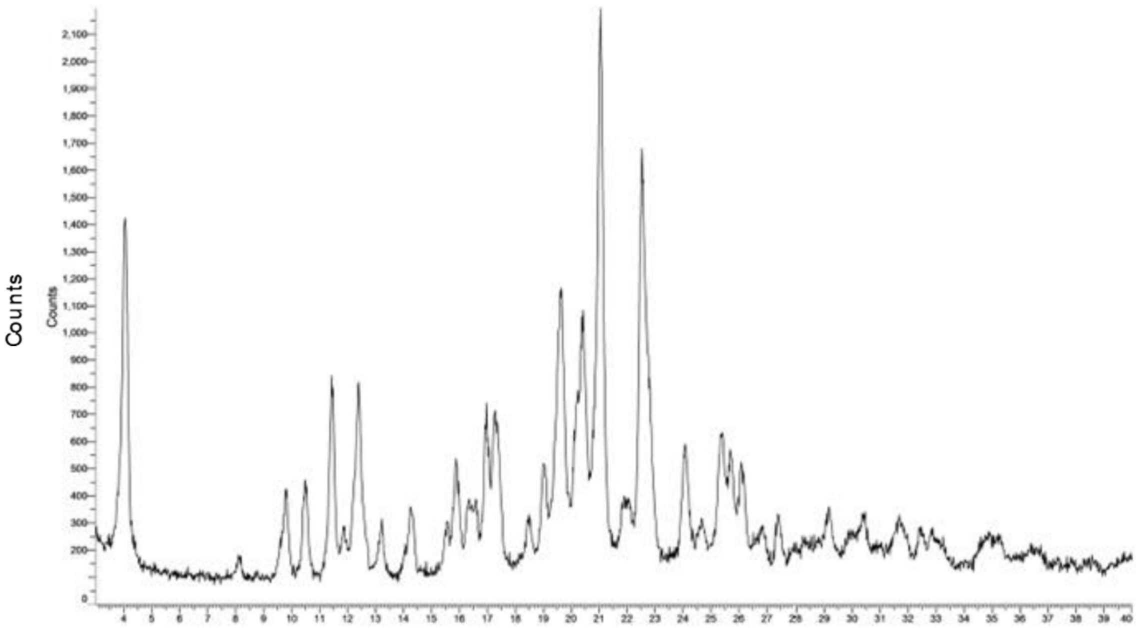


FIG.11-1

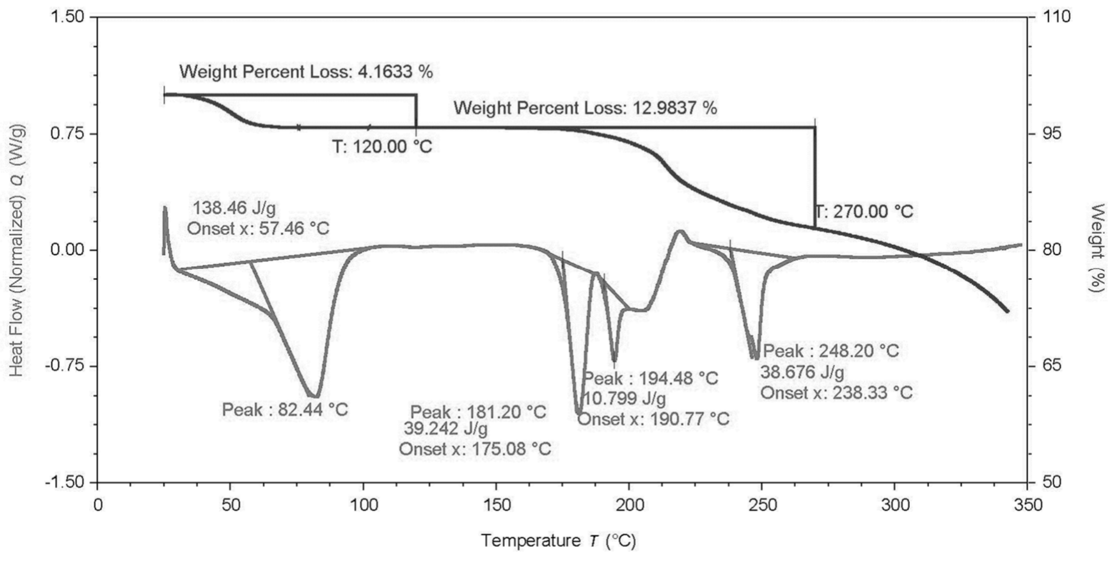


FIG.11-2

## INTERNATIONAL SEARCH REPORT

International application No.
**PCT/CN2024/072459**

**A. CLASSIFICATION OF SUBJECT MATTER**

C07D498/14(2006.01)i; C07D519/00(2006.01)i; A61K31/519(2006.01)i; A61K31/4985(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:C07D498/-,C07D519/-,A61K31/-

Documentation searched other than minimum documentation to the extent that such documents are included in the fields searched

Electronic data base consulted during the international search (name of data base and, where practicable, search terms used)

VEN, CNKI, CNTXT, STN(Reg, Caplus): 激酶, 蛋白依赖性, CDK, kinase, inhibitor?, cyclin dependent, 权利要求1式Istructural formula search

**C. DOCUMENTS CONSIDERED TO BE RELEVANT**

| Category* | Citation of document, with indication, where appropriate, of the relevant passages | Relevant to claim No. |
|---|---|---|
| X | WO 2021139817 A1 (HANGZHOU INNOGATE PHARMA CO., LTD.) 15 July 2021 (2021-07-15)<br>claims 1-17, and embodiment 2 | 1-16 |
| X | CN 108699055 A (HANGZHOU INNOGATE PHARMA CO., LTD.) 23 October 2018 (2018-10-23)<br>entire document | 1-16 |
| A | CN 105849099 A (DANA-FARBER CANCER INSTITUTE, INC.) 10 August 2016 (2016-08-10)<br>entire document | 1-16 |
| A | WO 2016030439 A1 (RATIOPHARM GMBH) 03 March 2016 (2016-03-03)<br>entire document | 1-16 |
| A | WO 2018108084 A1 (HANGZHOU INNOGATE PHARMA CO., LTD.) 21 June 2018 (2018-06-21)<br>entire document | 1-16 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **18 April 2024** | **30 April 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/072459**

| Patent document cited in search report | Publication date (day/month/year) | Patent family member(s) | Publication date (day/month/year) |
|---|---|---|---|
| WO 2021139817 A1 | 15 July 2021 | None | |
| CN 108699055 A | 23 October 2018 | US 2018370991 A1 | 27 December 2018 |
| | | US 11225492 B2 | 18 January 2022 |
| | | WO 2017101763 A1 | 22 June 2017 |
| | | EP 3386981 A1 | 17 October 2018 |
| | | EP 3386981 A4 | 19 June 2019 |
| | | EP 3386981 B1 | 13 October 2021 |
| | | JP 2018537482 A | 20 December 2018 |
| | | JP 6951767 B2 | 20 October 2021 |
| CN 105849099 A | 10 August 2016 | WO 2015058140 A1 | 23 April 2015 |
| | | CA 2927920 A1 | 23 April 2015 |
| | | US 2019031642 A1 | 31 January 2019 |
| | | US 10906889 B2 | 02 February 2021 |
| | | JP 2016533379 A | 27 October 2016 |
| | | JP 6491202 B2 | 27 March 2019 |
| | | AU 2014337044 A1 | 05 May 2016 |
| | | US 2016264554 A1 | 15 September 2016 |
| | | US 10047070 B2 | 14 August 2018 |
| | | EP 3057956 A1 | 24 August 2016 |
| | | EP 3057956 B1 | 05 May 2021 |
| | | AU 2019200372 A1 | 07 February 2019 |
| | | AU 2019200372 B2 | 23 July 2020 |
| WO 2016030439 A1 | 03 March 2016 | IL 250744 A0 | 30 April 2017 |
| | | EP 3186252 A1 | 05 July 2017 |
| WO 2018108084 A1 | 21 June 2018 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310199886
- WO 2021139817 A1


### Non-patent literature cited in the description


- Chinese National Health Commission's Breast Cancer Diagnosis and Treatment Guidelines. the CSCO Breast Cancer Diagnosis and Treatment Guidelines. 2022
- NCCN Clinical Practice Guidelines in Oncology: Breast Cancer. 2022